# EUROPEAN PATENT SPECIFICATION

(11) **EP 2 538 222 B1**
(45) Date of publication and mention of the grant of the patent: **25.08.2021**
(21) Application number: 12185053.1
(22) Date of filing: 04.11.2008
(51) Int. Cl.: G01N 33/68, C07K 14/47, C12Q 1/37, G01N 33/542

(54) **Biochemical markers for CVD risk assessment**
Biochemische Marker zur Bewertung des CVD-Risikos
Marqueurs biochimiques pour évaluation de risque CVD

(30) Priority: 05.11.2007 GB 0721713; 20.11.2007 GB 0722748; 15.02.2008 GB 0802814
(43) Date of publication of application: 26.12.2012
(62) Divisional of application: 08847014.1
(73) Proprietor: Nordic Bioscience A/S, 2730 Herlev (DK)
(72) Inventor: Karsdal, Morten, 2100 Copenhagen (DK); Qvist, Per, 2930 Klampenborg (DK); Barascuk, Natasha, 2100 Copenhagen (DK)
(74) Representative: Beck Greener LLP

(56) References cited:
- EP-A- 1 182 213
- YING S-C ET AL: "Localization of sequence-determined neoepitopes and neutrophil digestion fragments of C-reactive protein utilizing monoclonal antibodies and synthetic peptides", MOLECULAR IMMUNOLOGY, PERGAMON, GB, vol. 29, no. 5, 1 May 1992 (1992-05-01), pages 677-687, XP023683021, ISSN: 0161-5890 [retrieved on 1992-05-01]
- THOMAS J. WANG ET AL: "Multiple Biomarkers for the Prediction of First Major Cardiovascular Events and Death", NEW ENGLAND JOURNAL OF MEDICINE, vol. 355, no. 25, 21 December 2006 (2006-12-21), pages 2631-2639, XP55055202, ISSN: 0028-4793, DOI: 10.1056/NEJMoa055373
- NABATA ET AL: "C-reactive protein induces endothelial cell apoptosis and matrix metalloproteinase-9 production in human mononuclear cells: Implications for the destabilization of atherosclerotic plaque", ATHEROSCLEROSIS, ELSEVIER IRELAND LTD, IE, vol. 196, no. 1, 24 May 2007 (2007-05-24), pages 129-135, XP022416013, ISSN: 0021-9150, DOI: 10.1016/J.ATHEROSCLEROSIS.2007.03.003
- MORT J S ET AL: "The use of cleavage site specific antibodies to delineate protein processing and breakdown pathways", MP. MOLECULAR PATHOLOGY, BMJ PUBLISHING GROUP, LONDON, GB, vol. 52, no. 1, 1 February 1999 (1999-02-01), pages 11-18, XP002670376, ISSN: 1366-8714
- P. Bianco ET AL: "Expression and localization of the two small proteoglycans biglycan and decorin in developing human skeletal and non-skeletal tissues.", JOURNAL OF HISTOCHEMISTRY AND CYTOCHEMISTRY, vol. 38, no. 11, 1 November 1990 (1990-11-01), pages 1549-1563, XP055325532, US ISSN: 0022-1554, DOI: 10.1177/38.11.2212616

### SEQUENCE LISTING

<110> Nordic Bioscience A/s
<120> Biochemical Markers for CVD Risk Assessment
<130> P15861WO
<150> GB 0721713.6
   <151> 2007-11-05
<150> GB 0722748.1
   <151> 2007-11-20
<150> GB 0802814.4
   <151> 2008-02-15
<160> 800
<170> PatentIn version 3.5
<210> 1
   <211> 3396
   <212> PRT
   <213> Homo sapiens
<400> 1
   Met Phe Ile Asn Ile Lys Ser Ile Leu Trp Met Cys Ser Thr Leu Ile
   1               5                   10                  15
   Val Thr His Ala Leu His Lys Val Lys Val Gly Lys Ser Pro Pro Val
               20                  25                  30
   Arg Gly Ser Leu Ser Gly Lys Val Ser Leu Pro Cys His Phe Ser Thr
           35                  40                  45
   Met Pro Thr Leu Pro Pro Ser Tyr Asn Thr Ser Glu Phe Leu Arg Ile
       50                  55                  60
   Lys Trp Ser Lys Ile Glu Val Asp Lys Asn Gly Lys Asp Leu Lys Glu
   65                  70                  75                  80
   Thr Thr Val Leu Val Ala Gln Asn Gly Asn Ile Lys Ile Gly Gln Asp
                   85                  90                  95
   Tyr Lys Gly Arg Val Ser Val Pro Thr His Pro Glu Ala Val Gly Asp
               100                 105                 110
   Ala Ser Leu Thr Val Val Lys Leu Leu Ala Ser Asp Ala Gly Leu Tyr
           115                 120                 125
   Arg Cys Asp Val Met Tyr Gly Ile Glu Asp Thr Gln Asp Thr Val Ser
       130                 135                 140
   Leu Thr Val Asp Gly Val Val Phe His Tyr Arg Ala Ala Thr Ser Arg
   145                 150                 155                 160
   Tyr Thr Leu Asn Phe Glu Ala Ala Gln Lys Ala Cys Leu Asp Val Gly
                   165                 170                 175
   Ala Val Ile Ala Thr Pro Glu Gln Leu Phe Ala Ala Tyr Glu Asp Gly
               180                 185                 190
   Phe Glu Gln Cys Asp Ala Gly Trp Leu Ala Asp Gln Thr Val Arg Tyr
           195                 200                 205
   Pro Ile Arg Ala Pro Arg Val Gly Cys Tyr Gly Asp Lys Met Gly Lys
       210                 215                 220
   Ala Gly Val Arg Thr Tyr Gly Phe Arg Ser Pro Gln Glu Thr Tyr Asp
   225                 230                 235                 240
   Val Tyr Cys Tyr Val Asp His Leu Asp Gly Asp Val Phe His Leu Thr
                   245                 250                 255
   Val Pro Ser Lys Phe Thr Phe Glu Glu Ala Ala Lys Glu Cys Glu Asn
               260                 265                 270
   Gln Asp Ala Arg Leu Ala Thr Val Gly Glu Leu Gln Ala Ala Trp Arg
           275                 280                 285
   Asn Gly Phe Asp Gln Cys Asp Tyr Gly Trp Leu Ser Asp Ala Ser Val
       290                 295                 300
   Arg His Pro Val Thr Val Ala Arg Ala Gln Cys Gly Gly Gly Leu Leu
   305                 310                 315                 320
   Gly Val Arg Thr Leu Tyr Arg Phe Glu Asn Gln Thr Gly Phe Pro Pro
                   325                 330                 335
   Pro Asp Ser Arg Phe Asp Ala Tyr Cys Phe Lys Pro Lys Glu Ala Thr
               340                 345                 350
   Thr Ile Asp Leu Ser Ile Leu Ala Glu Thr Ala Ser Pro Ser Leu Ser
           355                 360                 365
   Lys Glu Pro Gln Met Val Ser Asp Arg Thr Thr Pro Ile Ile Pro Leu
       370                 375                 380
   Val Asp Glu Leu Pro Val Ile Pro Thr Glu Phe Pro Pro Val Gly Asn
   385                 390                 395                 400
   Ile Val Ser Phe Glu Gln Lys Ala Thr Val Gln Pro Gln Ala Ile Thr
                   405                 410                 415
   Asp Ser Leu Ala Thr Lys Leu Pro Thr Pro Thr Gly Ser Thr Lys Lys
               420                 425                 430
   Pro Trp Asp Met Asp Asp Tyr Ser Pro Ser Ala Ser Gly Pro Leu Gly
           435                 440                 445
   Lys Leu Asp Ile Ser Glu Ile Lys Glu Glu Val Leu Gln Ser Thr Thr
       450                 455                 460
   Gly Val Ser His Tyr Ala Thr Asp Ser Trp Asp Gly Val Val Glu Asp
   465                 470                 475                 480
   Lys Gln Thr Gln Glu Ser Val Thr Gln Ile Glu Gln Ile Glu Val Gly
                   485                 490                 495
   Pro Leu Val Thr Ser Met Glu Ile Leu Lys His Ile Pro Ser Lys Glu
               500                 505                 510
   Phe Pro Val Thr Glu Thr Pro Leu Val Thr Ala Arg Met Ile Leu Glu
           515                 520                 525
   Ser Lys Thr Glu Lys Lys Met Val Ser Thr Val Ser Glu Leu Val Thr
       530                 535                 540
   Thr Gly His Tyr Gly Phe Thr Leu Gly Glu Glu Asp Asp Glu Asp Arg
   545                 550                 555                 560
   Thr Leu Thr Val Gly Ser Asp Glu Ser Thr Leu Ile Phe Asp Gln Ile
                   565                 570                 575
   Pro Glu Val Ile Thr Val Ser Lys Thr Ser Glu Asp Thr Ile His Thr
               580                 585                 590
   His Leu Glu Asp Leu Glu Ser Val Ser Ala Ser Thr Thr Val Ser Pro
           595                 600                 605
   Leu Ile Met Pro Asp Asn Asn Gly Ser Ser Met Asp Asp Trp Glu Glu
       610                 615                 620
   Arg Gln Thr Ser Gly Arg Ile Thr Glu Glu Phe Leu Gly Lys Tyr Leu
   625                 630                 635                 640
   Ser Thr Thr Pro Phe Pro Ser Gln His Arg Thr Glu Ile Glu Leu Phe
                   645                 650                 655
   Pro Tyr Ser Gly Asp Lys Ile Leu Val Glu Gly Ile Ser Thr Val Ile
               660                 665                 670
   Tyr Pro Ser Leu Gln Thr Glu Met Thr His Arg Arg Glu Arg Thr Glu
           675                 680                 685
   Thr Leu Ile Pro Glu Met Arg Thr Asp Thr Tyr Thr Asp Glu Ile Gln
       690                 695                 700
   Glu Glu Ile Thr Lys Ser Pro Phe Met Gly Lys Thr Glu Glu Glu Val
   705                 710                 715                 720
   Phe Ser Gly Met Lys Leu Ser Thr Ser Leu Ser Glu Pro Ile His Val
                   725                 730                 735
   Thr Glu Ser Ser Val Glu Met Thr Lys Ser Phe Asp Phe Pro Thr Leu
               740                 745                 750
   Ile Thr Lys Leu Ser Ala Glu Pro Thr Glu Val Arg Asp Met Glu Glu
           755                 760                 765
   Asp Phe Thr Ala Thr Pro Gly Thr Thr Lys Tyr Asp Glu Asn Ile Thr
       770                 775                 780
   Thr Val Leu Leu Ala His Gly Thr Leu Ser Val Glu Ala Ala Thr Val
   785                 790                 795                 800
   Ser Lys Trp Ser Trp Asp Glu Asp Asn Thr Thr Ser Lys Pro Leu Glu
                   805                 810                 815
   Ser Thr Glu Pro Ser Ala Ser Ser Lys Leu Pro Pro Ala Leu Leu Thr
               820                 825                 830
   Thr Val Gly Met Asn Gly Lys Asp Lys Asp Ile Pro Ser Phe Thr Glu
           835                 840                 845
   Asp Gly Ala Asp Glu Phe Thr Leu Ile Pro Asp Ser Thr Gln Lys Gln
       850                 855                 860
   Leu Glu Glu Val Thr Asp Glu Asp Ile Ala Ala His Gly Lys Phe Thr
   865                 870                 875                 880
   Ile Arg Phe Gln Pro Thr Thr Ser Thr Gly Ile Ala Glu Lys Ser Thr
                   885                 890                 895
   Leu Arg Asp Ser Thr Thr Glu Glu Lys Val Pro Pro Ile Thr Ser Thr
               900                 905                 910
   Glu Gly Gln Val Tyr Ala Thr Met Glu Gly Ser Ala Leu Gly Glu Val
           915                 920                 925
   Glu Asp Val Asp Leu Ser Lys Pro Val Ser Thr Val Pro Gln Phe Ala
   930 935 940
   His Thr Ser Glu Val Glu Gly Leu Ala Phe Val Ser Tyr Ser Ser Thr
   945 950 955 960
   Gln Glu Pro Thr Thr Tyr Val Asp Ser Ser His Thr Ile Pro Leu Ser
   965 970 975
   Val Ile Pro Lys Thr Asp Trp Gly Val Leu Val Pro Ser Val Pro Ser
   980 985 990
   Glu Asp Glu Val Leu Gly Glu Pro Ser Gln Asp Ile Leu Val Ile Asp
   995 1000 1005
   Gln Thr Arg Leu Glu Ala Thr Ile Ser Pro Glu Thr Met Arg Thr
   1010 1015 1020
   Thr Lys Ile Thr Glu Gly Thr Thr Gln Glu Glu Phe Pro Trp Lys
   1025 1030 1035
   Glu Gln Thr Ala Glu Lys Pro Val Pro Ala Leu Ser Ser Thr Ala
   1040 1045 1050
   Trp Thr Pro Lys Glu Ala Val Thr Pro Leu Asp Glu Gln Glu Gly
   1055 1060 1065
   Asp Gly Ser Ala Tyr Thr Val Ser Glu Asp Glu Leu Leu Thr Gly
   1070 1075 1080
   Ser Glu Arg Val Pro Val Leu Glu Thr Thr Pro Val Gly Lys Ile
   1085 1090 1095
   Asp His Ser Val Ser Tyr Pro Pro Gly Ala Val Thr Glu His Lys
   1100 1105 1110
   Val Lys Thr Asp Glu Val Val Thr Leu Thr Pro Arg Ile Gly Pro
   1115 1120 1125
   Lys Val Ser Leu Ser Pro Gly Pro Glu Gln Lys Tyr Glu Thr Glu
   1130 1135 1140
   Gly Ser Ser Thr Thr Gly Phe Thr Ser Ser Leu Ser Pro Phe Ser
   1145 1150 1155
   Thr His Ile Thr Gln Leu Met Glu Glu Thr Thr Thr Glu Lys Thr
   1160 1165 1170
   Ser Leu Glu Asp Ile Asp Leu Gly Ser Gly Leu Phe Glu Lys Pro
       1175                1180                1185
   Lys Ala  Thr Glu Leu Ile Glu  Phe Ser Thr Ile Lys  Val Thr Val
       1190                1195                1200
   Pro Ser  Asp Ile Thr Thr Ala  Phe Ser Ser Val Asp  Arg Leu His
       1205                1210                1215
   Thr Thr  Ser Ala Phe Lys Pro  Ser Ser Ala Ile Thr  Lys Lys Pro
       1220                1225                1230
   Pro Leu  Ile Asp Arg Glu Pro  Gly Glu Glu Thr Thr  Ser Asp Met
       1235                1240                1245
   Val Ile  Ile Gly Glu Ser Thr  Ser His Val Pro Pro  Thr Thr Leu
       1250                1255                1260
   Glu Asp  Ile Val Ala Lys Glu  Thr Glu Thr Asp Ile  Asp Arg Glu
       1265                1270                1275
   Tyr Phe  Thr Thr Ser Ser Pro  Pro Ala Thr Gln Pro  Thr Arg Pro
       1280                1285                1290
   Pro Thr  Val Glu Asp Lys Glu  Ala Phe Gly Pro Gln  Ala Leu Ser
       1295                1300                1305
   Thr Pro  Gln Pro Pro Ala Ser  Thr Lys Phe His Pro  Asp Ile Asn
       1310                1315                1320
   Val Tyr  Ile Ile Glu Val Arg  Glu Asn Lys Thr Gly  Arg Met Ser
       1325                1330                1335
   Asp Leu  Ser Val Ile Gly His  Pro Ile Asp Ser Glu  Ser Lys Glu
       1340                1345                1350
   Asp Glu  Pro Cys Ser Glu Glu  Thr Asp Pro Val His  Asp Leu Met
       1355                1360                1365
   Ala Glu  Ile Leu Pro Glu Phe  Pro Asp Ile Ile Glu  Ile Asp Leu
       1370                1375                1380
   Tyr His  Ser Glu Glu Asn Glu  Glu Glu Glu Glu Glu  Cys Ala Asn
       1385                1390                1395
   Ala Thr  Asp Val Thr Thr Thr  Pro Ser Val Gln Tyr  Ile Asn Gly
       1400                1405                1410
   Lys His  Leu Val Thr Thr Val  Pro Lys Asp Pro Glu  Ala Ala Glu
       1415                1420                1425
   Ala Arg  Arg Gly Gln Phe Glu  Ser Val Ala Pro Ser  Gln Asn Phe
       1430                 1435                 1440
   Ser Asp  Ser Ser Glu Ser Asp  Thr His Pro Phe Val  Ile Ala Lys
       1445                 1450                 1455
   Thr Glu  Leu Ser Thr Ala Val  Gln Pro Asn Glu Ser  Thr Glu Thr
       1460                 1465                 1470
   Thr Glu  Ser Leu Glu Val Thr  Trp Lys Pro Glu Thr  Tyr Pro Glu
       1475                 1480                 1485
   Thr Ser  Glu His Phe Ser Gly  Gly Glu Pro Asp Val  Phe Pro Thr
       1490                 1495                 1500
   Val Pro  Phe His Glu Glu Phe  Glu Ser Gly Thr Ala  Lys Lys Gly
       1505                 1510                 1515
   Ala Glu  Ser Val Thr Glu Arg  Asp Thr Glu Val Gly  His Gln Ala
       1520                 1525                 1530
   His Glu  His Thr Glu Pro Val  Ser Leu Phe Pro Glu  Glu Ser Ser
       1535                 1540                 1545
   Gly Glu  Ile Ala Ile Asp Gln  Glu Ser Gln Lys Ile  Ala Phe Ala
       1550                 1555                 1560
   Arg Ala  Thr Glu Val Thr Phe  Gly Glu Glu Val Glu  Lys Ser Thr
       1565                 1570                 1575
   Ser Val  Thr Tyr Thr Pro Thr  Ile Val Pro Ser Ser  Ala Ser Ala
       1580                 1585                 1590
   Tyr Val  Ser Glu Glu Glu Ala  Val Thr Leu Ile Gly  Asn Pro Trp
       1595                 1600                 1605
   Pro Asp  Asp Leu Leu Ser Thr  Lys Glu Ser Trp Val  Glu Ala Thr
       1610                 1615                 1620
   Pro Arg  Gln Val Val Glu Leu  Ser Gly Ser Ser Ser  Ile Pro Ile
       1625                 1630                 1635
   Thr Glu  Gly Ser Gly Glu Ala  Glu Glu Asp Glu Asp  Thr Met Phe
       1640                 1645                 1650
   Thr Met  Val Thr Asp Leu Ser  Gln Arg Asn Thr Thr  Asp Thr Leu
       1655                 1660                 1665
   Ile Thr Leu Asp Thr Ser Arg Ile Ile Thr Glu Ser Phe Phe Glu
   1670 1675 1680
   Val Pro Ala Thr Thr Ile Tyr Pro Val Ser Glu Gln Pro Ser Ala
   1685 1690 1695
   Lys Val Val Pro Thr Lys Phe Val Ser Glu Thr Asp Thr Ser Glu
   1700 1705 1710
   Trp Ile Ser Ser Thr Thr Val Glu Glu Lys Lys Arg Lys Glu Glu
   1715 1720 1725
   Glu Gly Thr Thr Gly Thr Ala Ser Thr Phe Glu Val Tyr Ser Ser
   1730 1735 1740
   Thr Gln Arg Ser Asp Gln Leu Ile Leu Pro Phe Glu Leu Glu Ser
   1745 1750 1755
   Pro Asn Val Ala Thr Ser Ser Asp Ser Gly Thr Arg Lys Ser Phe
   1760 1765 1770
   Met Ser Leu Thr Thr Pro Thr Gln Ser Glu Arg Glu Met Thr Asp
   1775 1780 1785
   Ser Thr Pro Val Phe Thr Glu Thr Asn Thr Leu Glu Asn Leu Gly
   1790 1795 1800
   Ala Gln Thr Thr Glu His Ser Ser Ile His Gln Pro Gly Val Gln
   1805 1810 1815
   Glu Gly Leu Thr Thr Leu Pro Arg Ser Pro Ala Ser Val Phe Met
   1820 1825 1830
   Glu Gln Gly Ser Gly Glu Ala Ala Ala Asp Pro Glu Thr Thr Thr
   1835 1840 1845
   Val Ser Ser Phe Ser Leu Asn Val Glu Tyr Ala Ile Gln Ala Glu
   1850 1855 1860
   Lys Glu Val Ala Gly Thr Leu Ser Pro His Val Glu Thr Thr Phe
   1865 1870 1875
   Ser Thr Glu Pro Thr Gly Leu Val Leu Ser Thr Val Met Asp Arg
   1880 1885 1890
   Val Val Ala Glu Asn Ile Thr Gln Thr Ser Arg Glu Ile Val Ile
   1895 1900 1905
   Ser Glu Arg Leu Gly Glu Pro Asn Tyr Gly Ala Glu Ile Arg Gly
       1910                1915                1920
   Phe Ser Thr Gly Phe Pro Leu Glu Glu Asp Phe Ser Gly Asp Phe
       1925                1930                1935
   Arg Glu Tyr Ser Thr Val Ser His Pro Ile Ala Lys Glu Glu Thr
       1940                1945                1950
   Val Met Met Glu Gly Ser Gly Asp Ala Ala Phe Arg Asp Thr Gln
       1955                1960                1965
   Thr Ser Pro Ser Thr Val Pro Thr Ser Val His Ile Ser His Ile
       1970                1975                1980
   Ser Asp Ser Glu Gly Pro Ser Ser Thr Met Val Ser Thr Ser Ala
       1985                1990                1995
   Phe Pro Trp Glu Glu Phe Thr Ser Ser Ala Glu Gly Ser Gly Glu
       2000                2005                2010
   Gln Leu Val Thr Val Ser Ser Ser Val Val Pro Val Leu Pro Ser
       2015                2020                2025
   Ala Val Gln Lys Phe Ser Gly Thr Ala Ser Ser Ile Ile Asp Glu
       2030                2035                2040
   Gly Leu Gly Glu Val Gly Thr Val Asn Glu Ile Asp Arg Arg Ser
       2045                2050                2055
   Thr Ile Leu Pro Thr Ala Glu Val Glu Gly Thr Lys Ala Pro Val
       2060                2065                2070
   Glu Lys Glu Glu Val Lys Val Ser Gly Thr Val Ser Thr Asn Phe
       2075                2080                2085
   Pro Gln Thr Ile Glu Pro Ala Lys Leu Trp Ser Arg Gln Glu Val
       2090                2095                2100
   Asn Pro Val Arg Gln Glu Ile Glu Ser Glu Thr Thr Ser Glu Glu
       2105                2110                2115
   Gln Ile Gln Glu Glu Lys Ser Phe Glu Ser Pro Gln Asn Ser Pro
       2120                2125                2130
   Ala Thr Glu Gln Thr Ile Phe Asp Ser Gln Thr Phe Thr Glu Thr
       2135                2140                2145
   Glu Leu Lys Thr Thr Asp Tyr Ser Val Leu Thr Thr Lys Lys Thr
   2150 2155 2160
   Tyr Ser Asp Asp Lys Glu Met Lys Glu Glu Asp Thr Ser Leu Val
   2165 2170 2175
   Asn Met Ser Thr Pro Asp Pro Asp Ala Asn Gly Leu Glu Ser Tyr
   2180 2185 2190
   Thr Thr Leu Pro Glu Ala Thr Glu Lys Ser His Phe Phe Leu Ala
   2195 2200 2205
   Thr Ala Leu Val Thr Glu Ser Ile Pro Ala Glu His Val Val Thr
   2210 2215 2220
   Asp Ser Pro Ile Lys Lys Glu Glu Ser Thr Lys His Phe Pro Lys
   2225 2230 2235
   Gly Met Arg Pro Thr Ile Gln Glu Ser Asp Thr Glu Leu Leu Phe
   2240 2245 2250
   Ser Gly Leu Gly Ser Gly Glu Glu Val Leu Pro Thr Leu Pro Thr
   2255 2260 2265
   Glu Ser Val Asn Phe Thr Glu Val Glu Gln Ile Asn Asn Thr Leu
   2270 2275 2280
   Tyr Pro His Thr Ser Gln Val Glu Ser Thr Ser Ser Asp Lys Ile
   2285 2290 2295
   Glu Asp Phe Asn Arg Met Glu Asn Val Ala Lys Glu Val Gly Pro
   2300 2305 2310
   Leu Val Ser Gln Thr Asp Ile Phe Glu Gly Ser Gly Ser Val Thr
   2315 2320 2325
   Ser Thr Thr Leu Ile Glu Ile Leu Ser Asp Thr Gly Ala Glu Gly
   2330 2335 2340
   Pro Thr Val Ala Pro Leu Pro Phe Ser Thr Asp Ile Gly His Pro
   2345 2350 2355
   Gln Asn Gln Thr Val Arg Trp Ala Glu Glu Ile Gln Thr Ser Arg
   2360 2365 2370
   Pro Gln Thr Ile Thr Glu Gln Asp Ser Asn Lys Asn Ser Ser Thr
   2375 2380 2385
   Ala Glu Ile Asn Glu Thr Thr Thr Ser Ser Thr Asp Phe Leu Ala
   2390 2395 2400
   Arg Ala Tyr Gly Phe Glu Met Ala Lys Glu Phe Val Thr Ser Ala
   2405 2410 2415
   Pro Lys Pro Ser Asp Leu Tyr Tyr Glu Pro Ser Gly Glu Gly Ser
   2420 2425 2430
   Gly Glu Val Asp Ile Val Asp Ser Phe His Thr Ser Ala Thr Thr
   2435 2440 2445
   Gln Ala Thr Arg Gln Glu Ser Ser Thr Thr Phe Val Ser Asp Gly
   2450 2455 2460
   Ser Leu Glu Lys His Pro Glu Val Pro Ser Ala Lys Ala Val Thr
   2465 2470 2475
   Ala Asp Gly Phe Pro Thr Val Ser Val Met Leu Pro Leu His Ser
   2480 2485 2490
   Glu Gln Asn Lys Ser Ser Pro Asp Pro Thr Ser Thr Leu Ser Asn
   2495 2500 2505
   Thr Val Ser Tyr Glu Arg Ser Thr Asp Gly Ser Phe Gln Asp Arg
   2510 2515 2520
   Phe Arg Glu Phe Glu Asp Ser Thr Leu Lys Pro Asn Arg Lys Lys
   2525 2530 2535
   Pro Thr Glu Asn Ile Ile Ile Asp Leu Asp Lys Glu Asp Lys Asp
   2540 2545 2550
   Leu Ile Leu Thr Ile Thr Glu Ser Thr Ile Leu Glu Ile Leu Pro
   2555 2560 2565
   Glu Leu Thr Ser Asp Lys Asn Thr Ile Ile Asp Ile Asp His Thr
   2570 2575 2580
   Lys Pro Val Tyr Glu Asp Ile Leu Gly Met Gln Thr Asp Ile Asp
   2585 2590 2595
   Thr Glu Val Pro Ser Glu Pro His Asp Ser Asn Asp Glu Ser Asn
   2600 2605 2610
   Asp Asp Ser Thr Gln Val Gln Glu Ile Tyr Glu Ala Ala Val Asn
   2615 2620 2625
   Leu Ser Leu Thr Glu Glu Thr Phe Glu Gly Ser Ala Asp Val Leu
   2630 2635 2640
   Ala Ser  Tyr Thr Gln Ala Thr  His Asp Glu Ser Met  Thr Tyr Glu
       2645                 2650                 2655
   Asp Arg  Ser Gln Leu Asp His  Met Gly Phe His Phe  Thr Thr Gly
       2660                 2665                 2670
   Ile Pro  Ala Pro Ser Thr Glu  Thr Glu Leu Asp Val  Leu Leu Pro
       2675                 2680                 2685
   Thr Ala  Thr Ser Leu Pro Ile  Pro Arg Lys Ser Ala  Thr Val Ile
       2690                 2695                 2700
   Pro Glu  Ile Glu Gly Ile Lys  Ala Glu Ala Lys Ala  Leu Asp Asp
       2705                 2710                 2715
   Met Phe  Glu Ser Ser Thr Leu  Ser Asp Gly Gln Ala  Ile Ala Asp
       2720                 2725                 2730
   Gln Ser  Glu Ile Ile Pro Thr  Leu Gly Gln Phe Glu  Arg Thr Gln
       2735                 2740                 2745
   Glu Glu  Tyr Glu Asp Lys Lys  His Ala Gly Pro Ser  Phe Gln Pro
       2750                 2755                 2760
   Glu Phe  Ser Ser Gly Ala Glu  Glu Ala Leu Val Asp  His Thr Pro
       2765                 2770                 2775
   Tyr Leu  Ser Ile Ala Thr Thr  His Leu Met Asp Gln  Ser Val Thr
       2780                 2785                 2790
   Glu Val  Pro Asp Val Met Glu  Gly Ser Asn Pro Pro  Tyr Tyr Thr
       2795                 2800                 2805
   Asp Thr  Thr Leu Ala Val Ser  Thr Phe Ala Lys Leu  Ser Ser Gln
       2810                 2815                 2820
   Thr Pro  Ser Ser Pro Leu Thr  Ile Tyr Ser Gly Ser  Glu Ala Ser
       2825                 2830                 2835
   Gly His  Thr Glu Ile Pro Gln  Pro Ser Ala Leu Pro  Gly Ile Asp
       2840                 2845                 2850
   Val Gly  Ser Ser Val Met Ser  Pro Gln Asp Ser Phe  Lys Glu Ile
       2855                 2860                 2865
   His Val  Asn Ile Glu Ala Thr  Phe Lys Pro Ser Ser  Glu Glu Tyr
       2870                 2875                 2880
   Leu His Ile Thr Glu Pro Pro Ser Leu Ser Pro Asp Thr Lys Leu
   2885 2890 2895
   Glu Pro Ser Glu Asp Asp Gly Lys Pro Glu Leu Leu Glu Glu Met
   2900 2905 2910
   Glu Ala Ser Pro Thr Glu Leu Ile Ala Val Glu Gly Thr Glu Ile
   2915 2920 2925
   Leu Gln Asp Phe Gln Asn Lys Thr Asp Gly Gln Val Ser Gly Glu
   2930 2935 2940
   Ala Ile Lys Met Phe Pro Thr Ile Lys Thr Pro Glu Ala Gly Thr
   2945 2950 2955
   Val Ile Thr Thr Ala Asp Glu Ile Glu Leu Glu Gly Ala Thr Gln
   2960 2965 2970
   Trp Pro His Ser Thr Ser Ala Ser Ala Thr Tyr Gly Val Glu Ala
   2975 2980 2985
   Gly Val Val Pro Trp Leu Ser Pro Gln Thr Ser Glu Arg Pro Thr
   2990 2995 3000
   Leu Ser Ser Ser Pro Glu Ile Asn Pro Glu Thr Gln Ala Ala Leu
   3005 3010 3015
   Ile Arg Gly Gln Asp Ser Thr Ile Ala Ala Ser Glu Gln Gln Val
   3020 3025 3030
   Ala Ala Arg Ile Leu Asp Ser Asn Asp Gln Ala Thr Val Asn Pro
   3035 3040 3045
   Val Glu Phe Asn Thr Glu Val Ala Thr Pro Pro Phe Ser Leu Leu
   3050 3055 3060
   Glu Thr Ser Asn Glu Thr Asp Phe Leu Ile Gly Ile Asn Glu Glu
   3065 3070 3075
   Ser Val Glu Gly Thr Ala Ile Tyr Leu Pro Gly Pro Asp Arg Cys
   3080 3085 3090
   Lys Met Asn Pro Cys Leu Asn Gly Gly Thr Cys Tyr Pro Thr Glu
   3095 3100 3105
   Thr Ser Tyr Val Cys Thr Cys Val Pro Gly Tyr Ser Gly Asp Gln
   3110 3115 3120
   Cys Glu Leu Asp Phe Asp Glu Cys His Ser Asn Pro Cys Arg Asn
   3125 3130 3135
   Gly Ala Thr Cys Val Asp Gly Phe Asn Thr Phe Arg Cys Leu Cys
   3140 3145 3150
   Leu Pro Ser Tyr Val Gly Ala Leu Cys Glu Gln Asp Thr Glu Thr
   3155 3160 3165
   Cys Asp Tyr Gly Trp His Lys Phe Gln Gly Gln Cys Tyr Lys Tyr
   3170 3175 3180
   Phe Ala His Arg Arg Thr Trp Asp Ala Ala Glu Arg Glu Cys Arg
   3185 3190 3195
   Leu Gln Gly Ala His Leu Thr Ser Ile Leu Ser His Glu Glu Gln
   3200 3205 3210
   Met Phe Val Asn Arg Val Gly His Asp Tyr Gln Trp Ile Gly Leu
   3215 3220 3225
   Asn Asp Lys Met Phe Glu His Asp Phe Arg Trp Thr Asp Gly Ser
   3230 3235 3240
   Thr Leu Gln Tyr Glu Asn Trp Arg Pro Asn Gln Pro Asp Ser Phe
   3245 3250 3255
   Phe Ser Ala Gly Glu Asp Cys Val Val Ile Ile Trp His Glu Asn
   3260 3265 3270
   Gly Gln Trp Asn Asp Val Pro Cys Asn Tyr His Leu Thr Tyr Thr
   3275 3280 3285
   Cys Lys Lys Gly Thr Val Ala Cys Gly Gln Pro Pro Val Val Glu
   3290 3295 3300
   Asn Ala Lys Thr Phe Gly Lys Met Lys Pro Arg Tyr Glu Ile Asn
   3305 3310 3315
   Ser Leu Ile Arg Tyr His Cys Lys Asp Gly Phe Ile Gln Arg His
   3320 3325 3330
   Leu Pro Thr Ile Arg Cys Leu Gly Asn Gly Arg Trp Ala Ile Pro
   3335 3340 3345
   Lys Ile Thr Cys Met Asn Pro Ser Ala Tyr Gln Arg Thr Tyr Ser
   3350 3355 3360
   Met Lys Tyr Phe Lys Asn Ser Ser Ser Ala Lys Asp Asn Ser Ile
   3365 3370 3375
   Asn Thr Ser Lys His Asp His Arg Trp Ser Arg Arg Trp Gln Glu
       3380                3385                3390
   Ser Arg Arg
       3395
<210> 2
   <211> 338
   <212> PRT
   <213> Homo sapiens
<400> 2
   Met Ser Leu Ser Ala Phe Thr Leu Phe Leu Ala Leu Ile Gly Gly Thr
   1               5                   10                  15
   Ser Gly Gln Tyr Tyr Asp Tyr Asp Phe Pro Leu Ser Ile Tyr Gly Gln
               20                  25                  30
   Ser Ser Pro Asn Cys Ala Pro Glu Cys Asn Cys Pro Glu Ser Tyr Pro
           35                  40                  45
   Ser Ala Met Tyr Cys Asp Glu Leu Lys Leu Lys Ser Val Pro Met Val
       50                  55                  60
   Pro Pro Gly Ile Lys Tyr Leu Tyr Leu Arg Asn Asn Gln Ile Asp His
   65                  70                  75                  80
   Ile Asp Glu Lys Ala Phe Glu Asn Val Thr Asp Leu Gln Trp Leu Ile
                   85                  90                  95
   Leu Asp His Asn Leu Leu Glu Asn Ser Lys Ile Lys Gly Arg Val Phe
               100                 105                 110
   Ser Lys Leu Lys Gln Leu Lys Lys Leu His Ile Asn His Asn Asn Leu
           115                 120                 125
   Thr Glu Ser Val Gly Pro Leu Pro Lys Ser Leu Glu Asp Leu Gln Leu
       130                 135                 140
   Thr His Asn Lys Ile Thr Lys Leu Gly Ser Phe Glu Gly Leu Val Asn
   145                 150                 155                 160
   Leu Thr Phe Ile His Leu Gln His Asn Arg Leu Lys Glu Asp Ala Val
                   165                 170                 175
   Ser Ala Ala Phe Lys Gly Leu Lys Ser Leu Glu Tyr Leu Asp Leu Ser
               180                 185                 190
   Phe Asn Gln Ile Ala Arg Leu Pro Ser Gly Leu Pro Val Ser Leu Leu
           195                 200                 205
   Thr Leu Tyr Leu Asp Asn Asn Lys Ile Ser Asn Ile Pro Asp Glu Tyr
       210                 215                 220
   Phe Lys Arg Phe Asn Ala Leu Gln Tyr Leu Arg Leu Ser His Asn Glu
   225                 230                 235                 240
   Leu Ala Asp Ser Gly Ile Pro Gly Asn Ser Phe Asn Val Ser Ser Leu
                   245                 250                 255
   Val Glu Leu Asp Leu Ser Tyr Asn Lys Leu Lys Asn Ile Pro Thr Val
               260                 265                 270
   Asn Glu Asn Leu Glu Asn Tyr Tyr Leu Glu Val Asn Gln Leu Glu Lys
           275                 280                 285
   Phe Asp Ile Lys Ser Phe Cys Lys Ile Leu Gly Pro Leu Ser Tyr Ser
       290                 295                 300
   Lys Ile Lys His Leu Arg Leu Asp Gly Asn Arg Ile Ser Glu Thr Ser
   305                 310                 315                 320
   Leu Pro Pro Asp Met Tyr Glu Cys Leu Arg Val Ala Asn Glu Val Thr
                   325                 330                 335
   Leu Asn
<210> 3
   <211> 4391
   <212> PRT
   <213> Homo sapiens
<400> 3
   Met Gly Trp Arg Ala Pro Gly Ala Leu Leu Leu Ala Leu Leu Leu His
   1               5                   10                  15
   Gly Arg Leu Leu Ala Val Thr His Gly Leu Arg Ala Tyr Asp Gly Leu
               20                  25                  30
   Ser Leu Pro Glu Asp Ile Glu Thr Val Thr Ala Ser Gln Met Arg Trp
           35                  40                  45
   Thr His Ser Tyr Leu Ser Asp Asp Glu Asp Met Leu Ala Asp Ser Ile
       50                  55                  60
   Ser Gly Asp Asp Leu Gly Ser Gly Asp Leu Gly Ser Gly Asp Phe Gln
   65                  70                  75                  80
   Met Val Tyr Phe Arg Ala Leu Val Asn Phe Thr Arg Ser Ile Glu Tyr
   85 90 95
   Ser Pro Gln Leu Glu Asp Ala Gly Ser Arg Glu Phe Arg Glu Val Ser
   100 105 110
   Glu Ala Val Val Asp Thr Leu Glu Ser Glu Tyr Leu Lys Ile Pro Gly
   115 120 125
   Asp Gln Val Val Ser Val Val Phe Ile Lys Glu Leu Asp Gly Trp Val
   130 135 140
   Phe Val Glu Leu Asp Val Gly Ser Glu Gly Asn Ala Asp Gly Ala Gln
   145 150 155 160
   Ile Gln Glu Met Leu Leu Arg Val Ile Ser Ser Gly Ser Val Ala Ser
   165 170 175
   Tyr Val Thr Ser Pro Gln Gly Phe Gln Phe Arg Arg Leu Gly Thr Val
   180 185 190
   Pro Gln Phe Pro Arg Ala Cys Thr Glu Ala Glu Phe Ala Cys His Ser
   195 200 205
   Tyr Asn Glu Cys Val Ala Leu Glu Tyr Arg Cys Asp Arg Arg Pro Asp
   210 215 220
   Cys Arg Asp Met Ser Asp Glu Leu Asn Cys Glu Glu Pro Val Leu Gly
   225 230 235 240
   Ile Ser Pro Thr Phe Ser Leu Leu Val Glu Thr Thr Ser Leu Pro Pro
   245 250 255
   Arg Pro Glu Thr Thr Ile Met Arg Gln Pro Pro Val Thr His Ala Pro
   260 265 270
   Gln Pro Leu Leu Pro Gly Ser Val Arg Pro Leu Pro Cys Gly Pro Gln
   275 280 285
   Glu Ala Ala Cys Arg Asn Gly His Cys Ile Pro Arg Asp Tyr Leu Cys
   290 295 300
   Asp Gly Gln Glu Asp Cys Glu Asp Gly Ser Asp Glu Leu Asp Cys Gly
   305 310 315 320
   Pro Pro Pro Pro Cys Glu Pro Asn Glu Phe Pro Cys Gly Asn Gly His
   325 330 335
   Cys Ala Leu Lys Leu Trp Arg Cys Asp Gly Asp Phe Asp Cys Glu Asp
               340                 345                 350
   Arg Thr Asp Glu Ala Asn Cys Pro Thr Lys Arg Pro Glu Glu Val Cys
           355                 360                 365
   Gly Pro Thr Gln Phe Arg Cys Val Ser Thr Asn Met Cys Ile Pro Ala
       370                 375                 380
   Ser Phe His Cys Asp Glu Glu Ser Asp Cys Pro Asp Arg Ser Asp Glu
   385                 390                 395                 400
   Phe Gly Cys Met Pro Pro Gln Val Val Thr Pro Pro Arg Glu Ser Ile
                   405                 410                 415
   Gln Ala Ser Arg Gly Gln Thr Val Thr Phe Thr Cys Val Ala Ile Gly
               420                 425                 430
   Val Pro Thr Pro Ile Ile Asn Trp Arg Leu Asn Trp Gly His Ile Pro
           435                 440                 445
   Ser His Pro Arg Val Thr Val Thr Ser Glu Gly Gly Arg Gly Thr Leu
       450                 455                 460
   Ile Ile Arg Asp Val Lys Glu Ser Asp Gln Gly Ala Tyr Thr Cys Glu
   465                 470                 475                 480
   Ala Met Asn Ala Arg Gly Met Val Phe Gly Ile Pro Asp Gly Val Leu
                   485                 490                 495
   Glu Leu Val Pro Gln Arg Gly Pro Cys Pro Asp Gly His Phe Tyr Leu
               500                 505                 510
   Glu His Ser Ala Ala Cys Leu Pro Cys Phe Cys Phe Gly Ile Thr Ser
           515                 520                 525
   Val Cys Gln Ser Thr Arg Arg Phe Arg Asp Gln Ile Arg Leu Arg Phe
       530                 535                 540
   Asp Gln Pro Asp Asp Phe Lys Gly Val Asn Val Thr Met Pro Ala Gln
   545                 550                 555                 560
   Pro Gly Thr Pro Pro Leu Ser Ser Thr Gln Leu Gln Ile Asp Pro Ser
                   565                 570                 575
   Leu His Glu Phe Gln Leu Val Asp Leu Ser Arg Arg Phe Leu Val His
               580                 585                 590
   Asp Ser Phe Trp Ala Leu Pro Glu Gln Phe Leu Gly Asn Lys Val Asp
   595 600 605
   Ser Tyr Gly Gly Ser Leu Arg Tyr Asn Val Arg Tyr Glu Leu Ala Arg
   610 615 620
   Gly Met Leu Glu Pro Val Gln Arg Pro Asp Val Val Leu Val Gly Ala
   625 630 635 640
   Gly Tyr Arg Leu Leu Ser Arg Gly His Thr Pro Thr Gln Pro Gly Ala
   645 650 655
   Leu Asn Gln Arg Gln Val Gln Phe Ser Glu Glu His Trp Val His Glu
   660 665 670
   Ser Gly Arg Pro Val Gln Arg Ala Glu Leu Leu Gln Val Leu Gln Ser
   675 680 685
   Leu Glu Ala Val Leu Ile Gln Thr Val Tyr Asn Thr Lys Met Ala Ser
   690 695 700
   Val Gly Leu Ser Asp Ile Ala Met Asp Thr Thr Val Thr His Ala Thr
   705 710 715 720
   Ser His Gly Arg Ala His Ser Val Glu Glu Cys Arg Cys Pro Ile Gly
   725 730 735
   Tyr Ser Gly Leu Ser Cys Glu Ser Cys Asp Ala His Phe Thr Arg Val
   740 745 750
   Pro Gly Gly Pro Tyr Leu Gly Thr Cys Ser Gly Cys Ser Cys Asn Gly
   755 760 765
   His Ala Ser Ser Cys Asp Pro Val Tyr Gly His Cys Leu Asn Cys Gln
   770 775 780
   His Asn Thr Glu Gly Pro Gln Cys Asn Lys Cys Lys Ala Gly Phe Phe
   785 790 795 800
   Gly Asp Ala Met Lys Ala Thr Ala Thr Ser Cys Arg Pro Cys Pro Cys
   805 810 815
   Pro Tyr Ile Asp Ala Ser Arg Arg Phe Ser Asp Thr Cys Phe Leu Asp
   820 825 830
   Thr Asp Gly Gln Ala Thr Cys Asp Ala Cys Ala Pro Gly Tyr Thr Gly
   835 840 845
   Arg Arg Cys Glu Ser Cys Ala Pro Gly Tyr Glu Gly Asn Pro Ile Gln
   850 855 860
   Pro Gly Gly Lys Cys Arg Pro Val Asn Gln Glu Ile Val Arg Cys Asp
   865 870 875 880
   Glu Arg Gly Ser Met Gly Thr Ser Gly Glu Ala Cys Arg Cys Lys Asn
   885 890 895
   Asn Val Val Gly Arg Leu Cys Asn Glu Cys Ala Asp Gly Ser Phe His
   900 905 910
   Leu Ser Thr Arg Asn Pro Asp Gly Cys Leu Lys Cys Phe Cys Met Gly
   915 920 925
   Val Ser Arg His Cys Thr Ser Ser Ser Trp Ser Arg Ala Gln Leu His
   930 935 940
   Gly Ala Ser Glu Glu Pro Gly His Phe Ser Leu Thr Asn Ala Ala Ser
   945 950 955 960
   Thr His Thr Thr Asn Glu Gly Ile Phe Ser Pro Thr Pro Gly Glu Leu
   965 970 975
   Gly Phe Ser Ser Phe His Arg Leu Leu Ser Gly Pro Tyr Phe Trp Ser
   980 985 990
   Leu Pro Ser Arg Phe Leu Gly Asp Lys Val Thr Ser Tyr Gly Gly Glu
   995 1000 1005
   Leu Arg Phe Thr Val Thr Gln Arg Ser Gln Pro Gly Ser Thr Pro
   1010 1015 1020
   Leu His Gly Gln Pro Leu Val Val Leu Gln Gly Asn Asn Ile Ile
   1025 1030 1035
   Leu Glu His His Val Ala Gln Glu Pro Ser Pro Gly Gln Pro Ser
   1040 1045 1050
   Thr Phe Ile Val Pro Phe Arg Glu Gln Ala Trp Gln Arg Pro Asp
   1055 1060 1065
   Gly Gln Pro Ala Thr Arg Glu His Leu Leu Met Ala Leu Ala Gly
   1070 1075 1080
   Ile Asp Thr Leu Leu Ile Arg Ala Ser Tyr Ala Gln Gln Pro Ala
   1085 1090 1095
   Glu Ser Arg Val Ser Gly Ile Ser Met Asp Val Ala Val Pro Glu
   1100 1105 1110
   Glu Thr  Gly Gln Asp Pro Ala  Leu Glu Val Glu Gln  Cys Ser Cys
       1115                 1120                 1125
   Pro Pro  Gly Tyr Arg Gly Pro  Ser Cys Gln Asp Cys  Asp Thr Gly
       1130                 1135                 1140
   Tyr Thr  Arg Thr Pro Ser Gly  Leu Tyr Leu Gly Thr  Cys Glu Arg
       1145                 1150                 1155
   Cys Ser  Cys His Gly His Ser  Glu Ala Cys Glu Pro  Glu Thr Gly
       1160                 1165                 1170
   Ala Cys  Gln Gly Cys Gln His  His Thr Glu Gly Pro  Arg Cys Glu
       1175                 1180                 1185
   Gln Cys  Gln Pro Gly Tyr Tyr  Gly Asp Ala Gln Arg  Gly Thr Pro
       1190                 1195                 1200
   Gln Asp  Cys Gln Leu Cys Pro  Cys Tyr Gly Asp Pro  Ala Ala Gly
       1205                 1210                 1215
   Gln Ala  Ala His Thr Cys Phe  Leu Asp Thr Asp Gly  His Pro Thr
       1220                 1225                 1230
   Cys Asp  Ala Cys Ser Pro Gly  His Ser Gly Arg His  Cys Glu Arg
       1235                 1240                 1245
   Cys Ala  Pro Gly Tyr Tyr Gly  Asn Pro Ser Gln Gly  Gln Pro Cys
       1250                 1255                 1260
   Gln Arg  Asp Ser Gln Val Pro  Gly Pro Ile Gly Cys  Asn Cys Asp
       1265                 1270                 1275
   Pro Gln  Gly Ser Val Ser Ser  Gln Cys Asp Ala Ala  Gly Gln Cys
       1280                 1285                 1290
   Gln Cys  Lys Ala Gln Val Glu  Gly Leu Thr Cys Ser  His Cys Arg
       1295                 1300                 1305
   Pro His  His Phe His Leu Ser  Ala Ser Asn Pro Asp  Gly Cys Leu
       1310                 1315                 1320
   Pro Cys  Phe Cys Met Gly Ile  Thr Gln Gln Cys Ala  Ser Ser Ala
       1325                 1330                 1335
   Tyr Thr  Arg His Leu Ile Ser  Thr His Phe Ala Pro  Gly Asp Phe
       1340                 1345                 1350
   Gln Gly  Phe Ala Leu Val Asn  Pro Gln Arg Asn Ser  Arg Leu Thr
       1355                 1360                 1365
   Gly Glu  Phe Thr Val Glu Pro  Val Pro Glu Gly Ala  Gln Leu Ser
       1370                 1375                 1380
   Phe Gly  Asn Phe Ala Gln Leu  Gly His Glu Ser Phe  Tyr Trp Gln
       1385                 1390                 1395
   Leu Pro  Glu Thr Tyr Gln Gly  Asp Lys Val Ala Ala  Tyr Gly Gly
       1400                 1405                 1410
   Lys Leu  Arg Tyr Thr Leu Ser  Tyr Thr Ala Gly Pro  Gln Gly Ser
       1415                 1420                 1425
   Pro Leu  Ser Asp Pro Asp Val  Gln Ile Thr Gly Asn  Asn Ile Met
       1430                 1435                 1440
   Leu Val  Ala Ser Gln Pro Ala  Leu Gln Gly Pro Glu  Arg Arg Ser
       1445                 1450                 1455
   Tyr Glu  Ile Met Phe Arg Glu  Glu Phe Trp Arg Arg  Pro Asp Gly
       1460                 1465                 1470
   Gln Pro  Ala Thr Arg Glu His  Leu Leu Met Ala Leu  Ala Asp Leu
       1475                 1480                 1485
   Asp Glu  Leu Leu Ile Arg Ala  Thr Phe Ser Ser Val  Pro Leu Val
       1490                 1495                 1500
   Ala Ser  Ile Ser Ala Val Ser  Leu Glu Val Ala Gln  Pro Gly Pro
       1505                 1510                 1515
   Ser Asn  Arg Pro Arg Ala Leu  Glu Val Glu Glu Cys  Arg Cys Pro
       1520                 1525                 1530
   Pro Gly  Tyr Ile Gly Leu Ser  Cys Gln Asp Cys Ala  Pro Gly Tyr
       1535                 1540                 1545
   Thr Arg  Thr Gly Ser Gly Leu  Tyr Leu Gly His Cys  Glu Leu Cys
       1550                 1555                 1560
   Glu Cys  Asn Gly His Ser Asp  Leu Cys His Pro Glu  Thr Gly Ala
       1565                 1570                 1575
   Cys Ser  Gln Cys Gln His Asn  Ala Ala Gly Glu Phe  Cys Glu Leu
       1580                 1585                 1590
   Cys Ala  Pro Gly Tyr Tyr Gly  Asp Ala Thr Ala Gly  Thr Pro Glu
       1595                 1600                 1605
   Asp Cys  Gln Pro Cys Ala Cys  Pro Leu Thr Asn Pro  Glu Asn Met
       1610                 1615                 1620
   Phe Ser  Arg Thr Cys Glu Ser  Leu Gly Ala Gly Gly  Tyr Arg Cys
       1625                 1630                 1635
   Thr Ala  Cys Glu Pro Gly Tyr  Thr Gly Gln Tyr Cys  Glu Gln Cys
       1640                 1645                 1650
   Gly Pro  Gly Tyr Val Gly Asn  Pro Ser Val Gln Gly  Gly Gln Cys
       1655                 1660                 1665
   Leu Pro  Glu Thr Asn Gln Ala  Pro Leu Val Val Glu  Val His Pro
       1670                 1675                 1680
   Ala Arg  Ser Ile Val Pro Gln  Gly Gly Ser His Ser  Leu Arg Cys
       1685                 1690                 1695
   Gln Val  Ser Gly Ser Pro Pro  His Tyr Phe Tyr Trp  Ser Arg Glu
       1700                 1705                 1710
   Asp Gly  Arg Pro Val Pro Ser  Gly Thr Gln Gln Arg  His Gln Gly
       1715                 1720                 1725
   Ser Glu  Leu His Phe Pro Ser  Val Gln Pro Ser Asp  Ala Gly Val
       1730                 1735                 1740
   Tyr Ile  Cys Thr Cys Arg Asn  Leu His Gln Ser Asn  Thr Ser Arg
       1745                 1750                 1755
   Ala Glu  Leu Leu Val Thr Glu  Ala Pro Ser Lys Pro  Ile Thr Val
       1760                 1765                 1770
   Thr Val  Glu Glu Gln Arg Ser  Gln Ser Val Arg Pro  Gly Ala Asp
       1775                 1780                 1785
   Val Thr  Phe Ile Cys Thr Ala  Lys Ser Lys Ser Pro  Ala Tyr Thr
       1790                 1795                 1800
   Leu Val  Trp Thr Arg Leu His  Asn Gly Lys Leu Pro  Thr Arg Ala
       1805                 1810                 1815
   Met Asp  Phe Asn Gly Ile Leu  Thr Ile Arg Asn Val  Gln Leu Ser
       1820                 1825                 1830
   Asp Ala  Gly Thr Tyr Val Cys  Thr Gly Ser Asn Met  Phe Ala Met
   1835 1840 1845
   Asp Gln Gly Thr Ala Thr Leu His Val Gln Ala Ser Gly Thr Leu
   1850 1855 1860
   Ser Ala Pro Val Val Ser Ile His Pro Pro Gln Leu Thr Val Gln
   1865 1870 1875
   Pro Gly Gln Leu Ala Glu Phe Arg Cys Ser Ala Thr Gly Ser Pro
   1880 1885 1890
   Thr Pro Thr Leu Glu Trp Thr Gly Gly Pro Gly Gly Gln Leu Pro
   1895 1900 1905
   Ala Lys Ala Gln Ile His Gly Gly Ile Leu Arg Leu Pro Ala Val
   1910 1915 1920
   Glu Pro Thr Asp Gln Ala Gln Tyr Leu Cys Arg Ala His Ser Ser
   1925 1930 1935
   Ala Gly Gln Gln Val Ala Arg Ala Val Leu His Val His Gly Gly
   1940 1945 1950
   Gly Gly Pro Arg Val Gln Val Ser Pro Glu Arg Thr Gln Val His
   1955 1960 1965
   Ala Gly Arg Thr Val Arg Leu Tyr Cys Arg Ala Ala Gly Val Pro
   1970 1975 1980
   Ser Ala Thr Ile Thr Trp Arg Lys Glu Gly Gly Ser Leu Pro Pro
   1985 1990 1995
   Gln Ala Arg Ser Glu Arg Thr Asp Ile Ala Thr Leu Leu Ile Pro
   2000 2005 2010
   Ala Ile Thr Thr Ala Asp Ala Gly Phe Tyr Leu Cys Val Ala Thr
   2015 2020 2025
   Ser Pro Ala Gly Thr Ala Gln Ala Arg Ile Gln Val Val Val Leu
   2030 2035 2040
   Ser Ala Ser Asp Ala Ser Pro Pro Pro Val Lys Ile Glu Ser Ser
   2045 2050 2055
   Ser Pro Ser Val Thr Glu Gly Gln Thr Leu Asp Leu Asn Cys Val
   2060 2065 2070
   Val Ala Gly Ser Ala His Ala Gln Val Thr Trp Tyr Arg Arg Gly
   2075 2080 2085
   Gly Ser Leu Pro Pro His Thr Gln Val His Gly Ser Arg Leu Arg
   2090 2095 2100
   Leu Pro Gln Val Ser Pro Ala Asp Ser Gly Glu Tyr Val Cys Arg
   2105 2110 2115
   Val Glu Asn Gly Ser Gly Pro Lys Glu Ala Ser Ile Thr Val Ser
   2120 2125 2130
   Val Leu His Gly Thr His Ser Gly Pro Ser Tyr Thr Pro Val Pro
   2135 2140 2145
   Gly Ser Thr Arg Pro Ile Arg Ile Glu Pro Ser Ser Ser His Val
   2150 2155 2160
   Ala Glu Gly Gln Thr Leu Asp Leu Asn Cys Val Val Pro Gly Gln
   2165 2170 2175
   Ala His Ala Gln Val Thr Trp His Lys Arg Gly Gly Ser Leu Pro
   2180 2185 2190
   Ala Arg His Gln Thr His Gly Ser Leu Leu Arg Leu His Gln Val
   2195 2200 2205
   Thr Pro Ala Asp Ser Gly Glu Tyr Val Cys His Val Val Gly Thr
   2210 2215 2220
   Ser Gly Pro Leu Glu Ala Ser Val Leu Val Thr Ile Glu Ala Ser
   2225 2230 2235
   Val Ile Pro Gly Pro Ile Pro Pro Val Arg Ile Glu Ser Ser Ser
   2240 2245 2250
   Ser Thr Val Ala Glu Gly Gln Thr Leu Asp Leu Ser Cys Val Val
   2255 2260 2265
   Ala Gly Gln Ala His Ala Gln Val Thr Trp Tyr Lys Arg Gly Gly
   2270 2275 2280
   Ser Leu Pro Ala Arg His Gln Val Arg Gly Ser Arg Leu Tyr Ile
   2285 2290 2295
   Phe Gln Ala Ser Pro Ala Asp Ala Gly Gln Tyr Val Cys Arg Ala
   2300 2305 2310
   Ser Asn Gly Met Glu Ala Ser Ile Thr Val Thr Val Thr Gly Thr
   2315 2320 2325
   Gln Gly  Ala Asn Leu Ala Tyr  Pro Ala Gly Ser Thr  Gln Pro Ile
       2330                 2335                 2340
   Arg Ile  Glu Pro Ser Ser Ser  Gln Val Ala Glu Gly  Gln Thr Leu
       2345                 2350                 2355
   Asp Leu  Asn Cys Val Val Pro  Gly Gln Ser His Ala  Gln Val Thr
       2360                 2365                 2370
   Trp His  Lys Arg Gly Gly Ser  Leu Pro Val Arg His  Gln Thr His
       2375                 2380                 2385
   Gly Ser  Leu Leu Arg Leu Tyr  Gln Ala Ser Pro Ala  Asp Ser Gly
       2390                 2395                 2400
   Glu Tyr  Val Cys Arg Val Leu  Gly Ser Ser Val Pro  Leu Glu Ala
       2405                 2410                 2415
   Ser Val  Leu Val Thr Ile Glu  Pro Ala Gly Ser Val  Pro Ala Leu
       2420                 2425                 2430
   Gly Val  Thr Pro Thr Val Arg  Ile Glu Ser Ser Ser  Ser Gln Val
       2435                 2440                 2445
   Ala Glu  Gly Gln Thr Leu Asp  Leu Asn Cys Leu Val  Ala Gly Gln
       2450                 2455                 2460
   Ala His  Ala Gln Val Thr Trp  His Lys Arg Gly Gly  Ser Leu Pro
       2465                 2470                 2475
   Ala Arg  His Gln Val His Gly  Ser Arg Leu Arg Leu  Leu Gln Val
       2480                 2485                 2490
   Thr Pro  Ala Asp Ser Gly Glu  Tyr Val Cys Arg Val  Val Gly Ser
       2495                 2500                 2505
   Ser Gly  Thr Gln Glu Ala Ser  Val Leu Val Thr Ile  Gln Gln Arg
       2510                 2515                 2520
   Leu Ser  Gly Ser His Ser Gln  Gly Val Ala Tyr Pro  Val Arg Ile
       2525                 2530                 2535
   Glu Ser  Ser Ser Ala Ser Leu  Ala Asn Gly His Thr  Leu Asp Leu
       2540                 2545                 2550
   Asn Cys  Leu Val Ala Ser Gln  Ala Pro His Thr Ile  Thr Trp Tyr
       2555                 2560                 2565
   Lys Arg Gly Gly Ser Leu Pro Ser Arg His Gln Ile Val Gly Ser
       2570                2575                2580
   Arg Leu Arg Ile Pro Gln Val Thr Pro Ala Asp Ser Gly Glu Tyr
       2585                2590                2595
   Val Cys His Val Ser Asn Gly Ala Gly Ser Arg Glu Thr Ser Leu
       2600                2605                2610
   Ile Val Thr Ile Gln Gly Ser Gly Ser Ser His Val Pro Ser Val
       2615                2620                2625
   Ser Pro Pro Ile Arg Ile Glu Ser Ser Ser Pro Thr Val Val Glu
       2630                2635                2640
   Gly Gln Thr Leu Asp Leu Asn Cys Val Val Ala Arg Gln Pro Gln
       2645                2650                2655
   Ala Ile Ile Thr Trp Tyr Lys Arg Gly Gly Ser Leu Pro Ser Arg
       2660                2665                2670
   His Gln Thr His Gly Ser His Leu Arg Leu His Gln Met Ser Val
       2675                2680                2685
   Ala Asp Ser Gly Glu Tyr Val Cys Arg Ala Asn Asn Asn Ile Asp
       2690                2695                2700
   Ala Leu Glu Ala Ser Ile Val Ile Ser Val Ser Pro Ser Ala Gly
       2705                2710                2715
   Ser Pro Ser Ala Pro Gly Ser Ser Met Pro Ile Arg Ile Glu Ser
       2720                2725                2730
   Ser Ser Ser His Val Ala Glu Gly Glu Thr Leu Asp Leu Asn Cys
       2735                2740                2745
   Val Val Pro Gly Gln Ala His Ala Gln Val Thr Trp His Lys Arg
       2750                2755                2760
   Gly Gly Ser Leu Pro Ser His His Gln Thr Arg Gly Ser Arg Leu
       2765                2770                2775
   Arg Leu His His Val Ser Pro Ala Asp Ser Gly Glu Tyr Val Cys
       2780                2785                2790
   Arg Val Met Gly Ser Ser Gly Pro Leu Glu Ala Ser Val Leu Val
       2795                2800                2805
   Thr Ile Glu Ala Ser Gly Ser Ser Ala Val His Val Pro Ala Pro
   2810 2815 2820
   Gly Gly Ala Pro Pro Ile Arg Ile Glu Pro Ser Ser Ser Arg Val
   2825 2830 2835
   Ala Glu Gly Gln Thr Leu Asp Leu Lys Cys Val Val Pro Gly Gln
   2840 2845 2850
   Ala His Ala Gln Val Thr Trp His Lys Arg Gly Gly Asn Leu Pro
   2855 2860 2865
   Ala Arg His Gln Val His Gly Pro Leu Leu Arg Leu Asn Gln Val
   2870 2875 2880
   Ser Pro Ala Asp Ser Gly Glu Tyr Ser Cys Gln Val Thr Gly Ser
   2885 2890 2895
   Ser Gly Thr Leu Glu Ala Ser Val Leu Val Thr Ile Glu Pro Ser
   2900 2905 2910
   Ser Pro Gly Pro Ile Pro Ala Pro Gly Leu Ala Gln Pro Ile Tyr
   2915 2920 2925
   Ile Glu Ala Ser Ser Ser His Val Thr Glu Gly Gln Thr Leu Asp
   2930 2935 2940
   Leu Asn Cys Val Val Pro Gly Gln Ala His Ala Gln Val Thr Trp
   2945 2950 2955
   Tyr Lys Arg Gly Gly Ser Leu Pro Ala Arg His Gln Thr His Gly
   2960 2965 2970
   Ser Gln Leu Arg Leu His Leu Val Ser Pro Ala Asp Ser Gly Glu
   2975 2980 2985
   Tyr Val Cys Arg Ala Ala Ser Gly Pro Gly Pro Glu Gln Glu Ala
   2990 2995 3000
   Ser Phe Thr Val Thr Val Pro Pro Ser Glu Gly Ser Ser Tyr Arg
   3005 3010 3015
   Leu Arg Ser Pro Val Ile Ser Ile Asp Pro Pro Ser Ser Thr Val
   3020 3025 3030
   Gln Gln Gly Gln Asp Ala Ser Phe Lys Cys Leu Ile His Asp Gly
   3035 3040 3045
   Ala Ala Pro Ile Ser Leu Glu Trp Lys Thr Arg Asn Gln Glu Leu
   3050 3055 3060
   Glu Asp Asn Val His Ile Ser Pro Asn Gly Ser Ile Ile Thr Ile
   3065 3070 3075
   Val Gly Thr Arg Pro Ser Asn His Gly Thr Tyr Arg Cys Val Ala
   3080 3085 3090
   Ser Asn Ala Tyr Gly Val Ala Gln Ser Val Val Asn Leu Ser Val
   3095 3100 3105
   His Gly Pro Pro Thr Val Ser Val Leu Pro Glu Gly Pro Val Trp
   3110 3115 3120
   Val Lys Val Gly Lys Ala Val Thr Leu Glu Cys Val Ser Ala Gly
   3125 3130 3135
   Glu Pro Arg Ser Ser Ala Arg Trp Thr Arg Ile Ser Ser Thr Pro
   3140 3145 3150
   Ala Lys Leu Glu Gln Arg Thr Tyr Gly Leu Met Asp Ser His Ala
   3155 3160 3165
   Val Leu Gln Ile Ser Ser Ala Lys Pro Ser Asp Ala Gly Thr Tyr
   3170 3175 3180
   Val Cys Leu Ala Gln Asn Ala Leu Gly Thr Ala Gln Lys Gln Val
   3185 3190 3195
   Glu Val Ile Val Asp Thr Gly Ala Met Ala Pro Gly Ala Pro Gln
   3200 3205 3210
   Val Gln Ala Glu Glu Ala Glu Leu Thr Val Glu Ala Gly His Thr
   3215 3220 3225
   Ala Thr Leu Arg Cys Ser Ala Thr Gly Ser Pro Ala Pro Thr Ile
   3230 3235 3240
   His Trp Ser Lys Leu Arg Ser Pro Leu Pro Trp Gln His Arg Leu
   3245 3250 3255
   Glu Gly Asp Thr Leu Ile Ile Pro Arg Val Ala Gln Gln Asp Ser
   3260 3265 3270
   Gly Gln Tyr Ile Cys Asn Ala Thr Ser Pro Ala Gly His Ala Glu
   3275 3280 3285
   Ala Thr Ile Ile Leu His Val Glu Ser Pro Pro Tyr Ala Thr Thr
   3290 3295 3300
   Val Pro Glu His Ala Ser Val Gln Ala Gly Glu Thr Val Gln Leu
   3305 3310 3315
   Gln Cys Leu Ala His Gly Thr Pro Pro Leu Thr Phe Gln Trp Ser
   3320 3325 3330
   Arg Val Gly Ser Ser Leu Pro Gly Arg Ala Thr Ala Arg Asn Glu
   3335 3340 3345
   Leu Leu His Phe Glu Arg Ala Ala Pro Glu Asp Ser Gly Arg Tyr
   3350 3355 3360
   Arg Cys Arg Val Thr Asn Lys Val Gly Ser Ala Glu Ala Phe Ala
   3365 3370 3375
   Gln Leu Leu Val Gln Gly Pro Pro Gly Ser Leu Pro Ala Thr Ser
   3380 3385 3390
   Ile Pro Ala Gly Ser Thr Pro Thr Val Gln Val Thr Pro Gln Leu
   3395 3400 3405
   Glu Thr Lys Ser Ile Gly Ala Ser Val Glu Phe His Cys Ala Val
   3410 3415 3420
   Pro Ser Asp Arg Gly Thr Gln Leu Arg Trp Phe Lys Glu Gly Gly
   3425 3430 3435
   Gln Leu Pro Pro Gly His Ser Val Gln Asp Gly Val Leu Arg Ile
   3440 3445 3450
   Gln Asn Leu Asp Gln Ser Cys Gln Gly Thr Tyr Ile Cys Gln Ala
   3455 3460 3465
   His Gly Pro Trp Gly Lys Ala Gln Ala Ser Ala Gln Leu Val Ile
   3470 3475 3480
   Gln Ala Leu Pro Ser Val Leu Ile Asn Ile Arg Thr Ser Val Gln
   3485 3490 3495
   Thr Val Val Val Gly His Ala Val Glu Phe Glu Cys Leu Ala Leu
   3500 3505 3510
   Gly Asp Pro Lys Pro Gln Val Thr Trp Ser Lys Val Gly Gly His
   3515 3520 3525
   Leu Arg Pro Gly Ile Val Gln Ser Gly Gly Val Val Arg Ile Ala
   3530 3535 3540
   His Val Glu Leu Ala Asp Ala Gly Gln Tyr Arg Cys Thr Ala Thr
       3545                3550                3555
   Asn Ala Ala Gly Thr Thr Gln Ser His Val Leu Leu Leu Val Gln
       3560                3565                3570
   Ala Leu Pro Gln Ile Ser Met Pro Gln Glu Val Arg Val Pro Ala
       3575                3580                3585
   Gly Ser Ala Ala Val Phe Pro Cys Ile Ala Ser Gly Tyr Pro Thr
       3590                3595                3600
   Pro Asp Ile Ser Trp Ser Lys Leu Asp Gly Ser Leu Pro Pro Asp
       3605                3610                3615
   Ser Arg Leu Glu Asn Asn Met Leu Met Leu Pro Ser Val Arg Pro
       3620                3625                3630
   Gln Asp Ala Gly Thr Tyr Val Cys Thr Ala Thr Asn Arg Gln Gly
       3635                3640                3645
   Lys Val Lys Ala Phe Ala His Leu Gln Val Pro Glu Arg Val Val
       3650                3655                3660
   Pro Tyr Phe Thr Gln Thr Pro Tyr Ser Phe Leu Pro Leu Pro Thr
       3665                3670                3675
   Ile Lys Asp Ala Tyr Arg Lys Phe Glu Ile Lys Ile Thr Phe Arg
       3680                3685                3690
   Pro Asp Ser Ala Asp Gly Met Leu Leu Tyr Asn Gly Gln Lys Arg
       3695                3700                3705
   Val Pro Gly Ser Pro Thr Asn Leu Ala Asn Arg Gln Pro Asp Phe
       3710                3715                3720
   Ile Ser Phe Gly Leu Val Gly Gly Arg Pro Glu Phe Arg Phe Asp
       3725                3730                3735
   Ala Gly Ser Gly Met Ala Thr Ile Arg His Pro Thr Pro Leu Ala
       3740                3745                3750
   Leu Gly His Phe His Thr Val Thr Leu Leu Arg Ser Leu Thr Gln
       3755                3760                3765
   Gly Ser Leu Ile Val Gly Asp Leu Ala Pro Val Asn Gly Thr Ser
       3770                3775                3780
   Gln Gly Lys Phe Gln Gly Leu Asp Leu Asn Glu Glu Leu Tyr Leu
   3785 3790 3795
   Gly Gly Tyr Pro Asp Tyr Gly Ala Ile Pro Lys Ala Gly Leu Ser
   3800 3805 3810
   Ser Gly Phe Ile Gly Cys Val Arg Glu Leu Arg Ile Gln Gly Glu
   3815 3820 3825
   Glu Ile Val Phe His Asp Leu Asn Leu Thr Ala His Gly Ile Ser
   3830 3835 3840
   His Cys Pro Thr Cys Arg Asp Arg Pro Cys Gln Asn Gly Gly Gln
   3845 3850 3855
   Cys His Asp Ser Glu Ser Ser Ser Tyr Val Cys Val Cys Pro Ala
   3860 3865 3870
   Gly Phe Thr Gly Ser Arg Cys Glu His Ser Gln Ala Leu His Cys
   3875 3880 3885
   His Pro Glu Ala Cys Gly Pro Asp Ala Thr Cys Val Asn Arg Pro
   3890 3895 3900
   Asp Gly Arg Gly Tyr Thr Cys Arg Cys His Leu Gly Arg Ser Gly
   3905 3910 3915
   Leu Arg Cys Glu Glu Gly Val Thr Val Thr Thr Pro Ser Leu Ser
   3920 3925 3930
   Gly Ala Gly Ser Tyr Leu Ala Leu Pro Ala Leu Thr Asn Thr His
   3935 3940 3945
   His Glu Leu Arg Leu Asp Val Glu Phe Lys Pro Leu Ala Pro Asp
   3950 3955 3960
   Gly Val Leu Leu Phe Ser Gly Gly Lys Ser Gly Pro Val Glu Asp
   3965 3970 3975
   Phe Val Ser Leu Ala Met Val Gly Gly His Leu Glu Phe Arg Tyr
   3980 3985 3990
   Glu Leu Gly Ser Gly Leu Ala Val Leu Arg Ser Ala Glu Pro Leu
   3995 4000 4005
   Ala Leu Gly Arg Trp His Arg Val Ser Ala Glu Arg Leu Asn Lys
   4010 4015 4020
   Asp Gly Ser Leu Arg Val Asn Gly Gly Arg Pro Val Leu Arg Ser
   4025 4030 4035
   Ser Pro Gly Lys Ser Gln Gly Leu Asn Leu His Thr Leu Leu Tyr
   4040 4045 4050
   Leu Gly Gly Val Glu Pro Ser Val Pro Leu Ser Pro Ala Thr Asn
   4055 4060 4065
   Met Ser Ala His Phe Arg Gly Cys Val Gly Glu Val Ser Val Asn
   4070 4075 4080
   Gly Lys Arg Leu Asp Leu Thr Tyr Ser Phe Leu Gly Ser Gln Gly
   4085 4090 4095
   Ile Gly Gln Cys Tyr Asp Ser Ser Pro Cys Glu Arg Gln Pro Cys
   4100 4105 4110
   Gln His Gly Ala Thr Cys Met Pro Ala Gly Glu Tyr Glu Phe Gln
   4115 4120 4125
   Cys Leu Cys Arg Asp Gly Phe Lys Gly Asp Leu Cys Glu His Glu
   4130 4135 4140
   Glu Asn Pro Cys Gln Leu Arg Glu Pro Cys Leu His Gly Gly Thr
   4145 4150 4155
   Cys Gln Gly Thr Arg Cys Leu Cys Leu Pro Gly Phe Ser Gly Pro
   4160 4165 4170
   Arg Cys Gln Gln Gly Ser Gly His Gly Ile Ala Glu Ser Asp Trp
   4175 4180 4185
   His Leu Glu Gly Ser Gly Gly Asn Asp Ala Pro Gly Gln Tyr Gly
   4190 4195 4200
   Ala Tyr Phe His Asp Asp Gly Phe Leu Ala Phe Pro Gly His Val
   4205 4210 4215
   Phe Ser Arg Ser Leu Pro Glu Val Pro Glu Thr Ile Glu Leu Glu
   4220 4225 4230
   Val Arg Thr Ser Thr Ala Ser Gly Leu Leu Leu Trp Gln Gly Val
   4235 4240 4245
   Glu Val Gly Glu Ala Gly Gln Gly Lys Asp Phe Ile Ser Leu Gly
   4250 4255 4260
   Leu Gln Asp Gly His Leu Val Phe Arg Tyr Gln Leu Gly Ser Gly
   4265 4270 4275
   Glu Ala  Arg Leu Val Ser Glu  Asp Pro Ile Asn Asp  Gly Glu Trp
       4280                 4285                 4290
   His Arg  Val Thr Ala Leu Arg  Glu Gly Arg Arg Gly  Ser Ile Gln
       4295                 4300                 4305
   Val Asp  Gly Glu Glu Leu Val  Ser Gly Arg Ser Pro  Gly Pro Asn
       4310                 4315                 4320
   Val Ala  Val Asn Ala Lys Gly  Ser Val Tyr Ile Gly  Gly Ala Pro
       4325                 4330                 4335
   Asp Val  Ala Thr Leu Thr Gly  Gly Arg Phe Ser Ser  Gly Ile Thr
       4340                 4345                 4350
   Gly Cys  Val Lys Asn Leu Val  Leu His Ser Ala Arg  Pro Gly Ala
       4355                 4360                 4365
   Pro Pro  Pro Gln Pro Leu Asp  Leu Gln His Arg Ala  Gln Ala Gly
       4370                 4375                 4380
   Ala Asn  Thr Arg Pro Cys Pro  Ser
       4385                 4390
<210> 4
   <211> 368
   <212> PRT
   <213> Homo sapiens
<400> 4
<210> 5
   <211> 359
   <212> PRT
   <213> Homo sapiens
<400> 5
   Met Lys Ala Thr Ile Ile Leu Leu Leu Leu Ala Gln Val Ser Trp Ala
   1               5                   10                  15
   Gly Pro Phe Gln Gln Arg Gly Leu Phe Asp Phe Met Leu Glu Asp Glu
               20                  25                  30
   Ala Ser Gly Ile Gly Pro Glu Val Pro Asp Asp Arg Asp Phe Glu Pro
           35                  40                  45
   Ser Leu Gly Pro Val Cys Pro Phe Arg Cys Gln Cys His Leu Arg Val
       50                  55                  60
   Val Gln Cys Ser Asp Leu Gly Leu Asp Lys Val Pro Lys Asp Leu Pro
   65                  70                  75                  80
   Pro Asp Thr Thr Leu Leu Asp Leu Gln Asn Asn Lys Ile Thr Glu Ile
                   85                  90                  95
   Lys Asp Gly Asp Phe Lys Asn Leu Lys Asn Leu His Ala Leu Ile Leu
               100                 105                 110
   Val Asn Asn Lys Ile Ser Lys Val Ser Pro Gly Ala Phe Thr Pro Leu
           115                 120                 125
   Val Lys Leu Glu Arg Leu Tyr Leu Ser Lys Asn Gln Leu Lys Glu Leu
       130                 135                 140
   Pro Glu Lys Met Pro Lys Thr Leu Gln Glu Leu Arg Ala His Glu Asn
   145                 150                 155                 160
   Glu Ile Thr Lys Val Arg Lys Val Thr Phe Asn Gly Leu Asn Gln Met
                   165                 170                 175
   Ile Val Ile Glu Leu Gly Thr Asn Pro Leu Lys Ser Ser Gly Ile Glu
               180                 185                 190
   Asn Gly Ala Phe Gln Gly Met Lys Lys Leu Ser Tyr Ile Arg Ile Ala
           195                 200                 205
   Asp Thr Asn Ile Thr Ser Ile Pro Gln Gly Leu Pro Pro Ser Leu Thr
       210                 215                 220
   Glu Leu His Leu Asp Gly Asn Lys Ile Ser Arg Val Asp Ala Ala Ser
   225                 230                 235                 240
   Leu Lys Gly Leu Asn Asn Leu Ala Lys Leu Gly Leu Ser Phe Asn Ser
                   245                 250                 255
   Ile Ser Ala Val Asp Asn Gly Ser Leu Ala Asn Thr Pro His Leu Arg
               260                 265                 270
   Glu Leu His Leu Asp Asn Asn Lys Leu Thr Arg Val Pro Gly Gly Leu
           275                 280                 285
   Ala Glu His Lys Tyr Ile Gln Val Val Tyr Leu His Asn Asn Asn Ile
       290                 295                 300
   Ser Val Val Gly Ser Ser Asp Phe Cys Pro Pro Gly His Asn Thr Lys
   305                 310                 315                 320
   Lys Ala Ser Tyr Ser Gly Val Ser Leu Phe Ser Asn Pro Val Gln Tyr
                   325                 330                 335
   Trp Glu Ile Gln Pro Ser Thr Phe Arg Cys Val Tyr Val Arg Ser Ala
               340                 345                 350
   Ile Gln Leu Gly Asn Tyr Lys
           355
<210> 6
   <211> 24
   <212> PRT
   <213> Homo sapiens
<400> 6
   Lys Ser Val Pro Lys Glu Ile Ser Pro Asp Thr Thr Leu Leu Asp Leu
   1               5                   10                  15
   Gln Asn Asn Asp Ile Ser Glu Leu
               20
<210> 7
   <211> 25
   <212> PRT
   <213> Homo sapiens
<400> 7
   Leu Lys Ser Val Pro Lys Glu Ile Ser Pro Asp Thr Thr Leu Leu Asp
   1               5                   10                  15
   Leu Gln Asn Asn Asp Ile Ser Glu Leu
               20                  25
<210> 8
   <211> 25
   <212> PRT
   <213> Homo sapiens
<400> 8
   Glu Asn Ser Gly Phe Glu Pro Gly Ala Phe Asp Gly Leu Lys Leu Asn
   1               5                   10                  15
   Tyr Leu Arg Ile Ser Glu Ala Lys Leu
               20                  25
<210> 9
   <211> 26
   <212> PRT
   <213> Homo sapiens
<400> 9
   Gly Leu Lys Ser Val Pro Lys Glu Ile Ser Pro Asp Thr Thr Leu Leu
   1               5                   10                  15
   Asp Leu Gln Asn Asn Asp Ile Ser Glu Leu
               20                  25
<210> 10
   <211> 20
   <212> PRT
   <213> Homo sapiens
<400> 10
   Tyr Leu Arg Ile Ser Glu Ala Lys Leu Thr Gly Ile Pro Lys Asp Leu
   1               5                   10                  15
   Pro Glu Thr Leu
               20
<210> 11
   <211> 26
   <212> PRT
   <213> Homo sapiens
<400> 11
   Gly Leu Lys Ser Val Pro Lys Glu Ile Ser Pro Asp Thr Thr Leu Leu
   1               5                   10                  15
   Asp Leu Gln Asn Asn Asp Ile Ser Glu Leu
               20                  25
<210> 12
   <211> 26
   <212> PRT
   <213> Homo sapiens
<400> 12
   Leu Thr Gly Ile Pro Lys Asp Leu Pro Glu Thr Leu Asn Glu Leu His
   1               5                   10                  15
   Leu Asp His Asn Lys Ile Gln Ala Ile Glu
               20                  25
<210> 13
   <211> 22
   <212> PRT
   <213> Homo sapiens
<400> 13
   Lys Arg Ile Ser Glu Ala Lys Leu Thr Gly Ile Pro Lys Asp Leu Pro
   1               5                   10                  15
   Glu Thr Leu Asn Glu Leu
               20
<210> 14
   <211> 14
   <212> PRT
   <213> Homo sapiens
<400> 14
   Gln Ala Ile Glu Leu Glu Asp Leu Leu Arg Tyr Ser Lys Leu
   1               5                   10
<210> 15
   <211> 12
   <212> PRT
   <213> Homo sapiens
<400> 15
   Gln Ala Ile Glu Leu Glu Asp Leu Leu Arg Tyr Ser
   1               5                   10
<210> 16
   <211> 19
   <212> PRT
   <213> Homo sapiens
<400> 16
   Ser Glu Ala Lys Leu Thr Gly Ile Pro Lys Asp Leu Pro Glu Thr Leu
   1               5                   10                  15
   Asn Glu Leu
<210> 17
   <211> 25
   <212> PRT
   <213> Homo sapiens
<400> 17
   Leu Lys Ala Val Pro Lys Glu Ile Ser Pro Asp Thr Thr Leu Leu Asp
   1               5                   10                  15
   Leu Gln Asn Asn Asp Ile Ser Glu Leu
               20                  25
<210> 18
   <211> 18
   <212> PRT
   <213> Homo sapiens
<400> 18
   Thr Leu Leu Asp Leu Gln Asn Asn Asp Ile Ser Glu Leu Arg Lys Asp
   1               5                   10                  15
   Asp Phe
<210> 19
   <211> 12
   <212> PRT
   <213> Homo sapiens
<400> 19
   Ala Ile Glu Leu Glu Asp Leu Leu Arg Tyr Ser Lys
   1               5                   10
<210> 20
   <211> 15
   <212> PRT
   <213> Homo sapiens
<400> 20
   Glu Asn Ser Gly Phe Glu Pro Gly Ala Phe Asp Gly Leu Lys Leu
   1               5                   10                  15
<210> 21
   <211> 13
   <212> PRT
   <213> Homo sapiens
<400> 21
   Met Ile Val Ile Glu Leu Gly Thr Asn Pro Leu Lys Ser
   1               5                   10
<210> 22
   <211> 13
   <212> PRT
   <213> Homo sapiens
<400> 22
   Met Ile Val Ile Glu Leu Gly Thr Asn Pro Leu Lys Ser
   1               5                   10
<210> 23
   <211> 17
   <212> PRT
   <213> Homo sapiens
<400> 23
   Glu Leu His Leu Asp Gly Asn Lys Ile Ser Arg Val Asp Ala Ala Ser
   1               5                   10                  15
   Leu
<210> 24
   <211> 18
   <212> PRT
   <213> Homo sapiens
<400> 24
   Leu Val Asn Asn Lys Ile Ser Lys Val Ser Pro Gly Ala Phe Thr Pro
   1               5                   10                  15
   Leu Val
<210> 25
   <211> 26
   <212> PRT
   <213> Homo sapiens
<400> 25
   Ala Leu Ile Leu Val Asn Asn Lys Ile Ser Lys Val Ser Pro Gly Ala
   1               5                   10                  15
   Phe Thr Pro Leu Val Lys Leu Glu Arg Leu
               20                  25
<210> 26
   <211> 17
   <212> PRT
   <213> Homo sapiens
<400> 26
   Phe Ser Asn Pro Val Gln Tyr Trp Glu Ile Gln Pro Ser Thr Phe Arg
   1               5                   10                  15
   Cys
<210> 27
   <211> 15
   <212> PRT
   <213> Homo sapiens
<400> 27
   Lys Ser Ser Gly Ile Glu Asn Gly Ala Phe Gln Gly Met Lys Lys
   1               5                   10                  15
<210> 28
   <211> 18
   <212> PRT
   <213> Homo sapiens
<400> 28
   Lys Ser Ser Gly Ile Glu Asn Gly Ala Phe Gln Gly Met Lys Lys Leu
   1               5                   10                  15
   Ser Tyr
<210> 29
   <211> 13
   <212> PRT
   <213> Homo sapiens
<400> 29
   Asn Lys Ile Thr Glu Ile Lys Asp Gly Asp Phe Lys Asn
   1               5                   10
<210> 30
   <211> 17
   <212> PRT
   <213> Homo sapiens
<400> 30
   Gln Gly Leu Pro Pro Ser Leu Thr Glu Leu His Leu Asp Gly Asn Lys
   1               5                   10                  15
   Ile
<210> 31
   <211> 12
   <212> PRT
   <213> Homo sapiens
<400> 31
   Lys Leu Leu Ala Ser Asp Ala Gly Leu Tyr Arg Cys
   1               5                   10
<210> 32
   <211> 13
   <212> PRT
   <213> Homo sapiens
<400> 32
   Leu Ala Thr Val Gly Glu Leu Gln Ala Ala Trp Arg Asn
   1               5                   10
<210> 33
   <211> 15
   <212> PRT
   <213> Homo sapiens
<400> 33
   Lys Glu Thr Thr Val Leu Val Ala Gln Asn Gly Asn Ile Lys Ile
   1               5                   10                  15
<210> 34
   <211> 12
   <212> PRT
   <213> Homo sapiens
<400> 34
   Ser Leu Glu Asp Leu Gln Leu Thr His Asn Lys Ile
   1               5                   10
<210> 35
   <211> 13
   <212> PRT
   <213> Homo sapiens
<400> 35
   Arg Leu Lys Glu Asp Ala Val Ser Ala Ala Phe Lys Gly
   1               5                   10
<210> 36
   <211> 16
   <212> PRT
   <213> Homo sapiens
<400> 36
   Arg Ser Ile Glu Tyr Ser Pro Gln Leu Glu Asp Ala Gly Ser Arg Glu
   1 5 10 15
<210> 37
   <211> 12
   <212> PRT
   <213> Homo sapiens
<400> 37
   Arg Leu Glu Gly Asp Thr Leu Ile Ile Pro Arg Val
   1 5 10
<210> 38
   <211> 15
   <212> PRT
   <213> Homo sapiens
<400> 38
   Glu Val Ser Glu Ala Val Val Glu Lys Leu Glu Pro Glu Tyr Arg
   1 5 10 15
<210> 39
   <211> 16
   <212> PRT
   <213> Homo sapiens
<400> 39
   Arg Glu Val Ser Glu Ala Val Val Glu Lys Leu Glu Pro Glu Tyr Arg
   1 5 10 15
<210> 40
   <211> 12
   <212> PRT
   <213> Homo sapiens
<400> 40
   Arg Ser Ile Glu Tyr Ser Pro Gln Leu Glu Asp Ala
   1 5 10
<210> 41
   <211> 22
   <212> PRT
   <213> Homo sapiens
<400> 41
   Ser Val Pro Lys Glu Ile Ser Pro Asp Thr Thr Leu Leu Asp Leu Gln
   1 5 10 15
   Asn Asn Asp Ile Ser Glu
   20
<210> 42
   <211> 23
   <212> PRT
   <213> Homo sapiens
<400> 42
   Lys Ser Val Pro Lys Glu Ile Ser Pro Asp Thr Thr Leu Leu Asp Leu
   1               5                   10                  15
   Gln Asn Asn Asp Ile Ser Glu
               20
<210> 43
   <211> 23
   <212> PRT
   <213> Homo sapiens
<400> 43
   Asn Ser Gly Phe Glu Pro Gly Ala Phe Asp Gly Leu Lys Leu Asn Tyr
   1               5                   10                  15
   Leu Arg Ile Ser Glu Ala Lys
               20
<210> 44
   <211> 24
   <212> PRT
   <213> Homo sapiens
<400> 44
   Leu Lys Ser Val Pro Lys Glu Ile Ser Pro Asp Thr Thr Leu Leu Asp
   1               5                   10                  15
   Leu Gln Asn Asn Asp Ile Ser Glu
               20
<210> 45
   <211> 18
   <212> PRT
   <213> Homo sapiens
<400> 45
   Leu Arg Ile Ser Glu Ala Lys Leu Thr Gly Ile Pro Lys Asp Leu Pro
   1               5                   10                  15
   Glu Thr
<210> 46
   <211> 24
   <212> PRT
   <213> Homo sapiens
<400> 46
   Leu Lys Ser Val Pro Lys Glu Ile Ser Pro Asp Thr Thr Leu Leu Asp
   1               5                   10                  15
   Leu Gln Asn Asn Asp Ile Ser Glu
               20
<210> 47
   <211> 26
   <212> PRT
   <213> Homo sapiens
<400> 47
   Leu Thr Gly Ile Pro Lys Asp Leu Pro Glu Thr Leu Asn Glu Leu His
   1               5                   10                  15
   Leu Asp His Asn Lys Ile Gln Ala Ile Glu
               20                  25
<210> 48
   <211> 11
   <212> PRT
   <213> Homo sapiens
<400> 48
   Ile Val Ile Glu Leu Gly Thr Asn Pro Leu Lys
   1               5                   10
<210> 49
   <211> 10
   <212> PRT
   <213> Homo sapiens
<400> 49
   Leu Leu Ala Ser Asp Ala Gly Leu Tyr Arg
   1               5                   10
<210> 50
   <211> 12
   <212> PRT
   <213> Homo sapiens
<400> 50
   Leu Ala Thr Val Gly Glu Leu Gln Ala Ala Trp Arg
   1               5                   10
<210> 51
   <211> 13
   <212> PRT
   <213> Homo sapiens
<400> 51
   Glu Thr Thr Val Leu Val Ala Gln Asn Gly Asn Ile Lys
   1 5 10
<210> 52
   <211> 11
   <212> PRT
   <213> Homo sapiens
<400> 52
   Ser Leu Glu Asp Leu Gln Leu Thr His Asn Lys
   1 5 10
<210> 53
   <211> 11
   <212> PRT
   <213> Homo sapiens
<400> 53
   Leu Lys Glu Asp Ala Val Ser Ala Ala Phe Lys
   1 5 10
<210> 54
   <211> 14
   <212> PRT
   <213> Homo sapiens
<400> 54
   Ser Ile Glu Tyr Ser Pro Gln Leu Glu Asp Ala Gly Ser Arg
   1 5 10
<210> 55
   <211> 10
   <212> PRT
   <213> Homo sapiens
<400> 55
   Leu Glu Gly Asp Thr Leu Ile Ile Pro Arg
   1 5 10
<210> 56
   <211> 20
   <212> PRT
   <213> Homo sapiens
<400> 56
   Arg Ile Ser Glu Ala Lys Leu Thr Gly Ile Pro Lys Asp Leu Pro Glu
   1 5 10 15
   Thr Leu Asn Glu
   20
<210> 57
   <211> 12
   <212> PRT
   <213> Homo sapiens
<400> 57
   Ala Ile Glu Leu Glu Asp Leu Leu Arg Tyr Ser Lys
   1               5                   10
<210> 58
   <211> 10
   <212> PRT
   <213> Homo sapiens
<400> 58
   Ala Ile Glu Leu Glu Asp Leu Leu Arg Tyr
   1               5                   10
<210> 59
   <211> 17
   <212> PRT
   <213> Homo sapiens
<400> 59
   Glu Ala Lys Leu Thr Gly Ile Pro Lys Asp Leu Pro Glu Thr Leu Asn
   1               5                   10                  15
   Glu
<210> 60
   <211> 24
   <212> PRT
   <213> Homo sapiens
<400> 60
   Leu Lys Ala Val Pro Lys Glu Ile Ser Pro Asp Thr Thr Leu Leu Asp
   1               5                   10                  15
   Leu Gln Asn Asn Asp Ile Ser Glu
               20
<210> 61
   <211> 16
   <212> PRT
   <213> Homo sapiens
<400> 61
   Leu Leu Asp Leu Gln Asn Asn Asp Ile Ser Glu Leu Arg Lys Asp Asp
   1               5                   10                  15
<210> 62
   <211> 10
   <212> PRT
   <213> Homo sapiens
<400> 62
   Ile Glu Leu Glu Asp Leu Leu Arg Tyr Ser
   1               5                   10
<210> 63
   <211> 13
   <212> PRT
   <213> Homo sapiens
<400> 63
   Asn Ser Gly Phe Glu Pro Gly Ala Phe Asp Gly Leu Lys
   1               5                   10
<210> 64
   <211> 14
   <212> PRT
   <213> Homo sapiens
<400> 64
   Asp Glu Ala Ser Gly Ile Gly Pro Glu Val Pro Asp Asp Arg
   1               5                   10
<210> 65
   <211> 15
   <212> PRT
   <213> Homo sapiens
<400> 65
   Leu His Leu Asp Gly Asn Lys Ile Ser Arg Val Asp Ala Ala Ser
   1               5                   10                  15
<210> 66
   <211> 16
   <212> PRT
   <213> Homo sapiens
<400> 66
   Val Asn Asn Lys Ile Ser Lys Val Ser Pro Gly Ala Phe Thr Pro Leu
   1               5                   10                  15
<210> 67
   <211> 24
   <212> PRT
   <213> Homo sapiens
<400> 67
   Leu Ile Leu Val Asn Asn Lys Ile Ser Lys Val Ser Pro Gly Ala Phe
   1               5                   10                  15
   Thr Pro Leu Val Lys Leu Glu Arg
               20
<210> 68
   <211> 15
   <212> PRT
   <213> Homo sapiens
<400> 68
   Ser Asn Pro Val Gln Tyr Trp Glu Ile Gln Pro Ser Thr Phe Arg
   1               5                   10                  15
<210> 69
   <211> 13
   <212> PRT
   <213> Homo sapiens
<400> 69
   Ser Ser Gly Ile Glu Asn Gly Ala Phe Gln Gly Met Lys
   1               5                   10
<210> 70
   <211> 16
   <212> PRT
   <213> Homo sapiens
<400> 70
   Ser Ser Gly Ile Glu Asn Gly Ala Phe Gln Gly Met Lys Lys Leu Ser
   1               5                   10                  15
<210> 71
   <211> 11
   <212> PRT
   <213> Homo sapiens
<400> 71
   Lys Ile Thr Glu Ile Lys Asp Gly Asp Phe Lys
   1               5                   10
<210> 72
   <211> 15
   <212> PRT
   <213> Homo sapiens
<400> 72
   Gly Leu Pro Pro Ser Leu Thr Glu Leu His Leu Asp Gly Asn Lys
   1               5                   10                  15
<210> 73
   <211> 14
   <212> PRT
   <213> Homo sapiens
<400> 73
   Val Ser Glu Ala Val Val Glu Lys Leu Glu Pro Glu Tyr Arg
   1               5                   10
<210> 74
   <211> 15
   <212> PRT
   <213> Homo sapiens
<400> 74
   Glu Val Ser Glu Ala Val Val Glu Lys Leu Glu Pro Glu Tyr Arg
   1               5                   10                  15
<210> 75
   <211> 17
   <212> PRT
   <213> Homo sapiens
<400> 75
   Ser Ile Glu Tyr Ser Pro Gln Leu Glu Asp Ala Ser Ala Lys Glu Phe
   1               5                   10                  15
   Arg
<210> 76
   <211> 6
   <212> PRT
   <213> Homo sapiens
<400> 76
   Ser Val Pro Lys Glu Ile
   1               5
<210> 77
   <211> 6
   <212> PRT
   <213> Homo sapiens
<400> 77
   Asn Ser Gly Phe Glu Pro
   1               5
<210> 78
   <211> 6
   <212> PRT
   <213> Homo sapiens
<400> 78
   Leu Lys Ser Val Pro Lys
   1 5
<210> 79
   <211> 6
   <212> PRT
   <213> Homo sapiens
<400> 79
   Leu Arg Ile Ser Glu Ala
   1 5
<210> 80
   <211> 6
   <212> PRT
   <213> Homo sapiens
<400> 80
   Gly Leu Lys Leu Asn Tyr
   1 5
<210> 81
   <211> 6
   <212> PRT
   <213> Homo sapiens
<400> 81
   Leu Lys Ser Val Pro Lys
   1 5
<210> 82
   <211> 6
   <212> PRT
   <213> Homo sapiens
<400> 82
   Gln Cys Ser Asp Leu Gly
   1 5
<210> 83
   <211> 6
   <212> PRT
   <213> Homo sapiens
<400> 83
   Leu Thr Gly Ile Pro Lys
   1 5
<210> 84
   <211> 6
   <212> PRT
   <213> Homo sapiens
<400> 84
   Arg Ile Ser Glu Ala Lys
   1 5
<210> 85
   <211> 6
   <212> PRT
   <213> Homo sapiens
<400> 85
   Ala Ile Glu Leu Glu Asp
   1 5
<210> 86
   <211> 6
   <212> PRT
   <213> Homo sapiens
<400> 86
   Glu Ala Lys Leu Thr Gly
   1 5
<210> 87
   <211> 6
   <212> PRT
   <213> Homo sapiens
<400> 87
   Leu Lys Ala Val Pro Lys
   1 5
<210> 88
   <211> 6
   <212> PRT
   <213> Homo sapiens
<400> 88
   Leu Leu Asp Leu Gln Asn
   1 5
<210> 89
   <211> 6
   <212> PRT
   <213> Homo sapiens
<400> 89
   Ile Glu Leu Glu Asp Leu
   1 5
<210> 90
   <211> 6
   <212> PRT
   <213> Homo sapiens
<400> 90
   Asn Ser Gly Phe Glu Pro
   1 5
<210> 91
   <211> 6
   <212> PRT
   <213> Homo sapiens
<400> 91
   Ile Val Ile Glu Leu Gly
   1 5
<210> 92
   <211> 6
   <212> PRT
   <213> Homo sapiens
<400> 92
   Asn Gly Leu Asn Gln Met
   1 5
<210> 93
   <211> 6
   <212> PRT
   <213> Homo sapiens
<400> 93
   Asp Glu Ala Ser Gly Ile
   1 5
<210> 94
   <211> 6
   <212> PRT
   <213> Homo sapiens
<400> 94
   Leu His Leu Asp Gly Asn
   1 5
<210> 95
   <211> 6
   <212> PRT
   <213> Homo sapiens
<400> 95
   Val Asn Asn Lys Ile Ser
   1 5
<210> 96
   <211> 6
   <212> PRT
   <213> Homo sapiens
<400> 96
   Leu Ile Leu Val Asn Asn
   1 5
<210> 97
   <211> 6
   <212> PRT
   <213> Homo sapiens
<400> 97
   Ser Asn Pro Val Gln Tyr
   1 5
<210> 98
   <211> 6
   <212> PRT
   <213> Homo sapiens
<400> 98
   Ser Ser Gly Ile Glu Asn
   1 5
<210> 99
   <211> 6
   <212> PRT
   <213> Homo sapiens
<400> 99
   Lys Ile Thr Glu Ile Lys
   1 5
<210> 100
   <211> 6
   <212> PRT
   <213> Homo sapiens
<400> 100
   Gly Leu Pro Pro Ser Leu
   1 5
<210> 101
   <211> 6
   <212> PRT
   <213> Homo sapiens
<400> 101
   Leu Leu Ala Ser Asp Ala
   1               5
<210> 102
   <211> 6
   <212> PRT
   <213> Homo sapiens
<400> 102
   Leu Ala Thr Val Gly Glu
   1               5
<210> 103
   <211> 6
   <212> PRT
   <213> Homo sapiens
<400> 103
   Glu Thr Thr Val Leu Val
   1               5
<210> 104
   <211> 6
   <212> **PRT**
   <213> Homo sapiens
<400> 104
   Ser Leu Thr Val Val Lys
   1               5
<210> 105
   <211> 6
   <212> PRT
   <213> Homo sapiens
<400> 105
   Glu Asn Gln Asp Ala Arg
   1               5
<210> 106
   <211> 6
   <212> PRT
   <213> Homo sapiens
<400> 106
   Asn Gly Phe Asp Gln Cys
   1               5
<210> 107
   <211> 6
   <212> PRT
   <213> Homo sapiens
<400> 107
   Ser Leu Glu Asp Leu Gln
   1 5
<210> 108
   <211> 6
   <212> PRT
   <213> Homo sapiens
<400> 108
   Leu Lys Glu Asp Ala Val
   1 5
<210> 109
   <211> 6
   <212> PRT
   <213> Homo sapiens
<400> 109
   His Leu Gln His Asn Arg
   1 5
<210> 110
   <211> 6
   <212> PRT
   <213> Homo sapiens
<400> 110
   Leu Gln His Asn Arg Leu
   1 5
<210> 111
   <211> 6
   <212> PRT
   <213> Homo sapiens
<400> 111
   Ser Ile Glu Tyr Ser Pro
   1 5
<210> 112
   <211> 6
   <212> PRT
   <213> Homo sapiens
<400> 112
   Leu Val Asn Phe Thr Arg
   1 5
<210> 113
   <211> 6
   <212> PRT
   <213> Homo sapiens
<400> 113
   Val Ser Glu Ala Val Val
   1               5
<210> 114
   <211> 6
   <212> PRT
   <213> Homo sapiens
<400> 114
   Glu Val Ser Glu Ala Val
   1               5
<210> 115
   <211> 6
   <212> PRT
   <213> Homo sapiens
<400> 115
   Ser Ile Glu Tyr Ser Pro
   1               5
<210> 116
   <211> 6
   <212> PRT
   <213> Homo sapiens
<400> 116
   Asn Asn Asp Ile Ser Glu
   1               5
<210> 117
   <211> 6
   <212> PRT
   <213> Homo sapiens
<400> 117
   Arg Ile Ser Glu Ala Lys
   1               5
<210> 118
   <211> 6
   <212> PRT
   <213> Homo sapiens
<400> 118
   Leu Arg Lys Asp Asp Phe
   1               5
<210> 119
   <211> 6
   <212> PRT
   <213> Homo sapiens
<400> 119
   Lys Asp Leu Pro Glu Thr
   1               5
<210> 120
   <211> 6
   <212> **PRT**
   <213> Homo sapiens
<400> 120
   Leu Asn Glu Leu His Leu
   1               5
<210> 121
   <211> 6
   <212> **PRT**
   <213> Homo sapiens
<400> 121
   Tyr Trp Glu Val Gln Pro
   1               5
<210> 122
   <211> 6
   <212> PRT
   <213> Homo sapiens
<400> 122
   Lys Ile Gln Ala Ile Glu
   1               5
<210> 123
   <211> 6
   <212> PRT
   <213> Homo sapiens
<400> 123
   Pro Glu Thr Leu Asn Glu
   1               5
<210> 124
   <211> 6
   <212> PRT
   <213> Homo sapiens
<400> 124
   Leu Leu Arg Tyr Ser Lys
   1 5
<210> 125
   <211> 6
   <212> PRT
   <213> Homo sapiens
<400> 125
   Glu Asp Leu Leu Arg Tyr
   1 5
<210> 126
   <211> 6
   <212> PRT
   <213> Homo sapiens
<400> 126
   Asn Asn Asp Ile Ser Glu
   1 5
<210> 127
   <211> 6
   <212> PRT
   <213> Homo sapiens
<400> 127
   Glu Leu Arg Lys Asp Asp
   1 5
<210> 128
   <211> 6
   <212> PRT
   <213> Homo sapiens
<400> 128
   Asp Leu Leu Arg Tyr Ser
   1 5
<210> 129
   <211> 6
   <212> PRT
   <213> Homo sapiens
<400> 129
   Ala Phe Asp Gly Leu Lys
   1 5
<210> 130
   <211> 6
   <212> PRT
   <213> Homo sapiens
<400> 130
   Gly Thr Asn Pro Leu Lys
   1 5
<210> 131
   <211> 6
   <212> PRT
   <213> Homo sapiens
<400> 131
   Ser Ser Gly Ile Glu Asn
   1 5
<210> 132
   <211> 6
   <212> PRT
   <213> Homo sapiens
<400> 132
   Glu Val Pro Asp Asp Arg
   1 5
<210> 133
   <211> 6
   <212> PRT
   <213> Homo sapiens
<400> 133
   Arg Val Asp Ala Ala Ser
   1 5
<210> 134
   <211> 6
   <212> PRT
   <213> Homo sapiens
<400> 134
   Gly Ala Phe Thr Pro Leu
   1 5
<210> 135
   <211> 6
   <212> PRT
   <213> Homo sapiens
<400> 135
   Leu Val Lys Leu Glu Arg
   1 5
<210> 136
   <211> 6
   <212> PRT
   <213> Homo sapiens
<400> 136
   Gln Pro Ser Thr Phe Arg
   1 5
<210> 137
   <211> 6
   <212> PRT
   <213> Homo sapiens
<400> 137
   Ala Phe Gln Gly Met Lys
   1 5
<210> 138
   <211> 6
   <212> PRT
   <213> Homo sapiens
<400> 138
   Gly Met Lys Lys Leu Ser
   1 5
<210> 139
   <211> 6
   <212> PRT
   <213> Homo sapiens
<400> 139
   Lys Asp Gly Asp Phe Lys
   1 5
<210> 140
   <211> 6
   <212> PRT
   <213> Homo sapiens
<400> 140
   His Leu Asp Gly Asn Lys
   1 5
<210> 141
   <211> 6
   <212> PRT
   <213> Homo sapiens
<400> 141
   Cys Asp Val Met Tyr Gly
   1 5
<210> 142
   <211> 6
   <212> PRT
   <213> Homo sapiens
<400> 142
   Asn Gly Phe Asp Gln Cys
   1               5
<210> 143
   <211> 6
   <212> PRT
   <213> Homo sapiens
<400> 143
   Gln Asn Gly Ile Asn Lys
   1               5
<210> 144
   <211> 6
   <212> PRT
   <213> Homo sapiens
<400> 144
   Ile Gly Gln Asp Tyr Lys
   1               5
<210> 145
   <211> 6
   <212> PRT
   <213> Homo sapiens
<400> 145
   Gln Leu Thr His Asn Lys
   1               5
<210> 146
   <211> 6
   <212> PRT
   <213> Homo sapiens
<400> 146
   Val Ser Ala Ala Phe Lys
   1               5
<210> 147
   <211> 6
   <212> PRT
   <213> Homo sapiens
<400> 147
   Gly Leu Lys Ser Leu Glu
   1               5
<210> 148
   <211> 6
   <212> PRT
   <213> Homo sapiens
<400> 148
   Glu Asp Ala Gly Ser Arg
   1 5
<210> 149
   <211> 6
   <212> PRT
   <213> Homo sapiens
<400> 149
   Glu Phe Arg Glu Val Ser
   1 5
<210> 150
   <211> 6
   <212> PRT
   <213> Homo sapiens
<400> 150
   Val Ala Gln Gln Asp Ser
   1 5
<210> 151
   <211> 6
   <212> PRT
   <213> Homo sapiens
<400> 151
   Leu Glu Pro Glu Tyr Arg
   1 5
<210> 152
   <211> 6
   <212> PRT
   <213> Homo sapiens
<400> 152
   Ser Ala Lys Glu Phe Arg
   1 5
<210> 153
   <211> 1466
   <212> PRT
   <213> Homo sapiens
<400> 153
   Met Met Ser Phe Val Gln Lys Gly Ser Trp Leu Leu Leu Ala Leu Leu
   1               5                   10                  15
   His Pro Thr Ile Ile Leu Ala Gln Gln Glu Ala Val Glu Gly Gly Cys
               20                  25                  30
   Ser His Leu Gly Gln Ser Tyr Ala Asp Arg Asp Val Trp Lys Pro Glu
           35                  40                  45
   Pro Cys Gln Ile Cys Val Cys Asp Ser Gly Ser Val Leu Cys Asp Asp
       50                  55                  60
   Ile Ile Cys Asp Asp Gln Glu Leu Asp Cys Pro Asn Pro Glu Ile Pro
   65                  70                  75                  80
   Phe Gly Glu Cys Cys Ala Val Cys Pro Gln Pro Pro Thr Ala Pro Thr
                   85                  90                  95
   Arg Pro Pro Asn Gly Gln Gly Pro Gln Gly Pro Lys Gly Asp Pro Gly
               100                 105                 110
   Pro Pro Gly Ile Pro Gly Arg Asn Gly Asp Pro Gly Ile Pro Gly Gln
           115                 120                 125
   Pro Gly Ser Pro Gly Ser Pro Gly Pro Pro Gly Ile Cys Glu Ser Cys
       130                 135                 140
   Pro Thr Gly Pro Gln Asn Tyr Ser Pro Gln Tyr Asp Ser Tyr Asp Val
   145                 150                 155                 160
   Lys Ser Gly Val Ala Val Gly Gly Leu Ala Gly Tyr Pro Gly Pro Ala
                   165                 170                 175
   Gly Pro Pro Gly Pro Pro Gly Pro Pro Gly Thr Ser Gly His Pro Gly
   180 185 190
   Ser Pro Gly Ser Pro Gly Tyr Gln Gly Pro Pro Gly Glu Pro Gly Gln
   195 200 205
   Ala Gly Pro Ser Gly Pro Pro Gly Pro Pro Gly Ala Ile Gly Pro Ser
   210 215 220
   Gly Pro Ala Gly Lys Asp Gly Glu Ser Gly Arg Pro Gly Arg Pro Gly
   225 230 235 240
   Glu Arg Gly Leu Pro Gly Pro Pro Gly Ile Lys Gly Pro Ala Gly Ile
   245 250 255
   Pro Gly Phe Pro Gly Met Lys Gly His Arg Gly Phe Asp Gly Arg Asn
   260 265 270
   Gly Glu Lys Gly Glu Thr Gly Ala Pro Gly Leu Lys Gly Glu Asn Gly
   275 280 285
   Leu Pro Gly Glu Asn Gly Ala Pro Gly Pro Met Gly Pro Arg Gly Ala
   290 295 300
   Pro Gly Glu Arg Gly Arg Pro Gly Leu Pro Gly Ala Ala Gly Ala Arg
   305 310 315 320
   Gly Asn Asp Gly Ala Arg Gly Ser Asp Gly Gln Pro Gly Pro Pro Gly
   325 330 335
   Pro Pro Gly Thr Ala Gly Phe Pro Gly Ser Pro Gly Ala Lys Gly Glu
   340 345 350
   Val Gly Pro Ala Gly Ser Pro Gly Ser Asn Gly Ala Pro Gly Gln Arg
   355 360 365
   Gly Glu Pro Gly Pro Gln Gly His Ala Gly Ala Gln Gly Pro Pro Gly
   370 375 380
   Pro Pro Gly Ile Asn Gly Ser Pro Gly Gly Lys Gly Glu Met Gly Pro
   385 390 395 400
   Ala Gly Ile Pro Gly Ala Pro Gly Leu Met Gly Ala Arg Gly Pro Pro
   405 410 415
   Gly Pro Ala Gly Ala Asn Gly Ala Pro Gly Leu Arg Gly Gly Ala Gly
   420 425 430
   Glu Pro Gly Lys Asn Gly Ala Lys Gly Glu Pro Gly Pro Arg Gly Glu
   435 440 445
   Arg Gly Glu Ala Gly Ile Pro Gly Val Pro Gly Ala Lys Gly Glu Asp
   450 455 460
   Gly Lys Asp Gly Ser Pro Gly Glu Pro Gly Ala Asn Gly Leu Pro Gly
   465 470 475 480
   Ala Ala Gly Glu Arg Gly Ala Pro Gly Phe Arg Gly Pro Ala Gly Pro
   485 490 495
   Asn Gly Ile Pro Gly Glu Lys Gly Pro Ala Gly Glu Arg Gly Ala Pro
   500 505 510
   Gly Pro Ala Gly Pro Arg Gly Ala Ala Gly Glu Pro Gly Arg Asp Gly
   515 520 525
   Val Pro Gly Gly Pro Gly Met Arg Gly Met Pro Gly Ser Pro Gly Gly
   530 535 540
   Pro Gly Ser Asp Gly Lys Pro Gly Pro Pro Gly Ser Gln Gly Glu Ser
   545 550 555 560
   Gly Arg Pro Gly Pro Pro Gly Pro Ser Gly Pro Arg Gly Gln Pro Gly
   565 570 575
   Val Met Gly Phe Pro Gly Pro Lys Gly Asn Asp Gly Ala Pro Gly Lys
   580 585 590
   Asn Gly Glu Arg Gly Gly Pro Gly Gly Pro Gly Pro Gln Gly Pro Pro
   595 600 605
   Gly Lys Asn Gly Glu Thr Gly Pro Gln Gly Pro Pro Gly Pro Thr Gly
   610 615 620
   Pro Gly Gly Asp Lys Gly Asp Thr Gly Pro Pro Gly Pro Gln Gly Leu
   625 630 635 640
   Gln Gly Leu Pro Gly Thr Gly Gly Pro Pro Gly Glu Asn Gly Lys Pro
   645 650 655
   Gly Glu Pro Gly Pro Lys Gly Asp Ala Gly Ala Pro Gly Ala Pro Gly
   660 665 670
   Gly Lys Gly Asp Ala Gly Ala Pro Gly Glu Arg Gly Pro Pro Gly Leu
   675 680 685
   Ala Gly Ala Pro Gly Leu Arg Gly Gly Ala Gly Pro Pro Gly Pro Glu
   690 695 700
   Gly Gly Lys Gly Ala Ala Gly Pro Pro Gly Pro Pro Gly Ala Ala Gly
   705 710 715 720
   Thr Pro Gly Leu Gln Gly Met Pro Gly Glu Arg Gly Gly Leu Gly Ser
   725 730 735
   Pro Gly Pro Lys Gly Asp Lys Gly Glu Pro Gly Gly Pro Gly Ala Asp
   740 745 750
   Gly Val Pro Gly Lys Asp Gly Pro Arg Gly Pro Thr Gly Pro Ile Gly
   755 760 765
   Pro Pro Gly Pro Ala Gly Gln Pro Gly Asp Lys Gly Glu Gly Gly Ala
   770 775 780
   Pro Gly Leu Pro Gly Ile Ala Gly Pro Arg Gly Ser Pro Gly Glu Arg
   785 790 795 800
   Gly Glu Thr Gly Pro Pro Gly Pro Ala Gly Phe Pro Gly Ala Pro Gly
   805 810 815
   Gln Asn Gly Glu Pro Gly Gly Lys Gly Glu Arg Gly Ala Pro Gly Glu
   820 825 830
   Lys Gly Glu Gly Gly Pro Pro Gly Val Ala Gly Pro Pro Gly Gly Ser
   835 840 845
   Gly Pro Ala Gly Pro Pro Gly Pro Gln Gly Val Lys Gly Glu Arg Gly
   850 855 860
   Ser Pro Gly Gly Pro Gly Ala Ala Gly Phe Pro Gly Ala Arg Gly Leu
   865 870 875 880
   Pro Gly Pro Pro Gly Ser Asn Gly Asn Pro Gly Pro Pro Gly Pro Ser
   885 890 895
   Gly Ser Pro Gly Lys Asp Gly Pro Pro Gly Pro Ala Gly Asn Thr Gly
   900 905 910
   Ala Pro Gly Ser Pro Gly Val Ser Gly Pro Lys Gly Asp Ala Gly Gln
   915 920 925
   Pro Gly Glu Lys Gly Ser Pro Gly Ala Gln Gly Pro Pro Gly Ala Pro
   930 935 940
   Gly Pro Leu Gly Ile Ala Gly Ile Thr Gly Ala Arg Gly Leu Ala Gly
   945 950 955 960
   Pro Pro Gly Met Pro Gly Pro Arg Gly Ser Pro Gly Pro Gln Gly Val
   965 970 975
   Lys Gly Glu Ser Gly Lys Pro Gly Ala Asn Gly Leu Ser Gly Glu Arg
   980 985 990
   Gly Pro Pro Gly Pro Gln Gly Leu Pro Gly Leu Ala Gly Thr Ala Gly
   995 1000 1005
   Glu Pro Gly Arg Asp Gly Asn Pro Gly Ser Asp Gly Leu Pro Gly
   1010 1015 1020
   Arg Asp Gly Ser Pro Gly Gly Lys Gly Asp Arg Gly Glu Asn Gly
   1025 1030 1035
   Ser Pro Gly Ala Pro Gly Ala Pro Gly His Pro Gly Pro Pro Gly
   1040 1045 1050
   Pro Val Gly Pro Ala Gly Lys Ser Gly Asp Arg Gly Glu Ser Gly
   1055 1060 1065
   Pro Ala Gly Pro Ala Gly Ala Pro Gly Pro Ala Gly Ser Arg Gly
   1070 1075 1080
   Ala Pro Gly Pro Gln Gly Pro Arg Gly Asp Lys Gly Glu Thr Gly
   1085 1090 1095
   Glu Arg Gly Ala Ala Gly Ile Lys Gly His Arg Gly Phe Pro Gly
   1100 1105 1110
   Asn Pro Gly Ala Pro Gly Ser Pro Gly Pro Ala Gly Gln Gln Gly
   1115 1120 1125
   Ala Ile Gly Ser Pro Gly Pro Ala Gly Pro Arg Gly Pro Val Gly
   1130 1135 1140
   Pro Ser Gly Pro Pro Gly Lys Asp Gly Thr Ser Gly His Pro Gly
   1145 1150 1155
   Pro Ile Gly Pro Pro Gly Pro Arg Gly Asn Arg Gly Glu Arg Gly
   1160 1165 1170
   Ser Glu Gly Ser Pro Gly His Pro Gly Gln Pro Gly Pro Pro Gly
   1175 1180 1185
   Pro Pro Gly Ala Pro Gly Pro Cys Cys Gly Gly Val Gly Ala Ala
   1190 1195 1200
   Ala Ile Ala Gly Ile Gly Gly Glu Lys Ala Gly Gly Phe Ala Pro
       1205                1210                1215
   Tyr Tyr Gly Asp Glu Pro Met Asp Phe Lys Ile Asn Thr Asp Glu
       1220                1225                1230
   Ile Met Thr Ser Leu Lys Ser Val Asn Gly Gln Ile Glu Ser Leu
       1235                1240                1245
   Ile Ser Pro Asp Gly Ser Arg Lys Asn Pro Ala Arg Asn Cys Arg
       1250                1255                1260
   Asp Leu Lys Phe Cys His Pro Glu Leu Lys Ser Gly Glu Tyr Trp
       1265                1270                1275
   Val Asp Pro Asn Gln Gly Cys Lys Leu Asp Ala Ile Lys Val Phe
       1280                1285                1290
   Cys Asn Met Glu Thr Gly Glu Thr Cys Ile Ser Ala Asn Pro Leu
       1295                1300                1305
   Asn Val Pro Arg Lys His Trp Trp Thr Asp Ser Ser Ala Glu Lys
       1310                1315                1320
   Lys His Val Trp Phe Gly Glu Ser Met Asp Gly Gly Phe Gln Phe
       1325                1330                1335
   Ser Tyr Gly Asn Pro Glu Leu Pro Glu Asp Val Leu Asp Val Gln
       1340                1345                1350
   Leu Ala Phe Leu Arg Leu Leu Ser Ser Arg Ala Ser Gln Asn Ile
       1355                1360                1365
   Thr Tyr His Cys Lys Asn Ser Ile Ala Tyr Met Asp Gln Ala Ser
       1370                1375                1380
   Gly Asn Val Lys Lys Ala Leu Lys Leu Met Gly Ser Asn Glu Gly
       1385                1390                1395
   Glu Phe Lys Ala Glu Gly Asn Ser Lys Phe Thr Tyr Thr Val Leu
       1400                1405                1410
   Glu Asp Gly Cys Thr Lys His Thr Gly Glu Trp Ser Lys Thr Val
       1415                1420                1425
   Phe Glu Tyr Arg Thr Arg Lys Ala Val Arg Leu Pro Ile Val Asp
       1430                1435                1440
   Ile Ala Pro Tyr Asp Ile Gly Gly Pro Asp Gln Glu Phe Gly Val
   1445 1450 1455
   Asp Val Gly Pro Val Cys Phe Leu
   1460 1465
<210> 154
   <211> 20
   <212> PRT
   <213> Homo sapiens
<400> 154
   Ala Gly Ile Pro Gly Ala Pro Gly Leu Met Gly Ala Arg Gly Pro Pro
   1 5 10 15
   Gly Pro Ala Gly
   20
<210> 155
   <211> 19
   <212> PRT
   <213> Homo sapiens
<400> 155
   Lys Gly Asp Pro Gly Pro Pro Gly Ile Pro Gly Arg Asn Gly Asp Pro
   1 5 10 15
   Gly Ile Pro
<210> 156
   <211> 20
   <212> PRT
   <213> Homo sapiens
<400> 156
   Gly Leu Ala Gly Pro Pro Gly Met Pro Gly Pro Arg Gly Ser Pro Gly
   1 5 10 15
   Pro Gln Gly Val
   20
<210> 157
   <211> 21
   <212> PRT
   <213> Homo sapiens
<400> 157
   Gly Glu Arg Gly Leu Pro Gly Pro Pro Gly Ile Lys Gly Pro Ala Gly
   1               5                   10                  15
   Ile Pro Gly Phe Pro
               20
<210> 158
   <211> 22
   <212> PRT
   <213> Homo sapiens
<400> 158
   Gly Ile Ala Gly Ile Thr Gly Ala Arg Gly Leu Ala Gly Pro Pro Gly
   1               5                   10                  15
   Met Pro Gly Pro Arg Gly
               20
<210> 159
   <211> 23
   <212> PRT
   <213> Homo sapiens
<400> 159
   Gly Ile Lys Gly His Arg Gly Phe Pro Gly Asn Pro Gly Ala Pro Gly
   1               5                   10                  15
   Ser Pro Gly Pro Ala Gly Gln
               20
<210> 160
   <211> 23
   <212> PRT
   <213> Homo sapiens
<400> 160
   Ala Arg Gly Leu Ala Gly Pro Pro Gly Met Pro Gly Pro Arg Gly Ser
   1               5                   10                  15
   Pro Gly Pro Gln Gly Val Lys
               20
<210> 161
   <211> 25
   <212> PRT
   <213> Homo sapiens
<400> 161
   Ile Thr Gly Ala Arg Gly Leu Ala Gly Pro Pro Gly Met Pro Gly Pro
   1               5                   10                  15
   Arg Gly Ser Pro Gly Pro Gln Gly Val
               20                  25
<210> 162
   <211> 26
   <212> PRT
   <213> Homo sapiens
<400> 162
   Ile Thr Gly Ala Arg Gly Leu Ala Gly Pro Pro Gly Met Pro Gly Pro
   1               5                   10                  15
   Arg Gly Ser Pro Gly Pro Gln Gly Val Lys
               20                  25
<210> 163
   <211> 26
   <212> PRT
   <213> Homo sapiens
<400> 163
   Gly Ile Thr Gly Ala Arg Gly Leu Ala Gly Pro Pro Gly Met Pro Gly
   1               5                   10                  15
   Pro Arg Gly Ser Pro Gly Pro Gln Gly Val
               20                  25
<210> 164
   <211> 26
   <212> PRT
   <213> Homo sapiens
<400> 164
   Gly Val Lys Gly Glu Ser Gly Lys Pro Gly Ala Asn Gly Leu Ser Gly
   1               5                   10                  15
   Glu Arg Gly Pro Pro Gly Pro Gln Gly Leu
               20                  25
<210> 165
   <211> 26
   <212> PRT
   <213> Homo sapiens
<400> 165
   Gly Ser Arg Gly Ala Pro Gly Pro Gln Gly Pro Arg Gly Asp Lys Gly
   1               5                   10                  15
   Glu Thr Gly Glu Arg Gly Ala Ala Gly Ile
               20                  25
<210> 166
   <211> 28
   <212> PRT
   <213> Homo sapiens
<400> 166
   Pro Lys Gly Asp Ala Gly Gln Pro Gly Glu Lys Gly Ser Pro Gly Ala
   1               5                   10                  15
   Gln Gly Pro Pro Gly Ala Pro Gly Pro Leu Gly Ile
               20                  25
<210> 167
   <211> 27
   <212> PRT
   <213> Homo sapiens
<400> 167
   Gly Ile Thr Gly Ala Arg Gly Leu Ala Gly Pro Pro Gly Met Pro Gly
   1               5                   10                  15
   Pro Arg Gly Ser Pro Gly Pro Gln Gly Val Lys
               20                  25
<210> 168
   <211> 32
   <212> PRT
   <213> Homo sapiens
<400> 168
   Gly Leu Arg Gly Gly Ala Gly Pro Pro Gly Pro Glu Gly Gly Lys Gly
   1               5                   10                  15
   Ala Ala Gly Pro Pro Gly Pro Pro Gly Ala Ala Gly Thr Pro Gly Leu
               20                  25                  30
<210> 169
   <211> 35
   <212> PRT
   <213> Homo sapiens
<400> 169
   Gly His Ala Gly Ala Gln Gly Pro Pro Gly Pro Pro Gly Ile Asn Gly
   1 5 10 15
   Ser Pro Gly Gly Lys Gly Glu Met Gly Pro Ala Gly Ile Pro Gly Ala
   20 25 30
   Pro Gly Leu
   35
<210> 170
   <211> 48
   <212> PRT
   <213> Homo sapiens
<400> 170
   Ala Gly Lys Ser Gly Asp Arg Gly Glu Ser Gly Pro Ala Gly Pro Ala
   1 5 10 15
   Gly Ala Pro Gly Pro Ala Gly Ser Arg Gly Ala Pro Gly Pro Gln Gly
   20 25 30
   Pro Arg Gly Asp Lys Gly Glu Thr Gly Glu Arg Gly Ala Ala Gly Ile
   35 40 45
<210> 171
   <211> 50
   <212> PRT
   <213> Homo sapiens
<400> 171
   Gly Leu Gln Gly Leu Pro Gly Thr Gly Gly Pro Pro Gly Glu Asn Gly
   1 5 10 15
   Lys Pro Gly Glu Pro Gly Pro Lys Gly Asp Ala Gly Ala Pro Gly Ala
   20 25 30
   Pro Gly Gly Lys Gly Asp Ala Gly Ala Pro Gly Glu Arg Gly Pro Pro
   35 40 45
   Gly Leu
   50
<210> 172
   <211> 21
   <212> PRT
   <213> Homo sapiens
<400> 172
   Gly Glu Arg Gly Leu Pro Gly Pro Pro Gly Ile Lys Gly Pro Ala Gly
   1               5                   10                  15
   Ile Pro Gly Phe Pro
               20
<210> 173
   <211> 24
   <212> PRT
   <213> Homo sapiens
<400> 173
   Ala Val Gly Gly Leu Ala Gly Tyr Pro Gly Pro Ala Gly Pro Pro Gly
   1               5                   10                  15
   Pro Pro Gly Pro Pro Gly Thr Ser
               20
<210> 174
   <211> 24
   <212> PRT
   <213> Homo sapiens
<400> 174
   Lys Asp Gly Thr Ser Gly His Pro Gly Pro Ile Gly Pro Pro Gly Pro
   1               5                   10                  15
   Arg Gly Asn Arg Gly Glu Arg Gly
               20
<210> 175
   <211> 29
   <212> PRT
   <213> Homo sapiens
<400> 175
   Ala Val Gly Gly Leu Ala Gly Tyr Pro Gly Pro Ala Gly Pro Pro Gly
   1               5                   10                  15
   Pro Pro Gly Pro Pro Gly Thr Ser Gly His Pro Gly Ser
               20                  25
<210> 176
   <211> 29
   <212> PRT
   <213> Homo sapiens
<400> 176
   Gly Ile Ala Gly Ile Thr Gly Ala Arg Gly Leu Ala Gly Pro Pro Gly
   1 5 10 15
   Met Pro Gly Pro Arg Gly Ser Pro Gly Pro Gln Gly Val
   20 25
<210> 177
   <211> 31
   <212> PRT
   <213> Homo sapiens
<400> 177
   Ala Pro Gly Ala Pro Gly Gly Lys Gly Asp Ala Gly Ala Pro Gly Glu
   1 5 10 15
   Arg Gly Pro Pro Gly Leu Ala Gly Ala Pro Gly Leu Arg Gly Gly
   20 25 30
<210> 178
   <211> 32
   <212> PRT
   <213> Homo sapiens
<400> 178
   Ala Val Gly Gly Leu Ala Gly Tyr Pro Gly Pro Ala Gly Pro Pro Gly
   1 5 10 15
   Pro Pro Gly Pro Pro Gly Thr Ser Gly His Pro Gly Ser Pro Gly Ser
   20 25 30
<210> 179
   <211> 22
   <212> PRT
   <213> Homo sapiens
<400> 179
   Ala Ile Gly Ser Pro Gly Pro Ala Gly Pro Arg Gly Pro Val Gly Pro
   1 5 10 15
   Ser Gly Pro Pro Gly Lys
   20
<210> 180
   <211> 23
   <212> PRT
   <213> Homo sapiens
<400> 180
   Gly Ala Ile Gly Ser Pro Gly Pro Ala Gly Pro Arg Gly Pro Val Gly
   1               5                   10                  15
   Pro Ser Gly Pro Pro Gly Lys
               20
<210> 181
   <211> 15
   <212> PRT
   <213> Homo sapiens
<400> 181
   Pro Ala Gly Gln Gln Gly Ala Ile Gly Ser Pro Gly Pro Ala Gly
   1               5                   10                  15
<210> 182
   <211> 20
   <212> PRT
   <213> Homo sapiens
<400> 182
   Gly Gly Pro Pro Gly Val Ala Gly Pro Pro Gly Gly Ser Gly Pro Ala
   1               5                   10                  15
   Gly Pro Pro Gly
               20
<210> 183
   <211> 19
   <212> PRT
   <213> Homo sapiens
<400> 183
   Leu Ala Gly Pro Pro Gly Met Pro Gly Pro Arg Gly Ser Pro Gly Pro
   1               5                   10                  15
   Gln Gly Val
<210> 184
   <211> 19
   <212> PRT
   <213> Homo sapiens
<400> 184
   Gly Leu Ser Gly Glu Arg Gly Pro Pro Gly Pro Gln Gly Leu Pro Gly
   1               5                   10                  15
   Leu Ala Gly
<210> 185
   <211> 21
   <212> PRT
   <213> Homo sapiens
<400> 185
   Arg Gly Leu Ala Gly Pro Pro Gly Met Pro Gly Pro Arg Gly Ser Pro
   1               5                   10                  15
   Gly Pro Gln Gly Val
               20
<210> 186
   <211> 21
   <212> PRT
   <213> Homo sapiens
<400> 186
   Gly Leu Ala Gly Pro Pro Gly Met Pro Gly Pro Arg Gly Ser Pro Gly
   1               5                   10                  15
   Pro Gln Gly Val Lys
               20
<210> 187
   <211> 22
   <212> PRT
   <213> Homo sapiens
<400> 187
   Arg Gly Leu Ala Gly Pro Pro Gly Met Pro Gly Pro Arg Gly Ser Pro
   1               5                   10                  15
   Gly Pro Gln Gly Val Lys
               20
<210> 188
   <211> 21
   <212> PRT
   <213> Homo sapiens
<400> 188
   Gly Pro Gln Gly Pro Pro Gly Lys Asn Gly Glu Thr Gly Pro Gln Gly
   1               5                   10                  15
   Pro Pro Gly Pro Thr
               20
<210> 189
   <211> 22
   <212> PRT
   <213> Homo sapiens
<400> 189
   Gly Val Lys Gly Glu Arg Gly Ser Pro Gly Gly Pro Gly Ala Ala Gly
   1               5                   10                  15
   Phe Pro Gly Ala Arg Gly
               20
<210> 190
   <211> 22
   <212> PRT
   <213> Homo sapiens
<400> 190
   Ala Arg Gly Leu Ala Gly Pro Pro Gly Met Pro Gly Pro Arg Gly Ser
   1               5                   10                  15
   Pro Gly Pro Gln Gly Val
               20
<210> 191
   <211> 23
   <212> PRT
   <213> Homo sapiens
<400> 191
   Asn Gly Leu Ser Gly Glu Arg Gly Pro Pro Gly Pro Gln Gly Leu Pro
   1               5                   10                  15
   Gly Leu Ala Gly Thr Ala Gly
               20
<210> 192
   <211> 24
   <212> **PRT**
   <213> Homo sapiens
<400> 192
   Ala Val Gly Gly Leu Ala Gly Tyr Pro Gly Pro Ala Gly Pro Pro Gly
   1               5                   10                  15
   Pro Pro Gly Pro Pro Gly Thr Ser
               20
<210> 193
   <211> 24
   <212> PRT
   <213> Homo sapiens
<400> 193
   Gly Ser Pro Gly Gly Lys Gly Glu Met Gly Pro Ala Gly Ile Pro Gly
   1 5 10 15
   Ala Pro Gly Leu Met Gly Ala Arg
   20
<210> 194
   <211> 24
   <212> PRT
   <213> Homo sapiens
<400> 194
   Thr Gly Ala Arg Gly Leu Ala Gly Pro Pro Gly Met Pro Gly Pro Arg
   1 5 10 15
   Gly Ser Pro Gly Pro Gln Gly Val
   20
<210> 195
   <211> 25
   <212> PRT
   <213> Homo sapiens
<400> 195
   Val Lys Gly Glu Ser Gly Lys Pro Gly Ala Asn Gly Leu Ser Gly Glu
   1 5 10 15
   Arg Gly Pro Pro Gly Pro Gln Gly Leu
   20 25
<210> 196
   <211> 53
   <212> PRT
   <213> Homo sapiens
<400> 196
   Gly Val Lys Gly Glu Arg Gly Ser Pro Gly Gly Pro Gly Ala Ala Gly
   1 5 10 15
   Phe Pro Gly Ala Arg Gly Leu Pro Gly Pro Pro Gly Ser Asn Gly Asn
   20 25 30
   Pro Gly Pro Pro Gly Pro Ser Gly Ser Pro Gly Lys Asp Gly Pro Pro
   35 40 45
   Gly Pro Ala Gly Asn
   50
<210> 197
   <211> 32
   <212> PRT
   <213> Homo sapiens
<400> 197
   Gly Ser Pro Gly Ala Gln Gly Pro Pro Gly Ala Pro Gly Pro Leu Gly
   1               5                   10                  15
   Ile Ala Gly Ile Thr Gly Ala Arg Gly Leu Ala Gly Pro Pro Gly Met
               20                  25                  30
<210> 198
   <211> 32
   <212> PRT
   <213> Homo sapiens
<400> 198
   Arg Gly Ala Pro Gly Glu Lys Gly Glu Gly Gly Pro Pro Gly Val Ala
   1               5                   10                  15
   Gly Pro Pro Gly Gly Ser Gly Pro Ala Gly Pro Pro Gly Pro Gln Gly
               20                  25                  30
<210> 199
   <211> 32
   <212> PRT
   <213> Homo sapiens
<400> 199
   Arg Gly Ala Pro Gly Glu Lys Gly Glu Gly Gly Pro Pro Gly Val Ala
   1               5                   10                  15
   Gly Pro Pro Gly Gly Ser Gly Pro Ala Gly Pro Pro Gly Pro Gln Gly
               20                  25                  30
<210> 200
   <211> 29
   <212> PRT
   <213> Homo sapiens
<400> 200
   Gly Ile Ala Gly Ile Thr Gly Ala Arg Gly Leu Ala Gly Pro Pro Gly
   1               5                   10                  15
   Met Pro Gly Pro Arg Gly Ser Pro Gly Pro Gln Gly Val
               20                  25
<210> 201
   <211> 14
   <212> PRT
   <213> Homo sapiens
<400> 201
   Gly Ile Lys Gly Pro Ala Gly Ile Pro Gly Phe Pro Gly Met
   1               5                   10
<210> 202
   <211> 14
   <212> PRT
   <213> Homo sapiens
<400> 202
   Gly Gln Pro Gly Val Met Gly Phe Pro Gly Pro Lys Gly Asn
   1               5                   10
<210> 203
   <211> 16
   <212> PRT
   <213> Homo sapiens
<400> 203
   Gly Ile Lys Gly Pro Ala Gly Ile Pro Gly Phe Pro Gly Met Lys Gly
   1               5                   10                  15
<210> 204
   <211> 17
   <212> PRT
   <213> Homo sapiens
<400> 204
   Gly Ile Lys Gly Pro Ala Gly Ile Pro Gly Phe Pro Gly Met Lys Gly
   1               5                   10                  15
   His
<210> 205
   <211> 19
   <212> PRT
   <213> Homo sapiens
<400> 205
   Ile Pro Gly Ala Pro Gly Leu Met Gly Ala Arg Gly Pro Pro Gly Pro
   1               5                   10                  15
   Ala Gly Ala
<210> 206
   <211> 19
   <212> PRT
   <213> Homo sapiens
<400> 206
   Gly Glu Arg Gly Leu Pro Gly Pro Pro Gly Ile Lys Gly Pro Ala Gly
   1               5                   10                  15
   Ile Pro Gly
<210> 207
   <211> 20
   <212> PRT
   <213> Homo sapiens
<400> 207
   Gly Ile Pro Gly Ala Pro Gly Leu Met Gly Ala Arg Gly Pro Pro Gly
   1               5                   10                  15
   Pro Ala Gly Ala
               20
<210> 208
   <211> 20
   <212> PRT
   <213> Homo sapiens
<400> 208
   Gly Phe Arg Gly Pro Ala Gly Pro Asn Gly Ile Pro Gly Glu Lys Gly
   1               5                   10                  15
   Pro Ala Gly Glu
               20
<210> 209
   <211> 20
   <212> PRT
   <213> Homo sapiens
<400> 209
   Pro Gly Ile Pro Gly Gln Pro Gly Ser Pro Gly Ser Pro Gly Pro Pro
   1               5                   10                  15
   Gly Ile Cys Glu
               20
<210> 210
   <211> 21
   <212> PRT
   <213> Homo sapiens
<400> 210
   Gly Glu Arg Gly Leu Pro Gly Pro Pro Gly Ile Lys Gly Pro Ala Gly
   1 5 10 15
   Ile Pro Gly Phe Pro
   20
<210> 211
   <211> 23
   <212> PRT
   <213> Homo sapiens
<400> 211
   Ala Val Gly Gly Leu Ala Gly Tyr Pro Gly Pro Ala Gly Pro Pro Gly
   1 5 10 15
   Pro Pro Gly Pro Pro Gly Thr
   20
<210> 212
   <211> 23
   <212> PRT
   <213> Homo sapiens
<400> 212
   Gly Val Lys Gly Glu Arg Gly Ser Pro Gly Gly Pro Gly Ala Ala Gly
   1 5 10 15
   Phe Pro Gly Ala Arg Gly Leu
   20
<210> 213
   <211> 26
   <212> PRT
   <213> Homo sapiens
<400> 213
   Gly Asp Ala Gly Ala Pro Gly Ala Pro Gly Gly Lys Gly Asp Ala Gly
   1 5 10 15
   Ala Pro Gly Glu Arg Gly Pro Pro Gly Leu
   20 25
<210> 214
   <211> 25
   <212> PRT
   <213> Homo sapiens
<400> 214
   Gln Gly Pro Pro Gly Pro Thr Gly Pro Gly Gly Asp Lys Gly Asp Thr
   1 5 10 15
   Gly Pro Pro Gly Pro Gln Gly Leu Gln
   20 25
<210> 215
   <211> 25
   <212> PRT
   <213> Homo sapiens
<400> 215
   Gly Ile Asn Gly Ser Pro Gly Gly Lys Gly Glu Met Gly Pro Ala Gly
   1 5 10 15
   Ile Pro Gly Ala Pro Gly Leu Met Gly
   20 25
<210> 216
   <211> 26
   <212> PRT
   <213> Homo sapiens
<400> 216
   Gln Gly Pro Pro Gly Glu Pro Gly Gln Ala Gly Pro Ser Gly Pro Pro
   1 5 10 15
   Gly Pro Pro Gly Ala Ile Gly Pro Ser Gly
   20 25
<210> 217
   <211> 26
   <212> PRT
   <213> Homo sapiens
<400> 217
   Pro Gly Pro Pro Gly Ile Asn Gly Ser Pro Gly Gly Lys Gly Glu Met
   1 5 10 15
   Gly Pro Ala Gly Ile Pro Gly Ala Pro Gly
   20 25
<210> 218
   <211> 33
   <212> PRT
   <213> Homo sapiens
<400> 218
   Arg Gly Leu Pro Gly Pro Pro Gly Ser Asn Gly Asn Pro Gly Pro Pro
   1               5                   10                  15
   Gly Pro Ser Gly Ser Pro Gly Lys Asp Gly Pro Pro Gly Pro Ala Gly
               20                  25                  30
   Asn
<210> 219
   <211> 32
   <212> PRT
   <213> Homo sapiens
<400> 219
   Gly Lys Asn Gly Glu Thr Gly Pro Gln Gly Pro Pro Gly Pro Thr Gly
   1               5                   10                  15
   Pro Gly Gly Asp Lys Gly Asp Thr Gly Pro Pro Gly Pro Gln Gly Leu
               20                  25                  30
<210> 220
   <211> 32
   <212> PRT
   <213> Homo sapiens
<400> 220
   Gly Leu Pro Gly Ile Ala Gly Pro Arg Gly Ser Pro Gly Glu Arg Gly
   1               5                   10                  15
   Glu Thr Gly Pro Pro Gly Pro Ala Gly Phe Pro Gly Ala Pro Gly Gln
               20                  25                  30
<210> 221
   <211> 35
   <212> PRT
   <213> Homo sapiens
<400> 221
   Gly Ile Asn Gly Ser Pro Gly Gly Lys Gly Glu Met Gly Pro Ala Gly
   1               5                   10                  15
   Ile Pro Gly Ala Pro Gly Leu Met Gly Ala Arg Gly Pro Pro Gly Pro
               20                  25                  30
   Ala Gly Ala
           35
<210> 222
   <211> 36
   <212> PRT
   <213> Homo sapiens
<400> 222
   Pro Gly Ile Asn Gly Ser Pro Gly Gly Lys Gly Glu Met Gly Pro Ala
   1               5                   10                  15
   Gly Ile Pro Gly Ala Pro Gly Leu Met Gly Ala Arg Gly Pro Pro Gly
               20                  25                  30
   Pro Ala Gly Ala
           35
<210> 223
   <211> 40
   <212> PRT
   <213> Homo sapiens
<400> 223
   Pro Pro Gly Glu Asn Gly Lys Pro Gly Glu Pro Gly Pro Lys Gly Asp
   1               5                   10                  15
   Ala Gly Ala Pro Gly Ala Pro Gly Gly Lys Gly Asp Ala Gly Ala Pro
               20                  25                  30
   Gly Glu Arg Gly Pro Pro Gly Leu
           35                  40
<210> 224
   <211> 38
   <212> PRT
   <213> Homo sapiens
<400> 224
   Gly Leu Lys Gly Glu Asn Gly Leu Pro Gly Glu Asn Gly Ala Pro Gly
   1               5                   10                  15
   Pro Met Gly Pro Arg Gly Ala Pro Gly Glu Arg Gly Arg Pro Gly Leu
               20                  25                  30
   Pro Gly Ala Ala Gly Ala
           35
<210> 225
   <211> 41
   <212> PRT
   <213> Homo sapiens
<400> 225
   Gly Asn Thr Gly Ala Pro Gly Ser Pro Gly Val Ser Gly Pro Lys Gly
   1               5                   10                  15
   Asp Ala Gly Gln Pro Gly Glu Lys Gly Ser Pro Gly Ala Gln Gly Pro
               20                  25                  30
   Pro Gly Ala Pro Gly Pro Leu Gly Ile
           35                  40
<210> 226
   <211> 41
   <212> PRT
   <213> Homo sapiens
<400> 226
   Gly Leu Met Gly Ala Arg Gly Pro Pro Gly Pro Ala Gly Ala Asn Gly
   1               5                   10                  15
   Ala Pro Gly Leu Arg Gly Gly Ala Gly Glu Pro Gly Lys Asn Gly Ala
               20                  25                  30
   Lys Gly Glu Pro Gly Pro Arg Gly Glu
           35                  40
<210> 227
   <211> 50
   <212> PRT
   <213> Homo sapiens
<400> 227
   Gly Leu Arg Gly Gly Ala Gly Pro Pro Gly Pro Glu Gly Gly Lys Gly
   1               5                   10                  15
   Ala Ala Gly Pro Pro Gly Pro Pro Gly Ala Ala Gly Thr Pro Gly Leu
               20                  25                  30
   Gln Gly Met Pro Gly Glu Arg Gly Gly Leu Gly Ser Pro Gly Pro Lys
           35                  40                  45
   Gly Asp
       50
<210> 228
   <211> 26
   <212> PRT
   <213> Homo sapiens
<400> 228
   Gly Gln Gln Gly Ala Ile Gly Ser Pro Gly Pro Ala Gly Pro Arg Gly
   1               5                   10                  15
   Pro Val Gly Pro Ser Gly Pro Pro Gly Lys
               20                  25
<210> 229
   <211> 24
   <212> PRT
   <213> Homo sapiens
<400> 229
   Lys Gly Asp Pro Gly Pro Pro Gly Ile Pro Gly Arg Asn Gly Asp Pro
   1               5                   10                  15
   Gly Ile Pro Gly Gln Pro Gly Ser
               20
<210> 230
   <211> 26
   <212> PRT
   <213> Homo sapiens
<400> 230
   Gly Leu Arg Gly Gly Ala Gly Pro Pro Gly Pro Glu Gly Gly Lys Gly
   1               5                   10                  15
   Ala Ala Gly Pro Pro Gly Pro Pro Gly Ala
               20                  25
<210> 231
   <211> 32
   <212> PRT
   <213> Homo sapiens
<400> 231
   Gly Lys Asn Gly Glu Thr Gly Pro Gln Gly Pro Pro Gly Pro Thr Gly
   1               5                   10                  15
   Pro Gly Gly Asp Lys Gly Asp Thr Gly Pro Pro Gly Pro Gln Gly Leu
               20                  25                  30
<210> 232
   <211> 23
   <212> PRT
   <213> Homo sapiens
<400> 232
   Gly Tyr Gln Gly Pro Pro Gly Glu Pro Gly Gln Ala Gly Pro Ser Gly
   1 5 10 15
   Pro Pro Gly Pro Pro Gly Ala
   20
<210> 233
   <211> 20
   <212> PRT
   <213> Homo sapiens
<400> 233
   Gly Val Ala Gly Pro Pro Gly Gly Ser Gly Pro Ala Gly Pro Pro Gly
   1 5 10 15
   Pro Gln Gly Val
   20
<210> 234
   <211> 35
   <212> PRT
   <213> Homo sapiens
<400> 234
   Gly Asp Lys Gly Glu Pro Gly Gly Pro Gly Ala Asp Gly Val Pro Gly
   1 5 10 15
   Lys Asp Gly Pro Arg Gly Pro Thr Gly Pro Ile Gly Pro Pro Gly Pro
   20 25 30
   Ala Gly Gln
   35
<210> 235
   <211> 17
   <212> PRT
   <213> Homo sapiens
<400> 235
   Gln Gly His Ala Gly Ala Gln Gly Pro Pro Gly Pro Pro Gly Ile Asn
   1 5 10 15
   Gly
<210> 236
   <211> 11
   <212> PRT
   <213> Homo sapiens
<400> 236
   Ala Gly Glu Arg Gly Ala Pro Gly Pro Ala Gly
   1 5 10
<210> 237
   <211> 11
   <212> PRT
   <213> Homo sapiens
<400> 237
   Ala Gly Ile Pro Gly Phe Pro Gly Met Lys Gly
   1 5 10
<210> 238
   <211> 12
   <212> PRT
   <213> Homo sapiens
<400> 238
   Phe Pro Gly Met Lys Gly His Arg Gly Phe Asp Gly
   1 5 10
<210> 239
   <211> 14
   <212> PRT
   <213> Homo sapiens
<400> 239
   Gly Phe Pro Gly Ala Arg Gly Leu Pro Gly Pro Pro Gly Ser
   1 5 10
<210> 240
   <211> 15
   <212> PRT
   <213> Homo sapiens
<400> 240
   Ala Gly Phe Pro Gly Ala Arg Gly Leu Pro Gly Pro Pro Gly Ser
   1 5 10 15
<210> 241
   <211> 15
   <212> PRT
   <213> Homo sapiens
<400> 241
   Pro Pro Gly Pro Pro Gly Pro Pro Gly Thr Ser Gly His Pro Gly
   1 5 10 15
<210> 242
   <211> 13
   <212> PRT
   <213> Homo sapiens
<400> 242
   Gly Phe Pro Gly Met Lys Gly His Arg Gly Phe Asp Gly
   1               5                   10
<210> 243
   <211> 17
   <212> PRT
   <213> Homo sapiens
<400> 243
   Gln Pro Gly Asp Lys Gly Glu Gly Gly Ala Pro Gly Leu Pro Gly Ile
   1               5                   10                  15
   Ala
<210> 244
   <211> 17
   <212> PRT
   <213> Homo sapiens
<400> 244
   Arg Gly Asp Lys Gly Glu Thr Gly Glu Arg Gly Ala Ala Gly Ile Lys
   1               5                   10                  15
   Gly
<210> 245
   <211> 17
   <212> **PRT**
   <213> Homo sapiens
<400> 245
   Asp Gly Arg Asn Gly Glu Lys Gly Glu Thr Gly Ala Pro Gly Leu Lys
   1               5                   10                  15
   Gly
<210> 246
   <211> 20
   <212> PRT
   <213> Homo sapiens
<400> 246
   Ala Gly Gln Pro Gly Asp Lys Gly Glu Gly Gly Ala Pro Gly Leu Pro
   1               5                   10                  15
   Gly Ile Ala Gly
               20
<210> 247
   <211> 19
   <212> PRT
   <213> Homo sapiens
<400> 247
   Gly Gly Pro Pro Gly Glu Asn Gly Lys Pro Gly Glu Pro Gly Pro Lys
     1               5                   10                  15
   Gly Asp Ala
<210> 248
   <211> 17
   <212> PRT
   <213> Homo sapiens
<400> 248
   Ala Gly Ile Pro Gly Phe Pro Gly Met Lys Gly His Arg Gly Phe Asp
   1               5                   10                  15
   Gly
<210> 249
   <211> 20
   <212> PRT
   <213> Homo sapiens
<400> 249
   Arg Gly Gly Ala Gly Glu Pro Gly Lys Asn Gly Ala Lys Gly Glu Pro
   1               5                   10                  15
   Gly Pro Arg Gly
               20
<210> 250
   <211> 26
   <212> PRT
   <213> Homo sapiens
<400> 250
   Lys Gly Glu Arg Gly Ser Pro Gly Gly Pro Gly Ala Ala Gly Phe Pro
   1               5                   10                  15
   Gly Ala Arg Gly Leu Pro Gly Pro Pro Gly
               20                  25
<210> 251
   <211> 27
   <212> PRT
   <213> Homo sapiens
<400> 251
   Ile Pro Gly Val Pro Gly Ala Lys Gly Glu Asp Gly Lys Asp Gly Ser
   1               5                   10                  15
   Pro Gly Glu Pro Gly Ala Asn Gly Leu Pro Gly
               20                  25
<210> 252
   <211> 28
   <212> PRT
   <213> Homo sapiens
<400> 252
   Gly Ala Ala Gly Phe Pro Gly Ala Arg Gly Leu Pro Gly Pro Pro Gly
   1               5                   10                  15
   Ser Asn Gly Asn Pro Gly Pro Pro Gly Pro Ser Gly
               20                  25
<210> 253
   <211> 26
   <212> PRT
   <213> Homo sapiens
<400> 253
   Arg Pro Gly Pro Pro Gly Pro Ser Gly Pro Arg Gly Gln Pro Gly Val
   1               5                   10                  15
   Met Gly Phe Pro Gly Pro Lys Gly Asn Asp
               20                  25
<210> 254
   <211> 29
   <212> PRT
   <213> Homo sapiens
<400> 254
   Gln Gly Pro Pro Gly Pro Pro Gly Ile Asn Gly Ser Pro Gly Gly Lys
   1               5                   10                  15
   Gly Glu Met Gly Pro Ala Gly Ile Pro Gly Ala Pro Gly
               20                  25
<210> 255
   <211> 26
   <212> PRT
   <213> Homo sapiens
<400> 255
   Ala Gly Lys Asp Gly Glu Ser Gly Arg Pro Gly Arg Pro Gly Glu Arg
   1               5                   10                  15
   Gly Leu Pro Gly Pro Pro Gly Ile Lys Gly
               20                  25
<210> 256
   <211> 30
   <212> PRT
   <213> Homo sapiens
<400> 256
   Ala Gly Ala Arg Gly Asn Asp Gly Ala Arg Gly Ser Asp Gly Gln Pro
   1               5                   10                  15
   Gly Pro Pro Gly Pro Pro Gly Thr Ala Gly Phe Pro Gly Ser
               20                  25                  30
<210> 257
   <211> 31
   <212> PRT
   <213> Homo sapiens
<400> 257
   Ser Pro Gly Val Ser Gly Pro Lys Gly Asp Ala Gly Gln Pro Gly Glu
   1               5                   10                  15
   Lys Gly Ser Pro Gly Ala Gln Gly Pro Pro Gly Ala Pro Gly Pro
               20                  25                  30
<210> 258
   <211> 32
   <212> PRT
   <213> Homo sapiens
<400> 258
   Arg Gly Ser Asp Gly Gln Pro Gly Pro Pro Gly Pro Pro Gly Thr Ala
   1               5                   10                  15
   Gly Phe Pro Gly Ser Pro Gly Ala Lys Gly Glu Val Gly Pro Ala Gly
               20                  25                  30
<210> 259
   <211> 32
   <212> PRT
   <213> Homo sapiens
<400> 259
   Gln Gly Pro Pro Gly Pro Pro Gly Ile Asn Gly Ser Pro Gly Gly Lys
   1               5                   10                  15
   Gly Glu Met Gly Pro Ala Gly Ile Pro Gly Ala Pro Gly Leu Met Gly
               20                  25                  30
<210> 260
   <211> 33
   <212> PRT
   <213> Homo sapiens
<400> 260
   Ala Gly Pro Pro Gly Pro Pro Gly Pro Pro Gly Thr Ser Gly His Pro
   1               5                   10                  15
   Gly Ser Pro Gly Ser Pro Gly Tyr Gln Gly Pro Pro Gly Glu Pro Gly
               20                  25                  30
   Gln
<210> 261
   <211> 33
   <212> PRT
   <213> Homo sapiens
<400> 261
   Phe Pro Gly Ala Pro Gly Gln Asn Gly Glu Pro Gly Gly Lys Gly Glu
   1               5                   10                  15
   Arg Gly Ala Pro Gly Glu Lys Gly Glu Gly Gly Pro Pro Gly Val Ala
               20                  25                  30
   Gly
<210> 262
   <211> 33
   <212> PRT
   <213> Homo sapiens
<400> 262
   Ala Gly Phe Pro Gly Ala Pro Gly Gln Asn Gly Glu Pro Gly Gly Lys
   1               5                   10                  15
   Gly Glu Arg Gly Ala Pro Gly Glu Lys Gly Glu Gly Gly Pro Pro Gly
               20                  25                  30
   Val
<210> 263
   <211> 41
   <212> PRT
   <213> Homo sapiens
<400> 263
   Ala Gly Ala Arg Gly Asn Asp Gly Ala Arg Gly Ser Asp Gly Gln Pro
   1               5                   10                  15
   Gly Pro Pro Gly Pro Pro Gly Thr Ala Gly Phe Pro Gly Ser Pro Gly
               20                  25                  30
   Ala Lys Gly Glu Val Gly Pro Ala Gly
                         35                  40
<210> 264
   <211> 53
   <212> PRT
   <213> Homo sapiens
<400> 264
   Arg Gly Ala Ala Gly Glu Pro Gly Arg Asp Gly Val Pro Gly Gly Pro
   1               5                   10                  15
   Gly Met Arg Gly Met Pro Gly Ser Pro Gly Gly Pro Gly Ser Asp Gly
               20                  25                  30
   Lys Pro Gly Pro Pro Gly Ser Gln Gly Glu Ser Gly Arg Pro Gly Pro
           35                  40                  45
   Pro Gly Pro Ser Gly
       50
<210> 265
   <211> 12
   <212> PRT
   <213> Homo sapiens
<400> 265
   Gly Ile Ala Gly Ile Thr Gly Ala Arg Gly Leu Ala
   1               5                   10
<210> 266
   <211> 18
   <212> PRT
   <213> Homo sapiens
<400> 266
   Ala Gly Pro Pro Gly Pro Pro Gly Ala Ala Gly Thr Pro Gly Leu Gln
   1               5                   10                  15
   Gly Met
<210> 267
   <211> 18
   <212> PRT
   <213> Homo sapiens
<400> 267
   Asn Gly Leu Ser Gly Glu Arg Gly Pro Pro Gly Pro Gln Gly Leu Pro
   1               5                   10                  15
   Gly Leu
<210> 268
   <211> 20
   <212> PRT
   <213> Homo sapiens
<400> 268
   Met Gly Ala Arg Gly Pro Pro Gly Pro Ala Gly Ala Asn Gly Ala Pro
   1               5                   10                  15
   Gly Leu Arg Gly
               20
<210> 269
   <211> 20
   <212> PRT
   <213> Homo sapiens
<400> 269
   Asn Gly Leu Ser Gly Glu Arg Gly Pro Pro Gly Pro Gln Gly Leu Pro
   1               5                   10                  15
   Gly Leu Ala Gly
               20
<210> 270
   <211> 23
   <212> PRT
   <213> Homo sapiens
<400> 270
   Gly Ile Ala Gly Ile Thr Gly Ala Arg Gly Leu Ala Gly Pro Pro Gly
   1               5                   10                  15
   Met Pro Gly Pro Arg Gly Ser
               20
<210> 271
   <211> 29
   <212> PRT
   <213> Homo sapiens
<400> 271
   Gly Ile Ala Gly Ile Thr Gly Ala Arg Gly Leu Ala Gly Pro Pro Gly
   1               5                   10                  15
   Met Pro Gly Pro Arg Gly Ser Pro Gly Pro Gln Gly Val
               20                  25
<210> 272
   <211> 32
   <212> PRT
   <213> Homo sapiens
<400> 272
   Arg Gly Gly Ala Gly Pro Pro Gly Pro Glu Gly Gly Lys Gly Ala Ala
   1               5                   10                  15
   Gly Pro Pro Gly Pro Pro Gly Ala Ala Gly Thr Pro Gly Leu Gln Gly
               20                  25                  30
<210> 273
   <211> 30
   <212> PRT
   <213> Homo sapiens
<400> 273
   Ser Gly Pro Lys Gly Asp Ala Gly Gln Pro Gly Glu Lys Gly Ser Pro
   1               5                   10                  15
   Gly Ala Gln Gly Pro Pro Gly Ala Pro Gly Pro Leu Gly Ile
               20                  25                  30
<210> 274
   <211> 31
   <212> PRT
   <213> Homo sapiens
<400> 274
   Gly Ile Ala Gly Ile Thr Gly Ala Arg Gly Leu Ala Gly Pro Pro Gly
   1               5                   10                  15
   Met Pro Gly Pro Arg Gly Ser Pro Gly Pro Gln Gly Val Lys Gly
               20                  25                  30
<210> 275
   <211> 37
   <212> PRT
   <213> Homo sapiens
<400> 275
   Ala Val Gly Gly Leu Ala Gly Tyr Pro Gly Pro Ala Gly Pro Pro Gly
   1               5                   10                  15
   Pro Pro Gly Pro Pro Gly Thr Ser Gly His Pro Gly Ser Pro Gly Ser
               20                  25                  30
   Pro Gly Tyr Gln Gly
           35
<210> 276
   <211> 40
   <212> PRT
   <213> Homo sapiens
<400> 276
   Glu Pro Gly Pro Gln Gly His Ala Gly Ala Gln Gly Pro Pro Gly Pro
   1               5                   10                  15
   Pro Gly Ile Asn Gly Ser Pro Gly Gly Lys Gly Glu Met Gly Pro Ala
               20                  25                  30
   Gly Ile Pro Gly Ala Pro Gly Leu
           35                  40
<210> 277
   <211> 12
   <212> PRT
   <213> Homo sapiens
<400> 277
   Ile Pro Gly Phe Pro Gly Met Lys Gly His Arg Gly
   1               5                   10
<210> 278
   <211> 17
   <212> PRT
   <213> Homo sapiens
<400> 278
   Arg Gly Ser Pro Gly Gly Pro Gly Ala Ala Gly Phe Pro Gly Ala Arg
   1 5 10 15
   Gly
<210> 279
   <211> 17
   <212> PRT
   <213> Homo sapiens
<400> 279
   Lys Gly Pro Ala Gly Ile Pro Gly Phe Pro Gly Met Lys Gly His Arg
   1 5 10 15
   Gly
<210> 280
   <211> 20
   <212> PRT
   <213> Homo sapiens
<400> 280
   Arg Gly Leu Ala Gly Pro Pro Gly Met Pro Gly Pro Arg Gly Ser Pro
   1 5 10 15
   Gly Pro Gln Gly
   20
<210> 281
   <211> 20
   <212> PRT
   <213> Homo sapiens
<400> 281
   Ala Gly Ile Thr Gly Ala Arg Gly Leu Ala Gly Pro Pro Gly Met Pro
   1 5 10 15
   Gly Pro Arg Gly
   20
<210> 282
   <211> 23
   <212> PRT
   <213> Homo sapiens
<400> 282
   Leu Gly Ile Ala Gly Ile Thr Gly Ala Arg Gly Leu Ala Gly Pro Pro
   1               5                   10                  15
   Gly Met Pro Gly Pro Arg Gly
               20
<210> 283
   <211> 23
   <212> PRT
   <213> Homo sapiens
<400> 283
   Thr Gly Ala Arg Gly Leu Ala Gly Pro Pro Gly Met Pro Gly Pro Arg
   1               5                   10                  15
   Gly Ser Pro Gly Pro Gln Gly
               20
<210> 284
   <211> 24
   <212> PRT
   <213> Homo sapiens
<400> 284
   Gln Gly Pro Pro Gly Pro Pro Gly Ile Asn Gly Ser Pro Gly Gly Lys
   1               5                   10                  15
   Gly Glu Met Gly Pro Ala Gly Ile
               20
<210> 285
   <211> 24
   <212> PRT
   <213> Homo sapiens
<400> 285
   Leu Pro Gly Pro Pro Gly Ile Lys Gly Pro Ala Gly Ile Pro Gly Phe
   1               5                   10                  15
   Pro Gly Met Lys Gly His Arg Gly
               20
<210> 286
   <211> 26
   <212> PRT
   <213> Homo sapiens
<400> 286
   Ala Gly Ile Thr Gly Ala Arg Gly Leu Ala Gly Pro Pro Gly Met Pro
   1               5                   10                  15
   Gly Pro Arg Gly Ser Pro Gly Pro Gln Gly
               20                  25
<210> 287
   <211> 26
   <212> PRT
   <213> Homo sapiens
<400> 287
   Thr Gly Ala Arg Gly Leu Ala Gly Pro Pro Gly Met Pro Gly Pro Arg
   1               5                   10                  15
   Gly Ser Pro Gly Pro Gln Gly Val Lys Gly
               20                  25
<210> 288
   <211> 26
   <212> PRT
   <213> Homo sapiens
<400> 288
   Arg Gly Leu Pro Gly Pro Pro Gly Ile Lys Gly Pro Ala Gly Ile Pro
   1               5                   10                  15
   Gly Phe Pro Gly Met Lys Gly His Arg Gly
               20                  25
<210> 289
   <211> 29
   <212> PRT
   <213> Homo sapiens
<400> 289
   Gly Arg Pro Gly Leu Pro Gly Ala Ala Gly Ala Arg Gly Asn Asp Gly
   1               5                   10                  15
   Ala Arg Gly Ser Asp Gly Gln Pro Gly Pro Pro Gly Pro
               20                  25
<210> 290
   <211> 29
   <212> PRT
   <213> Homo sapiens
<400> 290
   Asn Gly Ala Pro Gly Pro Met Gly Pro Arg Gly Ala Pro Gly Glu Arg
   1 5 10 15
   Gly Arg Pro Gly Leu Pro Gly Ala Ala Gly Ala Arg Gly
   20 25
<210> 291
   <211> 29
   <212> PRT
   <213> Homo sapiens
<400> 291
   Ala Gly Ser Arg Gly Ala Pro Gly Pro Gln Gly Pro Arg Gly Asp Lys
   1 5 10 15
   Gly Glu Thr Gly Glu Arg Gly Ala Ala Gly Ile Lys Gly
   20 25
<210> 292
   <211> 32
   <212> PRT
   <213> Homo sapiens
<400> 292
   Arg Gly Leu Ala Gly Pro Pro Gly Met Pro Gly Pro Arg Gly Ser Pro
   1 5 10 15
   Gly Pro Gln Gly Val Lys Gly Glu Ser Gly Lys Pro Gly Ala Asn Gly
   20 25 30
<210> 293
   <211> 34
   <212> PRT
   <213> Homo sapiens
<400> 293
   Arg Gly Leu Ala Gly Pro Pro Gly Met Pro Gly Pro Arg Gly Ser Pro
   1 5 10 15
   Gly Pro Gln Gly Val Lys Gly Glu Ser Gly Lys Pro Gly Ala Asn Gly
   20 25 30
   Leu Ser
<210> 294
   <211> 37
   <212> PRT
   <213> Homo sapiens
<400> 294
   Pro Gly Pro Pro Gly Ser Asn Gly Asn Pro Gly Pro Pro Gly Pro Ser
   1 5 10 15
   Gly Ser Pro Gly Lys Asp Gly Pro Pro Gly Pro Ala Gly Asn Thr Gly
   20 25 30
   Ala Pro Gly Ser Pro
   35
<210> 295
   <211> 35
   <212> PRT
   <213> Homo sapiens
<400> 295
   Thr Gly Ala Arg Gly Leu Ala Gly Pro Pro Gly Met Pro Gly Pro Arg
   1 5 10 15
   Gly Ser Pro Gly Pro Gln Gly Val Lys Gly Glu Ser Gly Lys Pro Gly
   20 25 30
   Ala Asn Gly
   35
<210> 296
   <211> 38
   <212> PRT
   <213> Homo sapiens
<400> 296
   Arg Gly Ala Pro Gly Glu Lys Gly Glu Gly Gly Pro Pro Gly Val Ala
   1 5 10 15
   Gly Pro Pro Gly Gly Ser Gly Pro Ala Gly Pro Pro Gly Pro Gln Gly
   20 25 30
   Val Lys Gly Glu Arg Gly
   35
<210> 297
   <211> 40
   <212> PRT
   <213> Homo sapiens
<400> 297
   Gly Gly Pro Pro Gly Val Ala Gly Pro Pro Gly Gly Ser Gly Pro Ala
   1               5                   10                  15
   Gly Pro Pro Gly Pro Gln Gly Val Lys Gly Glu Arg Gly Ser Pro Gly
               20                  25                  30
   Gly Pro Gly Ala Ala Gly Phe Pro
           35                  40
<210> 298
   <211> 48
   <212> PRT
   <213> Homo sapiens
<400> 298
   Lys Ser Gly Asp Arg Gly Glu Ser Gly Pro Ala Gly Pro Ala Gly Ala
   1               5                   10                  15
   Pro Gly Pro Ala Gly Ser Arg Gly Ala Pro Gly Pro Gln Gly Pro Arg
               20                  25                  30
   Gly Asp Lys Gly Glu Thr Gly Glu Arg Gly Ala Ala Gly Ile Lys Gly
           35                  40                  45
<210> 299
   <211> 12
   <212> PRT
   <213> Homo sapiens
<400> 299
   Ile Pro Gly Phe Pro Gly Met Lys Gly His Arg Gly
   1               5                   10
<210> 300
   <211> 14
   <212> PRT
   <213> Homo sapiens
<400> 300
   Arg Gly Leu Ala Gly Pro Pro Gly Met Pro Gly Pro Arg Gly
   1               5                   10
<210> 301
   <211> 16
   <212> PRT
   <213> Homo sapiens
<400> 301
   Gly Pro Gln Gly Leu Gln Gly Leu Pro Gly Thr Gly Gly Pro Pro Gly
   1               5                   10                  15
<210> 302
   <211> 17
   <212> PRT
   <213> Homo sapiens
<400> 302
   Lys Gly Pro Ala Gly Ile Pro Gly Phe Pro Gly Met Lys Gly His Arg
   1               5                   10                  15
   Gly
<210> 303
   <211> 21
   <212> PRT
   <213> Homo sapiens
<400> 303
   Arg Gly Leu Ala Gly Pro Pro Gly Met Pro Gly Pro Arg Gly Ser Pro
   1               5                   10                  15
   Gly Pro Gln Gly Val
               20
<210> 304
   <211> 23
   <212> PRT
   <213> Homo sapiens
<400> 304
   Gly Gly Pro Pro Gly Glu Asn Gly Lys Pro Gly Glu Pro Gly Pro Lys
   1               5                   10                  15
   Gly Asp Ala Gly Ala Pro Gly
               20
<210> 305
   <211> 24
   <212> PRT
   <213> Homo sapiens
<400> 305
   Ala Pro Gly Phe Arg Gly Pro Ala Gly Pro Asn Gly Ile Pro Gly Glu
   1               5                   10                  15
   Lys Gly Pro Ala Gly Glu Arg Gly
   20
<210> 306
   <211> 27
   <212> PRT
   <213> Homo sapiens
<400> 306
   Glu Lys Gly Ser Pro Gly Ala Gln Gly Pro Pro Gly Ala Pro Gly Pro
   1               5                   10                  15
   Leu Gly Ile Ala Gly Ile Thr Gly Ala Arg Gly
               20                  25
<210> 307
   <211> 27
   <212> PRT
   <213> Homo sapiens
<400> 307
   Glu Lys Gly Ser Pro Gly Ala Gln Gly Pro Pro Gly Ala Pro Gly Pro
   1               5                   10                  15
   Leu Gly Ile Ala Gly Ile Thr Gly Ala Arg Gly
               20                  25
<210> 308
   <211> 26
   <212> PRT
   <213> Homo sapiens
<400> 308
   Arg Gly Ala Pro Gly Phe Arg Gly Pro Ala Gly Pro Asn Gly Ile Pro
   1               5                   10                  15
   Gly Glu Lys Gly Pro Ala Gly Glu Arg Gly
               20                  25
<210> 309
   <211> 26
   <212> PRT
   <213> Homo sapiens
<400> 309
   Arg Gly Leu Pro Gly Pro Pro Gly Ile Lys Gly Pro Ala Gly Ile Pro
   1               5                   10                  15
   Gly Phe Pro Gly Met Lys Gly His Arg Gly
               20                  25
<210> 310
   <211> 29
   <212> PRT
   <213> Homo sapiens
<400> 310
   Arg Gly Pro Val Gly Pro Ser Gly Pro Pro Gly Lys Asp Gly Thr Ser
   1               5                   10                  15
   Gly His Pro Gly Pro Ile Gly Pro Pro Gly Pro Arg Gly
               20                  25
<210> 311
   <211> 27
   <212> PRT
   <213> Homo sapiens
<400> 311
   Ala Pro Gly Pro Gln Gly Pro Arg Gly Asp Lys Gly Glu Thr Gly Glu
   1               5                   10                  15
   Arg Gly Ala Ala Gly Ile Lys Gly His Arg Gly
               20                  25
<210> 312
   <211> 29
   <212> PRT
   <213> Homo sapiens
<400> 312
   Arg Gly Ala Pro Gly Pro Gln Gly Pro Arg Gly Asp Lys Gly Glu Thr
   1               5                   10                  15
   Gly Glu Arg Gly Ala Ala Gly Ile Lys Gly His Arg Gly
               20                  25
<210> 313
   <211> 32
   <212> PRT
   <213> Homo sapiens
<400> 313
   Arg Gly Phe Pro Gly Asn Pro Gly Ala Pro Gly Ser Pro Gly Pro Ala
   1               5                   10                  15
   Gly Gln Gln Gly Ala Ile Gly Ser Pro Gly Pro Ala Gly Pro Arg Gly
               20                  25                  30
<210> 314
   <211> 29
   <212> PRT
   <213> Homo sapiens
<400> 314
   Leu Pro Gly Pro Pro Gly Ile Lys Gly Pro Ala Gly Ile Pro Gly Phe
   1               5                   10                  15
   Pro Gly Met Lys Gly His Arg Gly Phe Asp Gly Arg Asn
               20                  25
<210> 315
   <211> 33
   <212> PRT
   <213> Homo sapiens
<400> 315
   Asp Ala Gly Gln Pro Gly Glu Lys Gly Ser Pro Gly Ala Gln Gly Pro
   1               5                   10                  15
   Pro Gly Ala Pro Gly Pro Leu Gly Ile Ala Gly Ile Thr Gly Ala Arg
               20                  25                  30
   Gly
<210> 316
   <211> 35
   <212> PRT
   <213> Homo sapiens
<400> 316
   Arg Gly Pro Thr Gly Pro Ile Gly Pro Pro Gly Pro Ala Gly Gln Pro
   1               5                   10                  15
   Gly Asp Lys Gly Glu Gly Gly Ala Pro Gly Leu Pro Gly Ile Ala Gly
               20                  25                  30
   Pro Arg Gly
           35
<210> 317
   <211> 35
   <212> PRT
   <213> Homo sapiens
<400> 317
   Lys Gly Asp Ala Gly Gln Pro Gly Glu Lys Gly Ser Pro Gly Ala Gln
   1               5                   10                  15
   Gly Pro Pro Gly Ala Pro Gly Pro Leu Gly Ile Ala Gly Ile Thr Gly
               20                  25                  30
   Ala Arg Gly
           35
<210> 318
   <211> 33
   <212> PRT
   <213> Homo sapiens
<400> 318
   Arg Asn Gly Glu Lys Gly Glu Thr Gly Ala Pro Gly Leu Lys Gly Glu
   1               5                   10                  15
   Asn Gly Leu Pro Gly Glu Asn Gly Ala Pro Gly Pro Met Gly Pro Arg
               20                  25                  30
   Gly
<210> 319
   <211> 35
   <212> PRT
   <213> Homo sapiens
<400> 319
   Ala Pro Gly Phe Arg Gly Pro Ala Gly Pro Asn Gly Ile Pro Gly Glu
   1               5                   10                  15
   Lys Gly Pro Ala Gly Glu Arg Gly Ala Pro Gly Pro Ala Gly Pro Arg
               20                  25                  30
   Gly Ala Ala
           35
<210> 320
   <211> 35
   <212> PRT
   <213> Homo sapiens
<400> 320
   Arg Gly Ala Pro Gly Phe Arg Gly Pro Ala Gly Pro Asn Gly Ile Pro
   1               5                   10                  15
   Gly Glu Lys Gly Pro Ala Gly Glu Arg Gly Ala Pro Gly Pro Ala Gly
               20                  25                  30
   Pro Arg Gly
           35
<210> 321
   <211> 35
   <212> PRT
   <213> Homo sapiens
<400> 321
   Arg Gly Ser Pro Gly Glu Arg Gly Glu Thr Gly Pro Pro Gly Pro Ala
   1               5                   10                  15
   Gly Phe Pro Gly Ala Pro Gly Gln Asn Gly Glu Pro Gly Gly Lys Gly
               20                  25                  30
   Glu Arg Gly
           35
<210> 322
   <211> 38
   <212> PRT
   <213> Homo sapiens
<400> 322
   Gly His Ala Gly Ala Gln Gly Pro Pro Gly Pro Pro Gly Ile Asn Gly
   1               5                   10                  15
   Ser Pro Gly Gly Lys Gly Glu Met Gly Pro Ala Gly Ile Pro Gly Ala
               20                  25                  30
   Pro Gly Leu Met Gly Ala
           35
<210> 323
   <211> 38
   <212> PRT
   <213> Homo sapiens
<400> 323
   Arg Gly Leu Ala Gly Pro Pro Gly Met Pro Gly Pro Arg Gly Ser Pro
   1               5                   10                  15
   Gly Pro Gln Gly Val Lys Gly Glu Ser Gly Lys Pro Gly Ala Asn Gly
               20                  25                  30
   Leu Ser Gly Glu Arg Gly
           35
<210> 324
   <211> 13
   <212> PRT
   <213> Homo sapiens
<400> 324
   Leu Gly Ile Ala Gly Ile Thr Gly Ala Arg Gly Leu Ala
   1               5                   10
<210> 325
   <211> 12
   <212> PRT
   <213> Homo sapiens
<400> 325
   Ile Pro Gly Phe Pro Gly Met Lys Gly His Arg Gly
   1               5                   10
<210> 326
   <211> 14
   <212> PRT
   <213> Homo sapiens
<400> 326
   Arg Gly Leu Ala Gly Pro Pro Gly Met Pro Gly Pro Arg Gly
   1               5                   10
<210> 327
   <211> 20
   <212> PRT
   <213> Homo sapiens
<400> 327
   Gln Gly Pro Pro Gly Ala Pro Gly Pro Leu Gly Ile Ala Gly Ile Thr
   1               5                   10                  15
   Gly Ala Arg Gly
               20
<210> 328
   <211> 20
   <212> PRT
   <213> Homo sapiens
<400> 328
   Ala Gly Ile Thr Gly Ala Arg Gly Leu Ala Gly Pro Pro Gly Met Pro
   1               5                   10                  15
   Gly Pro Arg Gly
               20
<210> 329
   <211> 23
   <212> PRT
   <213> Homo sapiens
<400> 329
   Ala Gly Ile Pro Gly Ala Pro Gly Leu Met Gly Ala Arg Gly Pro Pro
   1               5                   10                  15
   Gly Pro Ala Gly Ala Asn Gly
               20
<210> 330
   <211> 24
   <212> PRT
   <213> Homo sapiens
<400> 330
   Arg Gly Leu Ala Gly Pro Pro Gly Met Pro Gly Pro Arg Gly Ser Pro
   1               5                   10                  15
   Gly Pro Gln Gly Val Lys Gly Glu
               20
<210> 331
   <211> 26
   <212> PRT
   <213> Homo sapiens
<400> 331
   Lys Gly Ser Pro Gly Ala Gln Gly Pro Pro Gly Ala Pro Gly Pro Leu
   1               5                   10                  15
   Gly Ile Ala Gly Ile Thr Gly Ala Arg Gly
               20                  25
<210> 332
   <211> 24
   <212> PRT
   <213> Homo sapiens
<400> 332
   Leu Pro Gly Pro Pro Gly Ile Lys Gly Pro Ala Gly Ile Pro Gly Phe
   1               5                   10                  15
   Pro Gly Met Lys Gly His Arg Gly
               20
<210> 333
   <211> 24
   <212> PRT
   <213> Homo sapiens
<400> 333
   Lys Asp Gly Thr Ser Gly His Pro Gly Pro Ile Gly Pro Pro Gly Pro
   1               5                   10                  15
   Arg Gly Asn Arg Gly Glu Arg Gly
               20
<210> 334
   <211> 26
   <212> PRT
   <213> Homo sapiens
<400> 334
   Ala Gly Ile Thr Gly Ala Arg Gly Leu Ala Gly Pro Pro Gly Met Pro
     1               5                   10                  15
   Gly Pro Arg Gly Ser Pro Gly Pro Gln Gly
               20                  25
<210> 335
   <211> 27
   <212> PRT
   <213> Homo sapiens
<400> 335
   Arg Gly Leu Ala Gly Pro Pro Gly Met Pro Gly Pro Arg Gly Ser Pro
   1               5                   10                  15
   Gly Pro Gln Gly Val Lys Gly Glu Ser Gly Lys
               20                  25
<210> 336
   <211> 32
   <212> PRT
   <213> Homo sapiens
<400> 336
   Arg Gly Leu Ala Gly Pro Pro Gly Met Pro Gly Pro Arg Gly Ser Pro
   1               5                   10                  15
   Gly Pro Gln Gly Val Lys Gly Glu Ser Gly Lys Pro Gly Ala Asn Gly
               20                  25                  30
<210> 337
   <211> 34
   <212> PRT
   <213> Homo sapiens
<400> 337
   Arg Gly Leu Ala Gly Pro Pro Gly Met Pro Gly Pro Arg Gly Ser Pro
   1               5                   10                  15
   Gly Pro Gln Gly Val Lys Gly Glu Ser Gly Lys Pro Gly Ala Asn Gly
               20                  25                  30
   Leu Ser
<210> 338
   <211> 37
   <212> PRT
   <213> Homo sapiens
<400> 338
   Pro Gly Pro Pro Gly Ser Asn Gly Asn Pro Gly Pro Pro Gly Pro Ser
   1               5                   10                  15
   Gly Ser Pro Gly Lys Asp Gly Pro Pro Gly Pro Ala Gly Asn Thr Gly
               20                  25                  30
   Ala Pro Gly Ser Pro
           35
<210> 339
   <211> 38
   <212> PRT
   <213> Homo sapiens
<400> 339
   Arg Gly Ala Pro Gly Glu Lys Gly Glu Gly Gly Pro Pro Gly Val Ala
   1 5 10 15
   Gly Pro Pro Gly Gly Ser Gly Pro Ala Gly Pro Pro Gly Pro Gln Gly
   20 25 30
   Val Lys Gly Glu Arg Gly
   35
<210> 340
   <211> 44
   <212> PRT
   <213> Homo sapiens
<400> 340
   Lys Ser Gly Asp Arg Gly Glu Ser Gly Pro Ala Gly Pro Ala Gly Ala
   1 5 10 15
   Pro Gly Pro Ala Gly Ser Arg Gly Ala Pro Gly Pro Gln Gly Pro Arg
   20 25 30
   Gly Asp Lys Gly Glu Thr Gly Glu Arg Gly Ala Ala
   35 40
<210> 341
   <211> 53
   <212> PRT
   <213> Homo sapiens
<400> 341
   Arg Gly Ala Pro Gly Glu Lys Gly Glu Gly Gly Pro Pro Gly Val Ala
   1 5 10 15
   Gly Pro Pro Gly Gly Ser Gly Pro Ala Gly Pro Pro Gly Pro Gln Gly
   20 25 30
   Val Lys Gly Glu Arg Gly Ser Pro Gly Gly Pro Gly Ala Ala Gly Phe
   35 40 45
   Pro Gly Ala Arg Gly
   50
<210> 342
   <211> 6
   <212> PRT
   <213> Homo sapiens
<400> 342
   Ala Ile Gly Pro Ser Gly
   1 5
<210> 343
   <211> 6
   <212> PRT
   <213> Homo sapiens
<400> 343
   Gly Ile Pro Gly Ala Pro
   1 5
<210> 344
   <211> 6
   <212> PRT
   <213> Homo sapiens
<400> 344
   Ile Lys Gly His Arg Gly
   1 5
<210> 345
   <211> 6
   <212> PRT
   <213> Homo sapiens
<400> 345
   Val Lys Gly Glu Ser Gly
   1 5
<210> 346
   <211> 6
   <212> PRT
   <213> Homo sapiens
<400> 346
   His Ala Gly Ala Gln Gly
   1 5
<210> 347
   <211> 6
   <212> PRT
   <213> Homo sapiens
<400> 347
   Asp Gly Thr Ser Gly His
   1 5
<210> 348
   <211> 6
   <212> PRT
   <213> Homo sapiens
<400> 348
   Ile Gly Ser Pro Gly Pro
   1 5
<210> 349
   <211> 6
   <212> PRT
   <213> Homo sapiens
<400> 349
   Leu Ser Gly Glu Arg Gly
   1 5
<210> 350
   <211> 6
   <212> PRT
   <213> Homo sapiens
<400> 350
   Val Lys Gly Glu Arg Gly
   1 5
<210> 351
   <211> 6
   <212> PRT
   <213> Homo sapiens
<400> 351
   Gly Ala Arg Gly Leu Ala
   1 5
<210> 352
   <211> 6
   <212> PRT
   <213> Homo sapiens
<400> 352
   Gly Ala Pro Gly Glu Lys
   1 5
<210> 353
   <211> 6
   <212> PRT
   <213> Homo sapiens
<400> 353
   Ile Lys Gly Pro Ala Gly
   1 5
<210> 354
   <211> 6
   <212> PRT
   <213> Homo sapiens
<400> 354
   Gly Ile Pro Gly Gln Pro
   1 5
<210> 355
   <211> 6
   <212> PRT
   <213> Homo sapiens
<400> 355
   Gly Pro Pro Gly Pro Thr
   1 5
<210> 356
   <211> 6
   <212> PRT
   <213> Homo sapiens
<400> 356
   Lys Asn Gly Glu Thr Gly
   1 5
<210> 357
   <211> 6
   <212> PRT
   <213> Homo sapiens
<400> 357
   Leu Lys Gly Glu Asn Gly
   1 5
<210> 358
   <211> 6
   <212> PRT
   <213> Homo sapiens
<400> 358
   Gly Asp Pro Gly Pro Pro
   1 5
<210> 359
   <211> 6
   <212> PRT
   <213> Homo sapiens
<400> 359
   Gly Asp Pro Gly Pro Pro
   1 5
<210> 360
   <211> 6
   <212> PRT
   <213> Homo sapiens
<400> 360
   Phe Pro Gly Ala Arg Gly
   1 5
<210> 361
   <211> 6
   <212> PRT
   <213> Homo sapiens
<400> 361
   Gly Asp Lys Gly Glu Thr
   1 5
<210> 362
   <211> 6
   <212> PRT
   <213> Homo sapiens
<400> 362
   Gly Gly Ala Gly Glu Pro
   1 5
<210> 363
<400> 363
   000
<210> 364
   <211> 6
   <212> PRT
   <213> Homo sapiens
<400> 364
   Gly Pro Pro Gly Pro Pro
   1 5
<210> 365
   <211> 6
   <212> PRT
   <213> Homo sapiens
<400> 365
   Gly Ala Ala Gly Glu Pro
   1 5
<210> 366
   <211> 6
   <212> PRT
   <213> Homo sapiens
<400> 366
   Gly Leu Ser Gly Glu Arg
   1 5
<210> 367
   <211> 6
   <212> PRT
   <213> Homo sapiens
<400> 367
   Ile Ala Gly Ile Thr Gly
   1 5
<210> 368
   <211> 6
   <212> PRT
   <213> Homo sapiens
<400> 368
   Pro Gly Phe Pro Gly Met
   1 5
<210> 369
   <211> 6
   <212> PRT
   <213> Homo sapiens
<400> 369
   Gly Ile Ala Gly Ile Thr
   1 5
<210> 370
   <211> 6
   <212> PRT
   <213> Homo sapiens
<400> 370
   Gly Ala Arg Gly Leu Ala
   1 5
<210> 371
   <211> 6
   <212> PRT
   <213> Homo sapiens
<400> 371
   Gly Leu Ala Gly Pro Pro
   1 5
<210> 372
   <211> 6
   <212> PRT
   <213> Homo sapiens
<400> 372
   Gly Pro Pro Gly Val Ala
   1 5
<210> 373
   <211> 6
   <212> PRT
   <213> Homo sapiens
<400> 373
   Gly Pro Ala Gly Ile Pro
   1 5
<210> 374
   <211> 6
   <212> PRT
   <213> Homo sapiens
<400> 374
   Pro Gly Pro Pro Gly Ile
   1 5
<210> 375
   <211> 6
   <212> PRT
   <213> Homo sapiens
<400> 375
   Gly Ala Pro Gly Pro Gln
   1 5
<210> 376
   <211> 6
   <212> PRT
   <213> Homo sapiens
<400> 376
   Gly Asp Ala Gly Gln Pro
   1 5
<210> 377
   <211> 6
   <212> PRT
   <213> Homo sapiens
<400> 377
   His Ala Gly Ala Gln Gly
   1 5
<210> 378
   <211> 6
   <212> PRT
   <213> Homo sapiens
<400> 378
   Gly Pro Pro Gly Ala Pro
   1 5
<210> 379
   <211> 6
   <212> PRT
   <213> Homo sapiens
<400> 379
   Gly Leu Ala Gly Pro Pro
   1 5
<210> 380
   <211> 6
   <212> PRT
   <213> Homo sapiens
<400> 380
   Ala Ile Gly Pro Ser Gly
   1 5
<210> 381
   <211> 6
   <212> PRT
   <213> Homo sapiens
<400> 381
   Gly Pro Pro Gly Pro Ala
   1 5
<210> 382
   <211> 6
   <212> PRT
   <213> Homo sapiens
<400> 382
   Ser Pro Gly Pro Ala Gly
   1 5
<210> 383
   <211> 6
   <212> PRT
   <213> Homo sapiens
<400> 383
   Ala Ala Gly Thr Pro Gly
   1 5
<210> 384
   <211> 6
   <212> PRT
   <213> Homo sapiens
<400> 384
   Thr Ser Gly His Pro Gly
   1 5
<210> 385
   <211> 6
   <212> PRT
   <213> Homo sapiens
<400> 385
   Pro Ser Gly Pro Pro Gly
   1 5
<210> 386
   <211> 6
   <212> PRT
   <213> Homo sapiens
<400> 386
   Pro Gly Leu Met Gly Ala
   1 5
<210> 387
   <211> 6
   <212> PRT
   <213> Homo sapiens
<400> 387
   Ser Pro Gly Pro Gln Gly
   1 5
<210> 388
   <211> 6
   <212> PRT
   <213> Homo sapiens
<400> 388
   Pro Pro Gly Pro Pro Gly
   1 5
<210> 389
   <211> 6
   <212> PRT
   <213> Homo sapiens
<400> 389
   Gly Ala Ile Gly Pro Ser
   1 5
<210> 390
   <211> 6
   <212> PRT
   <213> Homo sapiens
<400> 390
   Pro Pro Gly Pro Ala Gly
   1 5
<210> 391
   <211> 6
   <212> PRT
   <213> Homo sapiens
<400> 391
   Ser Pro Gly Pro Lys Gly
   1 5
<210> 392
   <211> 6
   <212> PRT
   <213> Homo sapiens
<400> 392
   Pro Pro Gly Pro Ala Gly
   1 5
<210> 393
   <211> 6
   <212> PRT
   <213> Homo sapiens
<400> 393
   Leu Pro Gly Pro Pro Gly
   1 5
<210> 394
   <211> 6
   <212> PRT
   <213> Homo sapiens
<400> 394
   Gly Ala Pro Gly Leu Lys
   1 5
<210> 395
   <211> 6
   <212> PRT
   <213> Homo sapiens
<400> 395
   Gly Leu Pro Gly Pro Pro
   1 5
<210> 396
   <211> 6
   <212> PRT
   <213> Homo sapiens
<400> 396
   Gly Pro Pro Gly Ile Lys
   1 5
<210> 397
   <211> 6
   <212> PRT
   <213> Homo sapiens
<400> 397
   Gly Pro Pro Gly Val Ala
   1 5
<210> 398
   <211> 6
   <212> PRT
   <213> Homo sapiens
<400> 398
   Thr Pro Gly Leu Gln Gly
   1 5
<210> 399
   <211> 6
   <212> PRT
   <213> Homo sapiens
<400> 399
   Gly Thr Pro Gly Leu Gln
   1 5
<210> 400
   <211> 6
   <212> PRT
   <213> Homo sapiens
<400> 400
   Gly Phe Pro Gly Ala Arg
   1 5
<210> 401
   <211> 6
   <212> PRT
   <213> Homo sapiens
<400> 401
   Gln Pro Gly Pro Pro Gly
   1 5
<210> 402
   <211> 6
   <212> PRT
   <213> Homo sapiens
<400> 402
   Thr Gly Ala Pro Gly Ser
   1 5
<210> 403
   <211> 6
   <212> PRT
   <213> Homo sapiens
<400> 403
   Ser Pro Gly Pro Gln Gly
   1 5
<210> 404
   <211> 6
   <212> PRT
   <213> Homo sapiens
<400> 404
   Gly Pro Ala Gly Pro Arg
   1 5
<210> 405
   <211> 6
   <212> PRT
   <213> Homo sapiens
<400> 405
   Ala Pro Gly Leu Met Gly
   1 5
<210> 406
   <211> 6
   <212> PRT
   <213> Homo sapiens
<400> 406
   Gly Asn Arg Gly Glu Arg
   1 5
<210> 407
   <211> 6
   <212> PRT
   <213> Homo sapiens
<400> 407
   Thr Gly Ala Pro Gly Ser
   1               5
<210> 408
   <211> 6
   <212> PRT
   <213> Homo sapiens
<400> 408
   Gly Asp Pro Gly Pro Pro
   1               5
<210> 409
   <211> 6
   <212> PRT
   <213> Homo sapiens
<400> 409
   Arg Gly Leu Ala Gly Pro
   1               5
<210> 410
   <211> 6
   <212> PRT
   <213> Homo sapiens
<400> 410
   Ser Arg Gly Ala Pro Gly
   1               5
<210> 411
   <211> 6
   <212> PRT
   <213> Homo sapiens
<400> 411
   Gly Lys Ser Gly Asp Arg
   1               5
<210> 412
   <211> 6
   <212> PRT
   <213> Homo sapiens
<400> 412
   Val Gly Gly Leu Ala Gly
   1               5
<210> 413
   <211> 6
   <212> PRT
   <213> Homo sapiens
<400> 413
   Ala Ile Gly Ser Pro Gly
   1 5
<210> 414
   <211> 6
   <212> PRT
   <213> Homo sapiens
<400> 414
   Gly Leu Ala Gly Pro Pro
   1 5
<210> 415
   <211> 6
   <212> PRT
   <213> Homo sapiens
<400> 415
   Arg Gly Leu Ala Gly Pro
   1 5
<210> 416
   <211> 6
   <212> PRT
   <213> Homo sapiens
<400> 416
   Lys Gly Glu Ser Gly Lys
   1 5
<210> 417
   <211> 6
   <212> PRT
   <213> Homo sapiens
<400> 417
   Ile Ala Gly Ile Thr Gly
   1 5
<210> 418
   <211> 6
   <212> PRT
   <213> Homo sapiens
<400> 418
   Pro Gly Ala Pro Gly Leu
   1 5
<210> 419
   <211> 6
   <212> PRT
   <213> Homo sapiens
<400> 419
   Glu Arg Gly Leu Pro Gly
   1 5
<210> 420
   <211> 6
   <212> PRT
   <213> Homo sapiens
<400> 420
   Ile Asn Gly Ser Pro Gly
   1 5
<210> 421
   <211> 6
   <212> PRT
   <213> Homo sapiens
<400> 421
   Leu Pro Gly Ile Ala Gly
   1 5
<210> 422
   <211> 6
   <212> PRT
   <213> Homo sapiens
<400> 422
   Asn Thr Gly Ala Pro Gly
   1 5
<210> 423
   <211> 6
   <212> PRT
   <213> Homo sapiens
<400> 423
   Leu Arg Gly Gly Ala Gly
   1 5
<210> 424
   <211> 6
   <212> PRT
   <213> Homo sapiens
<400> 424
   Gly His Ala Gly Ala Gln
   1 5
<210> 425
   <211> 6
   <212> PRT
   <213> Homo sapiens
<400> 425
   Gly Phe Pro Gly Ala Arg
   1 5
<210> 426
   <211> 6
   <212> PRT
   <213> Homo sapiens
<400> 426
   Gly Arg Asn Gly Glu Lys
   1 5
<210> 427
   <211> 6
   <212> PRT
   <213> Homo sapiens
<400> 427
   Gly Glu Arg Gly Ser Pro
   1 5
<210> 428
   <211> 6
   <212> PRT
   <213> Homo sapiens
<400> 428
   Gly Lys Asp Gly Glu Ser
   1 5
<210> 429
   <211> 6
   <212> PRT
   <213> Homo sapiens
<400> 429
   Ile Ala Gly Ile Thr Gly
   1 5
<210> 430
   <211> 6
   <212> PRT
   <213> Homo sapiens
<400> 430
   Gly Pro Lys Gly Asp Ala
   1               5
<210> 431
   <211> 6
   <212> PRT
   <213> Homo sapiens
<400> 431
   Gly Ser Pro Gly Gly Pro
   1               5
<210> 432
   <211> 6
   <212> PRT
   <213> Homo sapiens
<400> 432
   Gly Leu Pro Gly Pro Pro
   1               5
<210> 433
   <211> 6
   <212> PRT
   <213> Homo sapiens
<400> 433
   Ser Gly Asp Arg Gly Glu
   1               5
<210> 434
   <211> 6
   <212> PRT
   <213> Homo sapiens
<400> 434
   Gly Leu Ala Gly Pro Pro
   1               5
<210> 435
   <211> 6
   <212> PRT
   <213> Homo sapiens
<400> 435
   Gly Ala Pro Gly Phe Arg
   1 5
<210> 436
   <211> 6
   <212> PRT
   <213> Homo sapiens
<400> 436
   Gly Phe Pro Gly Asn Pro
   1 5
<210> 437
   <211> 6
   <212> PRT
   <213> Homo sapiens
<400> 437
   Asn Gly Glu Lys Gly Glu
   1 5
<210> 438
   <211> 6
   <212> PRT
   <213> Homo sapiens
<400> 438
   Gly Leu Ala Gly Pro Pro
   1 5
<210> 439
   <211> 6
   <212> PRT
   <213> Homo sapiens
<400> 439
   Gly Ile Thr Gly Ala Arg
   1 5
<210> 440
   <211> 6
   <212> PRT
   <213> Homo sapiens
<400> 440
   Asp Gly Thr Ser Gly His
   1 5
<210> 441
   <211> 6
   <212> PRT
   <213> Homo sapiens
<400> 441
   Gly Pro Pro Gly Ser Asn
   1               5
<210> 442
   <211> 6
   <212> PRT
   <213> Homo sapiens
<400> 442
   Asn Gly Asp Pro Gly Ile
   1               5
<210> 443
   <211> 6
   <212> PRT
   <213> Homo sapiens
<400> 443
   Pro Gly Pro Gln Gly Val
   1               5
<210> 444
   <211> 6
   <212> PRT
   <213> Homo sapiens
<400> 444
   Ile Pro Gly Ala Pro Gly
   1               5
<210> 445
   <211> 6
   <212> PRT
   <213> Homo sapiens
<400> 445
   Ser Pro Gly Pro Gln Gly
   1               5
<210> 446
   <211> 6
   <212> PRT
   <213> Homo sapiens
<400> 446
   Gly Ser Pro Gly Pro Ala
   1               5
<210> 447
   <211> 6
   <212> PRT
   <213> Homo sapiens
<400> 447
   Gln Gly Pro Pro Gly Pro
   1               5
<210> 448
   <211> 6
   <212> PRT
   <213> Homo sapiens
<400> 448
   Ile Pro Gly Phe Pro Gly
   1               5
<210> 449
   <211> 6
   <212> PRT
   <213> Homo sapiens
<400> 449
   Gly Pro Ala Gly Ile Pro
   1               5
<210> 450
   <211> 6
   <212> PRT
   <213> Homo sapiens
<400> 450
   Phe Pro Gly Ala Arg Gly
   1               5
<210> 451
   <211> 6
   <212> PRT
   <213> Homo sapiens
<400> 451
   Gly Ile Pro Gly Ala Pro
   1               5
<210> 452
   <211> 6
   <212> PRT
   <213> Homo sapiens
<400> 452
   Glu Arg Gly Pro Pro Gly
   1               5
<210> 453
   <211> 6
   <212> PRT
   <213> Homo sapiens
<400> 453
   Pro Ser Gly Pro Pro Gly
   1 5
<210> 454
   <211> 6
   <212> PRT
   <213> Homo sapiens
<400> 454
   Gly Pro Pro Gly Ile Asn
   1 5
<210> 455
   <211> 6
   <212> PRT
   <213> Homo sapiens
<400> 455
   Gly Thr Ser Gly His Pro
   1 5
<210> 456
   <211> 6
   <212> PRT
   <213> Homo sapiens
<400> 456
   Gly Leu Pro Gly Ile Ala
   1 5
<210> 457
   <211> 6
   <212> PRT
   <213> Homo sapiens
<400> 457
   Gly Ala Asn Gly Leu Pro
   1 5
<210> 458
   <211> 6
   <212> PRT
   <213> Homo sapiens
<400> 458
   Thr Ala Gly Phe Pro Gly
   1 5
<210> 459
   <211> 6
   <212> PRT
   <213> Homo sapiens
<400> 459
   Glu Gly Gly Pro Pro Gly
   1               5
<210> 460
   <211> 6
   <212> PRT
   <213> Homo sapiens
<400> 460
   Pro Gln Gly Leu Pro Gly
   1               5
<210> 461
   <211> 6
   <212> PRT
   <213> Homo sapiens
<400> 461
   Ala Pro Gly Pro Leu Gly
   1               5
<210> 462
   <211> 6
   <212> PRT
   <213> Homo sapiens
<400> 462
   Gly Ser Pro Gly Pro Gln
   1               5
<210> 463
   <211> 6
   <212> PRT
   <213> Homo sapiens
<400> 463
   Gly Ala Ala Gly Ala Arg
   1               5
<210> 464
   <211> 6
   <212> PRT
   <213> Homo sapiens
<400> 464
   Gly Val Lys Gly Glu Arg
   1 5
<210> 465
   <211> 6
   <212> PRT
   <213> Homo sapiens
<400> 465
   Gly Asp Ala Gly Ala Pro
   1 5
<210> 466
   <211> 6
   <212> PRT
   <213> Homo sapiens
<400> 466
   Arg Gly Phe Asp Gly Arg
   1 5
<210> 467
   <211> 6
   <212> PRT
   <213> Homo sapiens
<400> 467
   Gly Leu Ser Gly Glu Arg
   1 5
<210> 468
   <211> 6
   <212> PRT
   <213> Homo sapiens
<400> 468
   Gly Ser Pro Gly Pro Gln
   1 5
<210> 469
   <211> 6
   <212> PRT
   <213> Homo sapiens
<400> 469
   Gly Val Lys Gly Glu Arg
   1 5
<210> 470
   <211> 6
   <212> PRT
   <213> Homo sapiens
<400> 470
   Leu Ala Gly Pro Pro Gly
   1 5
<210> 471
   <211> 6
   <212> PRT
   <213> Homo sapiens
<400> 471
   Thr Gly Ala Arg Gly Leu
   1 5
<210> 472
   <211> 6
   <212> PRT
   <213> Homo sapiens
<400> 472
   Lys Gly Asp Ala Gly Gln
   1 5
<210> 473
   <211> 6
   <212> PRT
   <213> Homo sapiens
<400> 473
   Leu Gln Gly Leu Pro Gly
   1 5
<210> 474
   <211> 6
   <212> PRT
   <213> Homo sapiens
<400> 474
   Ile Ala Gly Ile Thr Gly
   1 5
<210> 475
   <211> 6
   <212> PRT
   <213> Homo sapiens
<400> 475
   Ala Gly Gln Gln Gly Ala
   1 5
<210> 476
   <211> 6
   <212> PRT
   <213> Homo sapiens
<400> 476
   Leu Ala Gly Pro Pro Gly
   1 5
<210> 477
   <211> 6
   <212> PRT
   <213> Homo sapiens
<400> 477
   Gly Leu Ser Gly Glu Arg
   1 5
<210> 478
   <211> 6
   <212> PRT
   <213> Homo sapiens
<400> 478
   Val Lys Gly Glu Arg Gly
   1 5
<210> 479
   <211> 6
   <212> PRT
   <213> Homo sapiens
<400> 479
   Ile Lys Gly Pro Ala Gly
   1 5
<210> 480
   <211> 6
   <212> PRT
   <213> Homo sapiens
<400> 480
   Glu Arg Gly Leu Pro Gly
   1 5
<210> 481
   <211> 6
   <212> PRT
   <213> Homo sapiens
<400> 481
   Val Gly Gly Leu Ala Gly
   1 5
<210> 482
   <211> 6
   <212> PRT
   <213> Homo sapiens
<400> 482
   Gly Pro Pro Gly Glu Pro
   1               5
<210> 483
   <211> 6
   <212> PRT
   <213> Homo sapiens
<400> 483
   Ile Asn Gly Ser Pro Gly
   1               5
<210> 484
   <211> 6
   <212> PRT
   <213> Homo sapiens
<400> 484
   Leu Met Gly Ala Arg Gly
   1               5
<210> 485
   <211> 6
   <212> PRT
   <213> Homo sapiens
<400> 485
   Lys Asn Gly Glu Thr Gly
   1               5
<210> 486
   <211> 6
   <212> PRT
   <213> Homo sapiens
<400> 486
   Gly Glu Arg Gly Ala Pro
   1               5
<210> 487
   <211> 6
   <212> PRT
   <213> Homo sapiens
<400> 487
   Pro Gly Pro Pro Gly Pro
   1               5
<210> 488
   <211> 6
   <212> PRT
   <213> Homo sapiens
<400> 488
   Gly Gln Pro Gly Asp Lys
   1 5
<210> 489
   <211> 6
   <212> PRT
   <213> Homo sapiens
<400> 489
   Pro Gly Val Pro Gly Ala
   1 5
<210> 490
   <211> 6
   <212> PRT
   <213> Homo sapiens
<400> 490
   Gly Ala Arg Gly Asn Asp
   1 5
<210> 491
   <211> 6
   <212> PRT
   <213> Homo sapiens
<400> 491
   Pro Gly Ala Pro Gly Gln
   1 5
<210> 492
   <211> 6
   <212> PRT
   <213> Homo sapiens
<400> 492
   Gly Pro Pro Gly Pro Pro
   1 5
<210> 493
   <211> 6
   <212> PRT
   <213> Homo sapiens
<400> 493
   Ile Ala Gly Ile Thr Gly
   1 5
<210> 494
   <211> 6
   <212> PRT
   <213> Homo sapiens
<400> 494
   Gly Pro Ala Gly Ile Pro
   1 5
<210> 495
   <211> 6
   <212> PRT
   <213> Homo sapiens
<400> 495
   Gly Pro Pro Gly Pro Pro
   1 5
<210> 496
   <211> 6
   <212> PRT
   <213> Homo sapiens
<400> 496
   Arg Pro Gly Leu Pro Gly
   1 5
<210> 497
   <211> 6
   <212> PRT
   <213> Homo sapiens
<400> 497
   Gly Pro Pro Gly Ser Asn
   1 5
<210> 498
   <211> 6
   <212> PRT
   <213> Homo sapiens
<400> 498
   Pro Gly Phe Pro Gly Met
   1 5
<210> 499
   <211> 6
   <212> PRT
   <213> Homo sapiens
<400> 499
   Gly Pro Pro Gly Glu Asn
   1 5
<210> 500
   <211> 6
   <212> PRT
   <213> Homo sapiens
<400> 500
   Gly Leu Pro Gly Pro Pro
   1 5
<210> 501
   <211> 6
   <212> PRT
   <213> Homo sapiens
<400> 501
   Pro Gly Pro Pro Gly Ile
   1 5
<210> 502
   <211> 6
   <212> PRT
   <213> Homo sapiens
<400> 502
   Pro Gly Phe Arg Gly Pro
   1 5
<210> 503
   <211> 6
   <212> PRT
   <213> Homo sapiens
<400> 503
   Gly Ile Ala Gly Ile Thr
   1 5
<210> 504
   <211> 6
   <212> PRT
   <213> Homo sapiens
<400> 504
   Gly Ile Pro Gly Ala Pro
   1 5
<210> 505
   <211> 6
   <212> PRT
   <213> Homo sapiens
<400> 505
   Gly Ile Thr Gly Ala Arg
   1 5
<210> 506
   <211> 6
   <212> PRT
   <213> Homo sapiens
<400> 506
   Gly Ala Pro Gly Glu Lys
   1 5
<210> 507
   <211> 6
   <212> PRT
   <213> Homo sapiens
<400> 507
   Ser Pro Gly Pro Gln Gly
   1 5
<210> 508
   <211> 6
   <212> PRT
   <213> Homo sapiens
<400> 508
   Pro Pro Gly Pro Gln Gly
   1 5
<210> 509
   <211> 6
   <212> PRT
   <213> Homo sapiens
<400> 509
   Glu Arg Gly Pro Pro Gly
   1 5
<210> 510
   <211> 6
   <212> PRT
   <213> Homo sapiens
<400> 510
   Ala Pro Gly Leu Arg Gly
   1 5
<210> 511
   <211> 6
   <212> PRT
   <213> Homo sapiens
<400> 511
   Gly Pro Ala Gly Pro Pro
   1               5
<210> 512
   <211> 6
   <212> PRT
   <213> Homo sapiens
<400> 512
   Ser Pro Gly Pro Gln Gly
   1               5
<210> 513
   <211> 6
   <212> PRT
   <213> Homo sapiens
<400> 513
   Leu Ala Gly Pro Pro Gly
   1               5
<210> 514
   <211> 6
   <212> PRT
   <213> Homo sapiens
<400> 514
   Phe Pro Gly Pro Lys Gly
   1               5
<210> 515
   <211> 6
   <212> PRT
   <213> Homo sapiens
<400> 515
   Pro Pro Gly Pro Ala Gly
   1               5
<210> 516
   <211> 6
   <212> PRT
   <213> Homo sapiens
<400> 516
   Leu Pro Gly Ala Ala Gly
   1 5
<210> 517
   <211> 6
   <212> PRT
   <213> Homo sapiens
<400> 517
   Ile Pro Gly Gln Pro Gly
   1 5
<210> 518
   <211> 6
   <212> PRT
   <213> Homo sapiens
<400> 518
   Gly Ala Pro Gly Pro Ala
   1 5
<210> 519
   <211> 6
   <212> PRT
   <213> Homo sapiens
<400> 519
   Gly His Arg Gly Phe Asp
   1 5
<210> 520
   <211> 6
   <212> PRT
   <213> Homo sapiens
<400> 520
   Pro Gly Pro Lys Gly Asp
   1 5
<210> 521
   <211> 6
   <212> PRT
   <213> Homo sapiens
<400> 521
   Gly Pro Pro Gly Pro Ser
   1 5
<210> 522
   <211> 6
   <212> PRT
   <213> Homo sapiens
<400> 522
   Pro Pro Gly Ala Pro Gly
   1 5
<210> 523
   <211> 6
   <212> PRT
   <213> Homo sapiens
<400> 523
   Gly Glu Val Gly Pro Ala
   1 5
<210> 524
   <211> 6
   <212> PRT
   <213> Homo sapiens
<400> 524
   Gly Ala Pro Gly Leu Arg
   1 5
<210> 525
   <211> 6
   <212> PRT
   <213> Homo sapiens
<400> 525
   Gly Pro Gln Gly Val Lys
   1 5
<210> 526
   <211> 6
   <212> PRT
   <213> Homo sapiens
<400> 526
   Gly Met Pro Gly Pro Arg
   1 5
<210> 527
   <211> 6
   <212> PRT
   <213> Homo sapiens
<400> 527
   Gly Ala Ala Gly Ile Lys
   1 5
<210> 528
   <211> 6
   <212> PRT
   <213> Homo sapiens
<400> 528
   Pro Gly Ala Ala Gly Phe
   1 5
<210> 529
   <211> 6
   <212> PRT
   <213> Homo sapiens
<400> 529
   Gly Pro Ala Gly Glu Arg
   1 5
<210> 530
   <211> 6
   <212> PRT
   <213> Homo sapiens
<400> 530
   Gly Ile Ala Gly Pro Arg
   1 5
<210> 531
   <211> 6
   <212> PRT
   <213> Homo sapiens
<400> 531
   Thr Gly Ala Arg Gly Leu
   1 5
<210> 532
   <211> 6
   <212> PRT
   <213> Homo sapiens
<400> 532
   Val Lys Gly Glu Ser Gly
   1 5
<210> 533
   <211> 6
   <212> PRT
   <213> Homo sapiens
<400> 533
   Thr Gly Glu Arg Gly Ala
   1 5
<210> 534
   <211> 6
   <212> PRT
   <213> Homo sapiens
<400> 534
   Glu Arg Gly Leu Pro Gly
   1               5
<210> 535
   <211> 6
   <212> PRT
   <213> Homo sapiens
<400> 535
   Ile Thr Gly Ala Arg Gly
   1               5
<210> 536
   <211> 6
   <212> PRT
   <213> Homo sapiens
<400> 536
   Glu Arg Gly Leu Pro Gly
   1               5
<210> 537
   <211> 6
   <212> PRT
   <213> Homo sapiens
<400> 537
   Pro Gly Ala Pro Gly Gly
   1               5
<210> 538
   <211> 6
   <212> PRT
   <213> Homo sapiens
<400> 538
   Gly Pro Pro Gly Val Ala
   1               5
<210> 539
   <211> 6
   <212> PRT
   <213> Homo sapiens
<400> 539
   Gly Leu Ala Gly Pro Pro
   1               5
<210> 540
   <211> 6
   <212> PRT
   <213> Homo sapiens
<400> 540
   Val Gly Gly Leu Ala Gly
   1               5
<210> 541
   <211> 6
   <212> PRT
   <213> Homo sapiens
<400> 541
   Ser Pro Gly Ala Gln Gly
   1               5
<210> 542
   <211> 6
   <212> PRT
   <213> Homo sapiens
<400> 542
   Gln Pro Gly Val Met Gly
   1               5
<210> 543
   <211> 6
   <212> PRT
   <213> Homo sapiens
<400> 543
   Ile Pro Gly Ala Pro Gly
   1               5
<210> 544
   <211> 6
   <212> PRT
   <213> Homo sapiens
<400> 544
   Val Lys Gly Glu Arg Gly
   1               5
<210> 545
   <211> 6
   <212> PRT
   <213> Homo sapiens
<400> 545
   Gly Pro Pro Gly Ile Asn
   1 5
<210> 546
   <211> 6
   <212> PRT
   <213> Homo sapiens
<400> 546
   Gly Ile Asn Gly Ser Pro
   1 5
<210> 547
   <211> 6
   <212> PRT
   <213> Homo sapiens
<400> 547
   Leu Arg Gly Gly Ala Gly
   1 5
<210> 548
   <211> 6
   <212> PRT
   <213> Homo sapiens
<400> 548
   Tyr Gln Gly Pro Pro Gly
   1 5
<210> 549
   <211> 6
   <212> PRT
   <213> Homo sapiens
<400> 549
   Gly Ile Pro Gly Phe Pro
   1 5
<210> 550
   <211> 6
   <212> PRT
   <213> Homo sapiens
<400> 550
   Phe Pro Gly Met Lys Gly
   1 5
<210> 551
   <211> 6
   <212> PRT
   <213> Homo sapiens
<400> 551
   Gly Pro Pro Gly Glu Asn
   1 5
<210> 552
   <211> 6
   <212> PRT
   <213> Homo sapiens
<400> 552
   Ala Ala Gly Phe Pro Gly
   1 5
<210> 553
   <211> 6
   <212> PRT
   <213> Homo sapiens
<400> 553
   Pro Gly Val Ser Gly Pro
   1 5
<210> 554
   <211> 6
   <212> PRT
   <213> Homo sapiens
<400> 554
   Gly Phe Pro Gly Ala Pro
   1 5
<210> 555
   <211> 6
   <212> PRT
   <213> Homo sapiens
<400> 555
   Gly Leu Ser Gly Glu Arg
   1 5
<210> 556
   <211> 6
   <212> PRT
   <213> Homo sapiens
<400> 556
   Gly Gly Ala Gly Pro Pro
   1 5
<210> 557
   <211> 6
   <212> PRT
   <213> Homo sapiens
<400> 557
   Val Gly Gly Leu Ala Gly
   1 5
<210> 558
   <211> 6
   <212> PRT
   <213> Homo sapiens
<400> 558
   Gly Leu Ala Gly Pro Pro
   1 5
<210> 559
   <211> 6
   <212> PRT
   <213> Homo sapiens
<400> 559
   Pro Gly Pro Pro Gly Ile
   1 5
<210> 560
   <211> 6
   <212> PRT
   <213> Homo sapiens
<400> 560
   Gly Ala Pro Gly Pro Met
   1 5
<210> 561
   <211> 6
   <212> PRT
   <213> Homo sapiens
<400> 561
   Gly Ala Arg Gly Leu Ala
   1 5
<210> 562
   <211> 6
   <212> PRT
   <213> Homo sapiens
<400> 562
   Gly Leu Ala Gly Pro Pro
   1 5
<210> 563
   <211> 6
   <212> PRT
   <213> Homo sapiens
<400> 563
   Pro Gly Phe Arg Gly Pro
   1 5
<210> 564
   <211> 6
   <212> PRT
   <213> Homo sapiens
<400> 564
   Gly Pro Val Gly Pro Ser
   1 5
<210> 565
   <211> 6
   <212> PRT
   <213> Homo sapiens
<400> 565
   Ala Gly Gln Pro Gly Glu
   1 5
<210> 566
   <211> 6
   <212> PRT
   <213> Homo sapiens
<400> 566
   Gly Ala Pro Gly Phe Arg
   1 5
<210> 567
   <211> 6
   <212> PRT
   <213> Homo sapiens
<400> 567
   Pro Gly Phe Pro Gly Met
   1 5
<210> 568
   <211> 6
   <212> PRT
   <213> Homo sapiens
<400> 568
   Gly Leu Ala Gly Pro Pro
   1 5
<210> 569
   <211> 6
   <212> PRT
   <213> Homo sapiens
<400> 569
   Ser Gly Asp Arg Gly Glu
   1 5
<210> 570
   <211> 6
   <212> PRT
   <213> Homo sapiens
<400> 570
   Ala Gly Ile Pro Gly Phe
   1 5
<210> 571
   <211> 6
   <212> PRT
   <213> Homo sapiens
<400> 571
   Glu Arg Gly Ala Ala Gly
   1 5
<210> 572
   <211> 6
   <212> PRT
   <213> Homo sapiens
<400> 572
   Pro Gly Pro Pro Gly Thr
   1 5
<210> 573
   <211> 6
   <212> PRT
   <213> Homo sapiens
<400> 573
   His Pro Gly Ser Pro Gly
   1 5
<210> 574
   <211> 6
   <212> PRT
   <213> Homo sapiens
<400> 574
   Ser Pro Gly Pro Gln Gly
   1 5
<210> 575
   <211> 6
   <212> PRT
   <213> Homo sapiens
<400> 575
   Gly Leu Ala Gly Thr Ala
   1 5
<210> 576
   <211> 6
   <212> PRT
   <213> Homo sapiens
<400> 576
   Gly Pro Pro Gly Pro Gln
   1 5
<210> 577
   <211> 6
   <212> PRT
   <213> Homo sapiens
<400> 577
   Gly Phe Pro Gly Met Lys
   1 5
<210> 578
   <211> 6
   <212> PRT
   <213> Homo sapiens
<400> 578
   Glu Lys Gly Pro Ala Gly
   1 5
<210> 579
   <211> 6
   <212> PRT
   <213> Homo sapiens
<400> 579
   Pro Gly Pro Gln Gly Leu
   1 5
<210> 580
   <211> 6
   <212> PRT
   <213> Homo sapiens
<400> 580
   Phe Pro Gly Ala Pro Gly
   1               5
<210> 581
   <211> 6
   <212> PRT
   <213> Homo sapiens
<400> 581
   Ala Pro Gly Pro Leu Gly
   1               5
<210> 582
   <211> 6
   <212> PRT
   <213> Homo sapiens
<400> 582
   Gly Phe Pro Gly Met Lys
   1               5
<210> 583
   <211> 6
   <212> PRT
   <213> Homo sapiens
<400> 583
   Pro Gly Leu Pro Gly Ile
   1               5
<210> 584
   <211> 6
   <212> PRT
   <213> Homo sapiens
<400> 584
   Gly His Arg Gly Phe Asp
   1               5
<210> 585
   <211> 6
   <212> PRT
   <213> Homo sapiens
<400> 585
   Pro Gly Pro Lys Gly Asn
   1               5
<210> 586
   <211> 6
   <212> PRT
   <213> Homo sapiens
<400> 586
   Gly Glu Val Gly Pro Ala
   1               5
<210> 587
   <211> 6
   <212> PRT
   <213> Homo sapiens
<400> 587
   Gly Pro Pro Gly Pro Ser
   1               5
<210> 588
   <211> 6
   <212> PRT
   <213> Homo sapiens
<400> 588
   Gly Leu Pro Gly Leu Ala
   1               5
<210> 589
   <211> 6
   <212> PRT
   <213> Homo sapiens
<400> 589
   Gly Ser Pro Gly Tyr Gln
   1               5
<210> 590
   <211> 6
   <212> PRT
   <213> Homo sapiens
<400> 590
   Glu Met Gly Pro Ala Gly
   1               5
<210> 591
   <211> 6
   <212> PRT
   <213> Homo sapiens
<400> 591
   Gly Lys Pro Gly Ala Asn
   1 5
<210> 592
   <211> 6
   <212> PRT
   <213> Homo sapiens
<400> 592
   Gly Met Pro Gly Pro Arg
   1 5
<210> 593
   <211> 6
   <212> PRT
   <213> Homo sapiens
<400> 593
   Gly Ile Thr Gly Ala Arg
   1 5
<210> 594
   <211> 6
   <212> PRT
   <213> Homo sapiens
<400> 594
   Ala Gly Pro Arg Gly Ala
   1 5
<210> 595
   <211> 6
   <212> PRT
   <213> Homo sapiens
<400> 595
   Gly Pro Ala Gly Ala Asn
   1 5
<210> 596
   <211> 6
   <212> PRT
   <213> Homo sapiens
<400> 596
   Gly Lys Pro Gly Ala Asn
   1 5
<210> 597
   <211> 6
   <212> PRT
   <213> Homo sapiens
<400> 597
   Gly Phe Pro Gly Ala Arg
   1               5
<210> 598
   <211> 6
   <212> PRT
   <213> Homo sapiens
<400> 598
   Ile Ala Gly Ile Thr Gly
   1               5
<210> 599
   <211> 6
   <212> PRT
   <213> Homo sapiens
<400> 599
   Ile Thr Gly Ala Arg Gly
   1               5
<210> 600
   <211> 6
   <212> PRT
   <213> Homo sapiens
<400> 600
   Leu Arg Gly Gly Ala Gly
   1               5
<210> 601
   <211> 6
   <212> PRT
   <213> Homo sapiens
<400> 601
   Val Gly Gly Leu Ala Gly
   1               5
<210> 602
   <211> 6
   <212> PRT
   <213> Homo sapiens
<400> 602
   Ala Gly Pro Pro Gly Met
   1               5
<210> 603
   <211> 6
   <212> PRT
   <213> Homo sapiens
<400> 603
   Pro Gln Gly Pro Pro Gly
   1               5
<210> 604
   <211> 6
   <212> PRT
   <213> Homo sapiens
<400> 604
   Ser Pro Gly Gly Lys Gly
   1               5
<210> 605
   <211> 6
   <212> PRT
   <213> Homo sapiens
<400> 605
   Gly Ala Pro Gly Glu Lys
   1               5
<210> 606
   <211> 6
   <212> PRT
   <213> Homo sapiens
<400> 606
   Ile Lys Gly Pro Ala Gly
   1               5
<210> 607
   <211> 6
   <212> PRT
   <213> Homo sapiens
<400> 607
   Phe Arg Gly Pro Ala Gly
   1               5
<210> 608
   <211> 6
   <212> PRT
   <213> Homo sapiens
<400> 608
   Asp Ala Gly Ala Pro Gly
   1               5
<210> 609
   <211> 6
   <212> PRT
   <213> Homo sapiens
<400> 609
   Gly Leu Pro Gly Pro Pro
   1 5
<210> 610
   <211> 6
   <212> PRT
   <213> Homo sapiens
<400> 610
   Pro Gly Glu Asn Gly Lys
   1 5
<210> 611
   <211> 6
   <212> PRT
   <213> Homo sapiens
<400> 611
   Gln Gln Gly Ala Ile Gly
   1 5
<210> 612
   <211> 6
   <212> PRT
   <213> Homo sapiens
<400> 612
   Val Ala Gly Pro Pro Gly
   1 5
<210> 613
   <211> 6
   <212> PRT
   <213> Homo sapiens
<400> 613
   Pro Gly Met Lys Gly His
   1 5
<210> 614
   <211> 6
   <212> PRT
   <213> Homo sapiens
<400> 614
   Pro Gly Asp Lys Gly Glu
   1 5
<210> 615
   <211> 6
   <212> PRT
   <213> Homo sapiens
<400> 615
   Gly Ser Asp Gly Gln Pro
   1 5
<210> 616
   <211> 6
   <212> PRT
   <213> Homo sapiens
<400> 616
   Gly Ala Arg Gly Asn Asp
   1 5
<210> 617
   <211> 6
   <212> PRT
   <213> Homo sapiens
<400> 617
   Gly Ala Arg Gly Pro Pro
   1 5
<210> 618
   <211> 6
   <212> PRT
   <213> Homo sapiens
<400> 618
   Gly Pro Lys Gly Asp Ala
   1 5
<210> 619
   <211> 6
   <212> PRT
   <213> Homo sapiens
<400> 619
   Pro Gly Pro Gln Gly His
   1 5
<210> 620
   <211> 6
   <212> PRT
   <213> Homo sapiens
<400> 620
   Gly Ile Thr Gly Ala Arg
   1 5
<210> 621
   <211> 6
   <212> PRT
   <213> Homo sapiens
<400> 621
   Gly Ser Arg Gly Ala Pro
   1 5
<210> 622
   <211> 6
   <212> PRT
   <213> Homo sapiens
<400> 622
   Gly Ala Pro Gly Glu Lys
   1 5
<210> 623
   <211> 6
   <212> PRT
   <213> Homo sapiens
<400> 623
   Pro Gln Gly Leu Gln Gly
   1 5
<210> 624
   <211> 6
   <212> PRT
   <213> Homo sapiens
<400> 624
   Lys Gly Ser Pro Gly Ala
   1 5
<210> 625
   <211> 6
   <212> PRT
   <213> Homo sapiens
<400> 625
   Pro Gly Pro Gln Gly Pro
   1 5
<210> 626
   <211> 6
   <212> PRT
   <213> Homo sapiens
<400> 626
   Gly Pro Thr Gly Pro Ile
   1 5
<210> 627
   <211> 6
   <212> PRT
   <213> Homo sapiens
<400> 627
   Gly Ser Pro Gly Glu Arg
   1 5
<210> 628
   <211> 6
   <212> PRT
   <213> Homo sapiens
<400> 628
   Gly Ser Pro Gly Ala Gln
   1 5
<210> 629
   <211> 6
   <212> PRT
   <213> Homo sapiens
<400> 629
   Gly Leu Ala Gly Pro Pro
   1 5
<210> 630
   <211> 6
   <212> PRT
   <213> Homo sapiens
<400> 630
   Gly Ala Pro Gly Glu Lys
   1 5
<210> 631
   <211> 6
   <212> PRT
   <213> Homo sapiens
<400> 631
   Gly Met Pro Gly Pro Arg
   1 5
<210> 632
   <211> 6
   <212> PRT
   <213> Homo sapiens
<400> 632
   Pro Gly Pro Leu Gly Ile
   1 5
<210> 633
   <211> 6
   <212> PRT
   <213> Homo sapiens
<400> 633
   Gly Asn Arg Gly Glu Arg
   1 5
<210> 634
   <211> 6
   <212> PRT
   <213> Homo sapiens
<400> 634
   Pro Ser Gly Pro Pro Gly
   1 5
<210> 635
   <211> 6
   <212> PRT
   <213> Homo sapiens
<400> 635
   Gly Leu Pro Gly Leu Ala
   1 5
<210> 636
   <211> 6
   <212> PRT
   <213> Homo sapiens
<400> 636
   Pro Gly Pro Pro Gly Thr
   1 5
<210> 637
   <211> 6
   <212> PRT
   <213> Homo sapiens
<400> 637
   Gly Pro Pro Gly Pro Gln
   1 5
<210> 638
   <211> 6
   <212> PRT
   <213> Homo sapiens
<400> 638
   Phe Pro Gly Met Lys Gly
   1               5
<210> 639
   <211> 6
   <212> PRT
   <213> Homo sapiens
<400> 639
   Gly Pro Pro Gly Ile Cys
   1               5
<210> 640
   <211> 6
   <212> PRT
   <213> Homo sapiens
<400> 640
   Gly Pro Pro Gly Ile Cys
   1               5
<210> 641
   <211> 6
   <212> PRT
   <213> Homo sapiens
<400> 641
   Gly Ala Pro Gly Leu Met
   1               5
<210> 642
   <211> 6
   <212> PRT
   <213> Homo sapiens
<400> 642
   Glu Pro Gly Pro Arg Gly
   1               5
<210> 643
   <211> 6
   <212> PRT
   <213> Homo sapiens
<400> 643
   Gly His Arg Gly Phe Asp
   1 5
<210> 644
   <211> 6
   <212> PRT
   <213> Homo sapiens
<400> 644
   Gly Ala Ala Gly Ile Lys
   1 5
<210> 645
   <211> 6
   <212> PRT
   <213> Homo sapiens
<400> 645
   Gly Glu Pro Gly Pro Arg
   1 5
<210> 646
   <211> 6
   <212> PRT
   <213> Homo sapiens
<400> 646
   Gly Ile Pro Gly Ala Pro
   1 5
<210> 647
   <211> 6
   <212> PRT
   <213> Homo sapiens
<400> 647
   Gly Ala Pro Gly Leu Met
   1 5
<210> 648
   <211> 6
   <212> PRT
   <213> Homo sapiens
<400> 648
   Thr Gly Ala Arg Gly Leu
   1 5
<210> 649
   <211> 6
   <212> PRT
   <213> Homo sapiens
<400> 649
   Met Pro Gly Pro Arg Gly
   1 5
<210> 650
   <211> 6
   <212> PRT
   <213> Homo sapiens
<400> 650
   Gly Met Lys Gly His Arg
   1 5
<210> 651
   <211> 6
   <212> PRT
   <213> Homo sapiens
<400> 651
   Gly Pro Gln Gly Val Lys
   1 5
<210> 652
   <211> 6
   <212> PRT
   <213> Homo sapiens
<400> 652
   Pro Gly Ala Asn Gly Leu
   1 5
<210> 653
   <211> 6
   <212> PRT
   <213> Homo sapiens
<400> 653
   Gly Thr Gly Gly Pro Pro
   1 5
<210> 654
   <211> 6
   <212> PRT
   <213> Homo sapiens
<400> 654
   Gly Pro Pro Gly Pro Arg
   1 5
<210> 655
   <211> 6
   <212> PRT
   <213> Homo sapiens
<400> 655
   Gly Gly Lys Gly Glu Arg
   1 5
<210> 656
   <211> 6
   <212> PRT
   <213> Homo sapiens
<400> 656
   Pro Gln Gly Val Lys Gly
   1 5
<210> 657
   <211> 6
   <212> PRT
   <213> Homo sapiens
<400> 657
   Pro Gly Ala Asn Gly Leu
   1 5
<210> 658
   <211> 161
   <212> PRT
   <213> Homo sapiens
<400> 658
   Met Glu Lys Leu Leu Cys Phe Leu Val Leu Thr Ser Leu Ser His Ala
   1               5                   10                  15
   Phe Gly Gln Thr Asp Met Ser Arg Lys Ala Phe Val Phe Pro Lys Glu
               20                  25                  30
   Ser Asp Thr Ser Tyr Val Ser Leu Lys Ala Pro Leu Thr Lys Pro Leu
           35                  40                  45
   Lys Ala Phe Thr Val Cys Leu His Phe Tyr Thr Glu Leu Ser Ser Thr
       50                  55                  60
   Arg Gly Thr Val Phe Ser Arg Met Pro Pro Arg Asp Lys Thr Met Arg
   65                  70                  75                  80
   Phe Phe Ile Phe Trp Ser Lys Asp Ile Gly Tyr Ser Phe Thr Val Gly
                   85                  90                  95
   Gly Ser Glu Ile Leu Phe Glu Val Pro Glu Val Thr Val Ala Pro Val
               100                 105                 110
   His Ile Cys Thr Ser Trp Glu Ser Ala Ser Gly Ile Val Glu Phe Trp
           115                 120                 125
   Val Asp Gly Lys Pro Arg Val Arg Lys Ser Leu Lys Lys Gly Tyr Thr
       130                 135                 140
   Val Gly Ala Glu Ala Ser Ile Ile Leu Gly Gln Glu Gln Asp Ser Phe
   145                 150                 155                 160
   Gly
<210> 659
   <211> 317
   <212> PRT
   <213> Homo sapiens
<400> 659
<210> 660
   <211> 17
   <212> PRT
   <213> Homo sapiens
<400> 660
   Ala Lys Val Glu Gln Ala Val Glu Thr Glu Pro Glu Pro Glu Leu Arg
   1 5 10 15
   Gln
<210> 661
   <211> 17
   <212> PRT
   <213> Homo sapiens
<400> 661
   Ala Lys Val Glu Gln Ala Val Glu Thr Glu Pro Glu Pro Glu Leu Arg
   1 5 10 15
   Gln
<210> 662
   <211> 16
   <212> PRT
   <213> Homo sapiens
<400> 662
   Val Ala Glu Val Arg Ala Lys Leu Glu Glu Gln Ala Gln Gln Ile Arg
   1 5 10 15
<210> 663
   <211> 17
   <212> PRT
   <213> Homo sapiens
<400> 663
   Ala Lys Val Glu Gln Ala Val Glu Thr Glu Pro Glu Pro Glu Leu Arg
   1 5 10 15
   Gln
<210> 664
   <211> 13
   <212> PRT
   <213> Homo sapiens
<400> 664
   Ala Met Leu Gly Gln Ser Thr Glu Glu Leu Arg Val Arg
   1 5 10
<210> 665
   <211> 14
   <212> PRT
   <213> Homo sapiens
<400> 665
   Glu Gln Ala Val Glu Thr Glu Pro Glu Pro Glu Leu Arg Gln
   1 5 10
<210> 666
   <211> 14
   <212> PRT
   <213> Homo sapiens
<400> 666
   Arg Gln Gln Thr Glu Trp Gln Ser Gly Gln Arg Trp Glu Leu
   1 5 10
<210> 667
   <211> 18
   <212> PRT
   <213> Homo sapiens
<400> 667
   Leu Ala Val Tyr Gln Ala Gly Ala Arg Glu Gly Ala Glu Arg Gly Leu
   1               5                   10                  15
   Ser Ala
<210> 668
   <211> 13
   <212> PRT
   <213> Homo sapiens
<400> 668
   Arg Ala Lys Leu Glu Glu Gln Ala Gln Gln Ile Arg Leu
   1               5                   10
<210> 669
   <211> 14
   <212> PRT
   <213> Homo sapiens
<400> 669
   Ala Lys Leu Glu Glu Gln Ala Gln Gln Ile Arg Leu Gln Ala
   1               5                   10
<210> 670
   <211> 17
   <212> PRT
   <213> Homo sapiens
<400> 670
   Ala Lys Val Glu Gln Ala Val Glu Thr Glu Pro Glu Pro Glu Leu Arg
   1               5                   10                  15
   Gln
<210> 671
   <211> 16
   <212> PRT
   <213> Homo sapiens
<400> 671
   Lys Val Glu Gln Ala Val Glu Thr Glu Pro Glu Pro Glu Leu Arg Gln
   1               5                   10                  15
<210> 672
   <211> 14
   <212> PRT
   <213> Homo sapiens
<400> 672
   Glu Gln Ala Val Glu Thr Glu Pro Glu Pro Glu Leu Arg Gln
   1               5                   10
<210> 673
   <211> 16
   <212> PRT
   <213> Homo sapiens
<400> 673
   Asp Glu Val Lys Glu Gln Val Ala Glu Val Arg Ala Lys Leu Glu Glu
   1               5                   10                  15
<210> 674
   <211> 16
   <212> PRT
   <213> Homo sapiens
<400> 674
   Lys Glu Ser Asp Thr Ser Tyr Val Ser Leu Lys Ala Pro Leu Thr Lys
   1               5                   10                  15
<210> 675
   <211> 16
   <212> PRT
   <213> Homo sapiens
<400> 675
   Gly Gly Asn Phe Glu Gly Ser Gln Ser Leu Val Gly Asp Ile Gly Asn
   1               5                   10                  15
<210> 676
   <211> 16
   <212> PRT
   <213> Homo sapiens
<400> 676
   Ala Leu Lys Tyr Glu Val Gln Gly Glu Val Phe Thr Lys Pro Gln Leu
   1               5                   10                  15
<210> 677
   <211> 14
   <212> PRT
   <213> Homo sapiens
<400> 677
   Gly Ile Val Glu Phe Trp Val Asp Gly Lys Pro Arg Val Arg
   1               5                   10
<210> 678
   <211> 10
   <212> PRT
   <213> Homo sapiens
<400> 678
   Arg Lys Ala Phe Val Phe Pro Lys Glu Ser
   1               5                   10
<210> 679
   <211> 16
   <212> PRT
   <213> Homo sapiens
<400> 679
   Lys Tyr Glu Val Gln Gly Glu Val Phe Thr Lys Pro Gln Leu Trp Pro
   1               5                   10                  15
<210> 680
   <211> 13
   <212> PRT
   <213> Homo sapiens
<400> 680
   Asp Ser Phe Gly Gly Asn Phe Glu Gly Ser Gln Ser Leu
   1               5                   10
<210> 681
   <211> 12
   <212> PRT
   <213> Homo sapiens
<400> 681
   Asp Phe Val Leu Ser Pro Asp Glu Ile Asn Thr Ile
   1               5                   10
<210> 682
   <211> 13
   <212> PRT
   <213> Homo sapiens
<400> 682
   Ser Leu Lys Lys Gly Tyr Thr Val Gly Ala Glu Ala Ser
   1               5                   10
<210> 683
   <211> 12
   <212> PRT
   <213> Homo sapiens
<400> 683
   Ala Phe Gly Gln Thr Asp Met Ser Arg Lys Ala Phe
   1               5                   10
<210> 684
   <211> 14
   <212> PRT
   <213> Homo sapiens
<400> 684
   Ser Leu Lys Lys Gly Tyr Thr Val Gly Ala Glu Ala Ser Ile
   1               5                   10
<210> 685
   <211> 11
   <212> PRT
   <213> Homo sapiens
<400> 685
   Gly Glu Val Phe Thr Lys Pro Gln Leu Trp Pro
   1               5                   10
<210> 686
   <211> 15
   <212> PRT
   <213> Homo sapiens
<400> 686
   Ser Ile Ile Leu Gly Gln Glu Gln Asp Ser Phe Gly Gly Asn Phe
   1               5                   10                  15
<210> 687
   <211> 14
   <212> PRT
   <213> Homo sapiens
<400> 687
   Lys Tyr Glu Val Gln Gly Glu Val Phe Thr Lys Pro Gln Leu
   1               5                   10
<210> 688
   <211> 6
   <212> PRT
   <213> Homo sapiens
<400> 688
   Lys Val Glu Gln Ala Val
   1               5
<210> 689
   <211> 6
   <212> PRT
   <213> Homo sapiens
<400> 689
   Ala Glu Val Arg Ala Lys
   1               5
<210> 690
   <211> 6
   <212> PRT
   <213> Homo sapiens
<400> 690
   Met Leu Gly Gln Ser Thr
   1 5
<210> 691
   <211> 6
   <212> PRT
   <213> Homo sapiens
<400> 691
   Gln Ala Val Glu Thr Glu
   1 5
<210> 692
   <211> 6
   <212> PRT
   <213> Homo sapiens
<400> 692
   Gln Gln Thr Glu Trp Gln
   1 5
<210> 693
   <211> 6
   <212> PRT
   <213> Homo sapiens
<400> 693
   Ala Val Tyr Gln Ala Gly
   1 5
<210> 694
   <211> 6
   <212> PRT
   <213> Homo sapiens
<400> 694
   Ala Lys Leu Glu Glu Gln
   1 5
<210> 695
   <211> 6
   <212> PRT
   <213> Homo sapiens
<400> 695
   Lys Leu Glu Glu Gln Ala
   1 5
<210> 696
   <211> 6
   <212> PRT
   <213> Homo sapiens
<400> 696
   Val Glu Gln Ala Val Glu
   1 5
<210> 697
   <211> 6
   <212> PRT
   <213> Homo sapiens
<400> 697
   Gln Ala Val Glu Thr Glu
   1 5
<210> 698
   <211> 6
   <212> PRT
   <213> Homo sapiens
<400> 698
   Glu Val Lys Glu Gln Val
   1 5
<210> 699
   <211> 6
   <212> PRT
   <213> Homo sapiens
<400> 699
   Ala Phe Val Phe Pro Lys
   1 5
<210> 700
   <211> 6
   <212> PRT
   <213> Homo sapiens
<400> 700
   Tyr Glu Val Gln Gly Glu
   1 5
<210> 701
   <211> 6
   <212> PRT
   <213> Homo sapiens
<400> 701
   Lys Ala Phe Val Phe Pro
   1 5
<210> 702
   <211> 6
   <212> PRT
   <213> Homo sapiens
<400> 702
   Ser Phe Gly Gly Asn Phe
   1 5
<210> 703
   <211> 6
   <212> PRT
   <213> Homo sapiens
<400> 703
   Phe Val Leu Ser Pro Asp
   1 5
<210> 704
   <211> 6
   <212> PRT
   <213> Homo sapiens
<400> 704
   Leu Lys Lys Gly Tyr Thr
   1 5
<210> 705
   <211> 6
   <212> PRT
   <213> Homo sapiens
<400> 705
   Phe Gly Gln Thr Asp Met
   1 5
<210> 706
   <211> 6
   <212> PRT
   <213> Homo sapiens
<400> 706
   Leu Lys Lys Gly Tyr Thr
   1 5
<210> 707
   <211> 6
   <212> PRT
   <213> Homo sapiens
<400> 707
   Ile Ile Leu Gly Gln Glu
   1 5
<210> 708
   <211> 6
   <212> PRT
   <213> Homo sapiens
<400> 708
   Tyr Glu Val Gln Gly Glu
   1 5
<210> 709
   <211> 6
   <212> PRT
   <213> Homo sapiens
<400> 709
   Leu Lys Tyr Glu Val Gln
   1 5
<210> 710
   <211> 6
   <212> PRT
   <213> Homo sapiens
<400> 710
   Ile Val Glu Phe Trp Val
   1 5
<210> 711
   <211> 6
   <212> PRT
   <213> Homo sapiens
<400> 711
   Glu Ser Asp Thr Ser Tyr
   1 5
<210> 712
   <211> 6
   <212> PRT
   <213> Homo sapiens
<400> 712
   Gly Asn Phe Glu Gly Ser
   1 5
<210> 713
   <211> 6
   <212> PRT
   <213> Homo sapiens
<400> 713
   Thr Glu Pro Glu Pro Glu
   1 5
<210> 714
   <211> 6
   <212> PRT
   <213> Homo sapiens
<400> 714
   Thr Glu Pro Glu Pro Glu
   1 5
<210> 715
   <211> 6
   <212> PRT
   <213> Homo sapiens
<400> 715
   Glu Gln Ala Gln Gln Ile
   1 5
<210> 716
   <211> 6
   <212> PRT
   <213> Homo sapiens
<400> 716
   Thr Glu Glu Leu Arg Val
   1 5
<210> 717
   <211> 6
   <212> PRT
   <213> Homo sapiens
<400> 717
   Pro Glu Pro Glu Leu Arg
   1 5
<210> 718
   <211> 6
   <212> PRT
   <213> Homo sapiens
<400> 718
   Ser Gly Gln Arg Trp Glu
   1 5
<210> 719
   <211> 6
   <212> PRT
   <213> Homo sapiens
<400> 719
   Glu Gly Ala Glu Arg Gly
   1 5
<210> 720
   <211> 6
   <212> PRT
   <213> Homo sapiens
<400> 720
   Gln Ala Gln Gln Ile Arg
   1 5
<210> 721
   <211> 6
   <212> PRT
   <213> Homo sapiens
<400> 721
   Gln Gln Ile Arg Leu Gln
   1 5
<210> 722
   <211> 6
   <212> PRT
   <213> Homo sapiens
<400> 722
   Glu Pro Glu Pro Glu Leu
   1 5
<210> 723
   <211> 6
   <212> PRT
   <213> Homo sapiens
<400> 723
   Pro Glu Pro Glu Leu Arg
   1 5
<210> 724
   <211> 6
   <212> PRT
   <213> Homo sapiens
<400> 724
   Glu Val Arg Ala Lys Leu
   1               5
<210> 725
   <211> 7
   <212> PRT
   <213> Homo sapiens
<400> 725
   Lys Ala Phe Val Phe Pro Lys
   1 5
<210> 726
   <211> 6
   <212> PRT
   <213> Homo sapiens
<400> 726
   Ala Phe Val Phe Pro Lys
   1 5
<210> 727
   <211> 6
   <212> PRT
   <213> Homo sapiens
<400> 727
   Lys Pro Gln Leu Trp Pro
   1 5
<210> 728
   <211> 6
   <212> PRT
   <213> Homo sapiens
<400> 728
   Phe Val Phe Pro Lys Glu
   1 5
<210> 729
   <211> 6
   <212> PRT
   <213> Homo sapiens
<400> 729
   Pro Asp Glu Ile Asn Thr
   1 5
<210> 730
   <211> 6
   <212> PRT
   <213> Homo sapiens
<400> 730
   Asp Met Ser Arg Lys Ala
   1 5
<210> 731
   <211> 6
   <212> PRT
   <213> Homo sapiens
<400> 731
   Val Gly Ala Glu Ala Ser
   1 5
<210> 732
   <211> 6
   <212> PRT
   <213> Homo sapiens
<400> 732
   Lys Pro Gln Leu Trp Pro
   1 5
<210> 733
   <211> 6
   <212> PRT
   <213> Homo sapiens
<400> 733
   Asp Ser Phe Gly Gly Asn
   1 5
<210> 734
   <211> 6
   <212> PRT
   <213> Homo sapiens
<400> 734
   Val Phe Thr Lys Pro Gln
   1 5
<210> 735
   <211> 757
   <212> PRT
   <213> Homo sapiens
<400> 735
   Met Ala Gly Leu Thr Ala Ala Ala Pro Arg Pro Gly Val Leu Leu Leu
   1               5                   10                  15
   Leu Leu Ser Ile Leu His Pro Ser Arg Pro Gly Gly Val Pro Gly Ala
               20                  25                  30
   Ile Pro Gly Gly Val Pro Gly Gly Val Phe Tyr Pro Gly Ala Gly Leu
           35                  40                  45
   Gly Ala Leu Gly Gly Gly Ala Leu Gly Pro Gly Gly Lys Pro Leu Lys
       50                  55                  60
   Pro Val Pro Gly Gly Leu Ala Gly Ala Gly Leu Gly Ala Gly Leu Gly
   65                  70                  75                  80
   Ala Phe Pro Ala Val Thr Phe Pro Gly Ala Leu Val Pro Gly Gly Val
                   85                  90                  95
   Ala Asp Ala Ala Ala Ala Tyr Lys Ala Ala Lys Ala Gly Ala Gly Leu
               100                 105                 110
   Gly Gly Val Pro Gly Val Gly Gly Leu Gly Val Ser Ala Gly Ala Val
           115                 120                 125
   Val Pro Gln Pro Gly Ala Gly Val Lys Pro Gly Lys Val Pro Gly Val
       130                 135                 140
   Gly Leu Pro Gly Val Tyr Pro Gly Gly Val Leu Pro Gly Ala Arg Phe
   145                 150                 155                 160
   Pro Gly Val Gly Val Leu Pro Gly Val Pro Thr Gly Ala Gly Val Lys
                   165                 170                 175
   Pro Lys Ala Pro Gly Val Gly Gly Ala Phe Ala Gly Ile Pro Gly Val
               180                 185                 190
   Gly Pro Phe Gly Gly Pro Gln Pro Gly Val Pro Leu Gly Tyr Pro Ile
           195                 200                 205
   Lys Ala Pro Lys Leu Pro Gly Gly Tyr Gly Leu Pro Tyr Thr Thr Gly
       210                 215                 220
   Lys Leu Pro Tyr Gly Tyr Gly Pro Gly Gly Val Ala Gly Ala Ala Gly
   225                 230                 235                 240
   Lys Ala Gly Tyr Pro Thr Gly Thr Gly Val Gly Pro Gln Ala Ala Ala
                   245                 250                 255
   Ala Ala Ala Ala Lys Ala Ala Ala Lys Phe Gly Ala Gly Ala Ala Gly
               260                 265                 270
   Val Leu Pro Gly Val Gly Gly Ala Gly Val Pro Gly Val Pro Gly Ala
           275                 280                 285
   Ile Pro Gly Ile Gly Gly Ile Ala Gly Val Gly Thr Pro Ala Ala Ala
       290                 295                 300
   Ala Ala Ala Ala Ala Ala Ala Lys Ala Ala Lys Tyr Gly Ala Ala Ala
   305                 310                 315                 320
   Gly Leu Val Pro Gly Gly Pro Gly Phe Gly Pro Gly Val Val Gly Val
                   325                 330                 335
   Pro Gly Ala Gly Val Pro Gly Val Gly Val Pro Gly Ala Gly Ile Pro
               340                 345                 350
   Val Val Pro Gly Ala Gly Ile Pro Gly Ala Ala Val Pro Gly Val Val
           355                 360                 365
   Ser Pro Glu Ala Ala Ala Lys Ala Ala Ala Lys Ala Ala Lys Tyr Gly
       370                 375                 380
   Ala Arg Pro Gly Val Gly Val Gly Gly Ile Pro Thr Tyr Gly Val Gly
   385                 390                 395                 400
   Ala Gly Gly Phe Pro Gly Phe Gly Val Gly Val Gly Gly Ile Pro Gly
                   405                 410                 415
   Val Ala Gly Val Pro Ser Val Gly Gly Val Pro Gly Val Gly Gly Val
               420                 425                 430
   Pro Gly Val Gly Ile Ser Pro Glu Ala Gln Ala Ala Ala Ala Ala Lys
           435                 440                 445
   Ala Ala Lys Tyr Gly Val Gly Thr Pro Ala Ala Ala Ala Ala Lys Ala
       450                 455                 460
   Ala Ala Lys Ala Ala Gln Phe Gly Leu Val Pro Gly Val Gly Val Ala
   465                 470                 475                 480
   Pro Gly Val Gly Val Ala Pro Gly Val Gly Val Ala Pro Gly Val Gly
                   485                 490                 495
   Leu Ala Pro Gly Val Gly Val Ala Pro Gly Val Gly Val Ala Pro Gly
               500                 505                 510
   Val Gly Val Ala Pro Gly Ile Gly Pro Gly Gly Val Ala Ala Ala Ala
           515                 520                 525
   Lys Ser Ala Ala Lys Val Ala Ala Lys Ala Gln Leu Arg Ala Ala Ala
       530                 535                 540
   Gly Leu Gly Ala Gly Ile Pro Gly Leu Gly Val Gly Val Gly Val Pro
   545                 550                 555                 560
   Gly Leu Gly Val Gly Ala Gly Val Pro Gly Leu Gly Val Gly Ala Gly
                   565                 570                 575
   Val Pro Gly Phe Gly Ala Gly Ala Asp Glu Gly Val Arg Arg Ser Leu
               580                 585                 590
   Ser Pro Glu Leu Arg Glu Gly Asp Pro Ser Ser Ser Gln His Leu Pro
   595 600 605
   Ser Thr Pro Ser Ser Pro Arg Val Pro Gly Ala Leu Ala Ala Ala Lys
   610 615 620
   Ala Ala Lys Tyr Gly Ala Ala Val Pro Gly Val Leu Gly Gly Leu Gly
   625 630 635 640
   Ala Leu Gly Gly Val Gly Ile Pro Gly Gly Val Val Gly Ala Gly Pro
   645 650 655
   Ala Ala Ala Ala Ala Ala Ala Lys Ala Ala Ala Lys Ala Ala Gln Phe
   660 665 670
   Gly Leu Val Gly Ala Ala Gly Leu Gly Gly Leu Gly Val Gly Gly Leu
   675 680 685
   Gly Val Pro Gly Val Gly Gly Leu Gly Gly Ile Pro Pro Ala Ala Ala
   690 695 700
   Ala Lys Ala Ala Lys Tyr Gly Ala Ala Gly Leu Gly Gly Val Leu Gly
   705 710 715 720
   Gly Ala Gly Gln Phe Pro Leu Gly Gly Val Ala Ala Arg Pro Gly Phe
   725 730 735
   Gly Leu Ser Pro Ile Phe Pro Gly Gly Ala Cys Leu Gly Lys Ala Cys
   740 745 750
   Gly Arg Lys Arg Lys
   755
<210> 736
   <211> 20
   <212> PRT
   <213> Homo sapiens
<400> 736
   Ala Arg Pro Gly Val Gly Val Gly Gly Ile Pro Thr Tyr Gly Val Gly
   1 5 10 15
   Ala Gly Gly Phe
   20
<210> 737
   <211> 17
   <212> PRT
   <213> Homo sapiens
<400> 737
   Gly Leu Pro Tyr Thr Thr Gly Lys Leu Pro Tyr Gly Tyr Gly Pro Gly
   1               5                   10                  15
   Gly
<210> 738
   <211> 25
   <212> PRT
   <213> Homo sapiens
<400> 738
   Gly Val Ala Pro Gly Val Gly Val Ala Pro Gly Val Gly Val Ala Pro
   1               5                   10                  15
   Gly Ile Gly Pro Gly Gly Val Ala Ala
               20                  25
<210> 739
   <211> 23
   <212> PRT
   <213> Homo sapiens
<400> 739
   Gly Gly Ala Gly Val Pro Gly Val Pro Gly Ala Ile Pro Gly Ile Gly
   1               5                   10                  15
   Gly Ile Ala Gly Val Gly Thr
               20
<210> 740
   <211> 23
   <212> PRT
   <213> Homo sapiens
<400> 740
   Gly Gly Ala Gly Val Pro Gly Val Pro Gly Ala Ile Pro Gly Ile Gly
   1               5                   10                  15
   Gly Ile Ala Gly Val Gly Thr
               20
<210> 741
   <211> 16
   <212> PRT
   <213> Homo sapiens
<400> 741
   Gly Val Gly Ile Ser Pro Glu Ala Gln Ala Ala Ala Ala Ala Lys Ala
   1 5 10 15
<210> 742
   <211> 16
   <212> PRT
   <213> Homo sapiens
<400> 742
   Gly Val Gly Ile Ser Pro Glu Ala Gln Ala Ala Ala Ala Ala Lys Ala
   1 5 10 15
<210> 743
   <211> 6
   <212> PRT
   <213> Homo sapiens
<400> 743
   Arg Pro Gly Val Gly Val
   1 5
<210> 744
   <211> 6
   <212> PRT
   <213> Homo sapiens
<400> 744
   Leu Pro Tyr Thr Thr Gly
   1 5
<210> 745
   <211> 6
   <212> PRT
   <213> Homo sapiens
<400> 745
   Val Ala Pro Gly Val Gly
   1 5
<210> 746
   <211> 6
   <212> PRT
   <213> Homo sapiens
<400> 746
   Gly Ala Gly Val Pro Gly
   1 5
<210> 747
   <211> 6
   <212> PRT
   <213> Homo sapiens
<400> 747
   Val Gly Ile Ser Pro Glu
   1 5
<210> 748
   <211> 6
   <212> PRT
   <213> Homo sapiens
<400> 748
   Arg Pro Gly Val Gly Val
   1 5
<210> 749
   <211> 6
   <212> PRT
   <213> Homo sapiens
<400> 749
   Leu Pro Tyr Thr Thr Gly
   1 5
<210> 750
   <211> 6
   <212> PRT
   <213> Homo sapiens
<400> 750
   Gly Val Gly Ala Gly Gly
   1 5
<210> 751
   <211> 6
   <212> PRT
   <213> Homo sapiens
<400> 751
   Tyr Gly Tyr Gly Pro Gly
   1 5
<210> 752
   <211> 6
   <212> PRT
   <213> Homo sapiens
<400> 752
   Gly Pro Gly Gly Val Ala
   1 5
<210> 753
   <211> 6
   <212> PRT
   <213> Homo sapiens
<400> 753
   Gly Ile Ala Gly Val Gly
   1               5
<210> 754
   <211> 13
   <212> PRT
   <213> Homo sapiens
<400> 754
   Lys Asn Gly Glu Thr Gly Pro Gln Gly Pro Gly Gly Cys
   1               5                   10
<210> 755
   <211> 13
   <212> PRT
   <213> Homo sapiens
<400> 755
   Lys Asn Gly Glu Thr Gly Pro Gln Gly Pro Pro Gly Lys
   1               5                   10
<210> 756
   <211> 13
   <212> PRT
   <213> Homo sapiens
<400> 756
   Lys Asp Gly Glu Thr Gly Ala Ala Gly Pro Pro Gly Lys
   1               5                   10
<210> 757
   <211> 13
   <212> PRT
   <213> Homo sapiens
<400> 757
   Lys Asp Gly Glu Ala Gly Ala Gln Gly Pro Pro Gly Lys
   1               5                   10
<210> 758
   <211> 14
   <212> PRT
   <213> Homo sapiens
<400> 758
   Pro Gly Lys Asn Gly Glu Thr Pro Gly Pro Gln Gly Pro Lys
   1               5                   10
<210> 759
   <211> 13
   <212> PRT
   <213> Homo sapiens
<400> 759
   Ile Ala Gly Ile Thr Gly Ala Arg Gly Leu Gly Gly Cys
   1 5 10
<210> 760
   <211> 13
   <212> PRT
   <213> Homo sapiens
<400> 760
   Ile Ala Gly Ile Thr Gly Ala Arg Gly Leu Ala Gly Lys
   1 5 10
<210> 761
   <211> 13
   <212> PRT
   <213> Homo sapiens
<400> 761
   Ile Ala Gly Leu Thr Gly Ala Arg Gly Leu Ala Gly Lys
   1 5 10
<210> 762
   <211> 15
   <212> PRT
   <213> Homo sapiens
<400> 762
   Leu Gly Ile Ala Gly Ile Thr Gly Ala Arg Gly Leu Ala Gly Lys
   1 5 10 15
<210> 763
   <211> 6
   <212> PRT
   <213> Homo sapiens
<400> 763
   Lys Asp Gly Thr Ser Gly
   1 5
<210> 764
   <211> 13
   <212> PRT
   <213> Homo sapiens
<400> 764
   Lys Asp Gly Thr Ser Gly His Pro Gly Pro Gly Gly Cys
   1 5 10
<210> 765
   <211> 13
   <212> PRT
   <213> Homo sapiens
<400> 765
   Lys Asp Gly Thr Ser Gly His Pro Gly Pro Ile Gly Lys
   1 5 10
<210> 766
   <211> 13
   <212> PRT
   <213> Homo sapiens
<400> 766
   Lys Asp Gly Ser Ser Gly His Pro Gly Pro Ile Gly Lys
   1 5 10
<210> 767
   <211> 13
   <212> PRT
   <213> Homo sapiens
<400> 767
   Pro Gly Lys Asp Gly Thr Ser Gly His Pro Gly Pro Lys
   1 5 10
<210> 768
   <211> 13
   <212> PRT
   <213> Homo sapiens
<400> 768
   Cys Gly Gly Gly Pro Pro Gly Ala Pro Gly Pro Leu Gly
   1 5 10
<210> 769
   <211> 12
   <212> PRT
   <213> Homo sapiens
<400> 769
   Ala Gln Gly Pro Pro Gly Ala Pro Gly Pro Leu Gly
   1 5 10
<210> 770
   <211> 12
   <212> PRT
   <213> Homo sapiens
<400> 770
   Ala Gln Gly Pro Pro Gly Ser Pro Gly Pro Leu Gly
   1 5 10
<210> 771
   <211> 12
   <212> PRT
   <213> Homo sapiens
<400> 771
   Asp Asp Gly Pro Ser Gly Ala Glu Gly Pro Pro Gly
   1 5 10
<210> 772
   <211> 12
   <212> PRT
   <213> Homo sapiens
<400> 772
   Gly Pro Pro Gly Ala Pro Gly Pro Leu Gly Ile Ala
   1 5 10
<210> 773
   <211> 13
   <212> PRT
   <213> Homo sapiens
<400> 773
   Asn Thr Gly Ala Pro Gly Ser Pro Gly Val Cys Gly Gly
   1 5 10
<210> 774
   <211> 13
   <212> PRT
   <213> Homo sapiens
<400> 774
   Asn Thr Gly Ala Pro Gly Ser Pro Gly Val Ser Gly Lys
   1 5 10
<210> 775
   <211> 13
   <212> PRT
   <213> Homo sapiens
<400> 775
   Asn Ser Gly Ser Pro Gly Asn Pro Gly Val Ala Gly Lys
   1 5 10
<210> 776
   <211> 12
   <212> PRT
   <213> Homo sapiens
<400> 776
   Ala Gly Asn Thr Gly Ala Pro Gly Ser Pro Gly Val
   1 5 10
<210> 777
   <211> 13
   <212> PRT
   <213> Homo sapiens
<400> 777
   Ala Ile Gly Pro Ser Gly Pro Ala Gly Lys Gly Gly Cys
   1 5 10
<210> 778
   <211> 13
   <212> PRT
   <213> Homo sapiens
<400> 778
   Ala Ile Gly Pro Ser Gly Pro Ala Gly Lys Asp Gly Lys
   1 5 10
<210> 779
   <211> 13
   <212> PRT
   <213> Homo sapiens
<400> 779
   Ala Ile Gly Pro Ala Gly Pro Ala Gly Lys Asp Gly Lys
   1 5 10
<210> 780
   <211> 13
   <212> PRT
   <213> Homo sapiens
<400> 780
   Pro Gly Ala Ile Gly Pro Ser Gly Pro Ala Gly Lys Asp
   1 5 10
<210> 781
   <211> 6
   <212> PRT
   <213> Homo sapiens
<400> 781
   Ala Gly Gly Phe Ala Pro
   1 5
<210> 782
   <211> 11
   <212> PRT
   <213> Homo sapiens
<400> 782
   Cys Gly Gly Glu Lys Ala Gly Gly Phe Ala Pro
   1               5                   10
<210> 783
   <211> 10
   <212> PRT
   <213> Homo sapiens
<400> 783
   Cys Gly Glu Lys Ala Gly Gly Phe Ala Pro
   1               5                   10
<210> 784
   <211> 10
   <212> PRT
   <213> Homo sapiens
<400> 784
   Cys Gly Glu Lys Ser Gly Gly Phe Ser Pro
   1               5                   10
<210> 785
   <211> 12
   <212> PRT
   <213> Homo sapiens
<400> 785
   Gly Gly Glu Lys Ala Gly Gly Phe Ala Pro Tyr Tyr
   1               5                   10
<210> 786
   <211> 6
   <212> PRT
   <213> Homo sapiens
<400> 786
   Gln Asn Gly Asn Ile Lys
   1               5
<210> 787
   <211> 13
   <212> PRT
   <213> Homo sapiens
<400> 787
   Ile Val Ile Glu Leu Gly Thr Asn Pro Leu Gly Gly Cys
   1               5                   10
<210> 788
   <211> 13
   <212> PRT
   <213> Homo sapiens
<400> 788
   Ile Val Ile Glu Leu Gly Thr Asn Pro Leu Lys Ser Lys
   1               5                   10
<210> 789
   <211> 13
   <212> PRT
   <213> Homo sapiens
<400> 789
   Leu Val Ile Glu Leu Gly Gly Asn Pro Leu Lys Asn Lys
   1               5                   10
<210> 790
   <211> 13
   <212> PRT
   <213> Homo sapiens
<400> 790
   Ile Val Val Glu Leu Gly Gly Asn Pro Leu Thr Asn Lys
   1               5                   10
<210> 791
   <211> 14
   <212> PRT
   <213> Homo sapiens
<400> 791
   Gln Met Ile Val Ile Glu Leu Gly Thr Asn Pro Leu Lys Lys
   1               5                   10
<210> 792
   <211> 13
   <212> PRT
   <213> Homo sapiens
<400> 792
   Asn Val Leu Val Ile Glu Leu Gly Gly Asn Pro Leu Lys
   1               5                   10
<210> 793
   <211> 13
   <212> PRT
   <213> Homo sapiens
<400> 793
   Cys Gly Gly Leu Asp Leu Gln Asn Asn Asp Ile Ser Glu
   1 5 10
<210> 794
   <211> 12
   <212> PRT
   <213> Homo sapiens
<400> 794
   Thr Leu Leu Asp Leu Gln Asn Asn Asp Ile Ser Glu
   1 5 10
<210> 795
   <211> 12
   <212> PRT
   <213> Homo sapiens
<400> 795
   Leu Asp Leu Gln Asn Asn Asp Ile Ser Glu Leu Arg
   1 5 10
<210> 796
   <211> 16
   <212> PRT
   <213> Homo sapiens
<400> 796
   Gln Asn Gly Asn Ile Lys Ile Gly Gln Asp Gly Gly Cys Lys Leu His
   1 5 10 15
<210> 797
   <211> 12
   <212> PRT
   <213> Homo sapiens
<400> 797
   Gln Asn Gly Asn Ile Lys Ile Gly Gln Asp Tyr Lys
   1 5 10
<210> 798
   <211> 12
   <212> PRT
   <213> Homo sapiens
<400> 798
   Gln Asp Gly Asn Ile Lys Ile Gly Gln Asp Tyr Lys
   1 5 10
<210> 799
   <211> 13
   <212> PRT
   <213> Homo sapiens
<400> 799
   Val Ala Gln Asn Gly Asn Ile Lys Ile Gly Gln Asp Lys
   1               5                   10
<210> 800
   <211> 13
   <212> PRT
   <213> Homo sapiens
<400> 800
   Val Ala Gln Asp Gly Asn Ile Lys Ile Gly Gln Asp Lys
   1               5                   10

## Description

The present invention relates to assays for detection of biochemical markers valuable for diagnostic purposes in cardiovascular disease and prognosis of disease development, including biochemical markers indicative of the risk of cardiovascular events resulting from atherosclerotic development and plaque instability.

Worldwide, cardiovascular disease (CVD) is the leading cause of morbidity and mortality. At present, there are no effective and non-invasive diagnostic methods that allow for diagnosis and classification of patients into different risk-groups and for the diagnosis of low risk patients. Diagnostic and prognostic tools are composed mainly of multivariate analysis of simple markers, such as age, smoking and various lipid and lipoprotein concentrations.

CVD covers several clinical syndromes, primarily, angina pectoris, myocardial infarction (coronary thrombosis) and stroke. All of these syndromes are usually the sequelae of complicated atherosclerosis.

Atherosclerosis begins with intimal thickening in childhood and progresses to fatty streaks in the intima of arteries - these lesions are characterized as type I and II, respectively. Fatty streaks are the earliest macroscopically visible lesions in the development of atherosclerosis and occur among almost all human beings of all races and societies. In the non pathogenic state, endothelial cells (EC) resist adhesive interactions with leukocytes. However, the actions of proinflammatory cytokines and accumulated oxidized lipoprotein in the arterial wall during atherogenesis, initiate expression of adhesion molecules, such as intercellular adhesion molecules (ICAM)-1 and vascular cell adhesion molecules (VCAM)-1, on the surface of aortic ECs. This allows for capturing and transmigration of leukocytes through the endothelial surface, into the intimal part of the vessel wall. The development of plaques involves an increasing number of smooth muscle cells (SMC) that undergo displacement and apoptosis, which results in increased matrix turnover. The impaired collagen synthesis can result in a weakened fibrous cap and an atherosclerotic plaque that is more prone to rupture; however, most investigators believe that the actions of a proteolytic enzymes such as matrix metallo-proteases (MMPs) and other proteases importantly contribute to the risk of plaque rupture (Clarkson and Kaplan 509-28).

Plaques are divisible into two different types: 'vulnerable' and 'stabilized' plaques. However, for detailed histological analyses and molecular understanding, a more detailed classification is often used. There are three major stages in development of plaque: initiation, fatty streaks and the complex/advanced plaque (Stary H.C.).

Atherosclerotic plaques develop within the intima of arteries, and may be classified depending on their composition and structure. This classification divides lesions into eight types (Stary H.C.):
I. Macrophages loaded with and enlarged by lipid droplets (macrophage foam cells) are increased in the intima.
II. Macrophage foam cells accumulate in the deep part of the proteoglycan layer along with lipid droplets within the intimal SMC. The layers of foam cells are visible as fatty streaks. In type II lesions monocytes penetrate the endothelial lining by monocyte chemo attractant proteins (mainly MCP-1), which are over expressed in human atheroma. The early types of lesion (type I and II) can start in infancy and do not necessarily lead to plaque rupture. Furthermore, the development of atherosclerosis may end after the formation of type III lesion, and the formation of plaque is not predictable (Stary H.C.).
III. The type III lesion is determined as the intermediate lesion between the fatty streaks (type II) and the atheroma (type IV). These lesions contain pools of extracellular lipid and thereby expand the spaces between the normally closely adjoining SMCs of the deep musculo-elastic layer of the intima. The pools of material may replace proteoglycans and collagen fibres that normally reside here, but this occurs with little impact at this stage of atherogenesis.
IV. The atheroma is the first clinical sign of atherosclerosis. Displacement of SMCs in the intima of arteries by accumulating extracellular pools of lipids and disruption of the intimal architecture is a hallmark of a type IV lesion. The formation of the lipid cores is the end result of this SMC displacement. Formation of a lipid core accounts for the increased wall thickening. The lipid core is a large and well delineated region of the deep intima where the normal structural elements of this part of the arterial wall have been replaced by densely packed foam cell remnants, free lipids droplets, cholesterol crystals and calcium particles. SMCs normally resident in this area are decreased or completely absent at this stage of atherosclerosis progression. Any remnant SMCs become widely dispersed and have developed elongated cell bodies and very often unusually thick basement membranes. At this stage, the development of a layer overlying the lipid core begins. This layer consists of collagen and proteoglycan-rich intercellular matrix, SMCs with and without lipid droplets, macrophages, and foam cells.
V. The response to type IV lesion is the formation of a reparative fibrous tissue matrix, forming a fibrous "cap". Typically, these lesions will consist of layers of lipid cores and reparative tissue irregularly stacked on top of each other. Events such as hematoma and thrombus formation may additionally complicate these types of lesions. If not fatal, these lesion complications are integrated into the lesion and overgrown by a thin layer of reparative matrix tissue, consisting of collagens and proteoglycans. The content of extracellular matrix proteins collagen and proteoglycans increases in the atherosclerotic plaque during formation of the cap.
VI. The defects of the endothelium such as fissures, erosions, ulcerations, hematoma, thrombus, haemorrhage can if combined lead to more complicated lesion type designated type VI lesion.
VII. The lesion is often referred to as calcified lesion, where more than 50% of the lesion consists of mineral. In addition to calcifications, these lesions contain abundance of reparative fibrous connective tissue. When the SMCs trapped in this undergo apoptosis and disintegrate; their mineralized organelles become a part of the calcification.
VIII. The fibrotic lesion follows the calcific lesion. The fibrotic lesion may consist entirely of collagen and no lipid. (Stary H.C.)

Cardiovascular events are often the result of plaque rupture, in which inflammation and the release of proteases weaken the shoulder regions of the fibrous cap and allow the fatty materials in the plaque to come into contact with the blood precipitating a mural thrombus (Clarkson and Kaplan). Thinning of the fibrous cap by increased protease activity in the combination with decreased matrix production, is considered a hallmark of plaque instability increasing the risk of rupture. Vulnerability of plaques and their risk of rupture is an area of clinical interest. Definition of a vulnerable plaque (VP) is not standardized, but there is a general agreement stating existence of three histological hallmarks compared to stable plaque:
1) A larger lipid core (> 40 percent of total lesion).
2) A thinner fibrous cap (65 - 150 micrometers).
3) Large amount of acute inflammatory cells.
Major criteria for defining VP include: active inflammation (presence of monocytes, macrophages and T cells), thin cap with large lipid core, endothelial denudation with superficial platelet aggregation, fissured plaque, and > 90% stenosis of the artery. Other minor criteria include: superficial calcified nodule, intraplaque haemorrhage, endothelial dysfunction, and outward remodelling (Shin, Edelberg, and Hong).

Plaque complications, instability and rupture may be inhibited by medical treatment and/or lifestyle modification. In some cases, however, more invasive methods may be needed, i.e. angioplasty or bypass surgery.

Presently, diagnostic tools are based on either static image analyses still under development or low-technology methods such as systolic and diastolic blood pressure levels related to the risk of CVD. The field has devoted much attention to the development of multivariate analysis that may better identify patients at high risk. One such model is the SCORE-model (Systematic Coronary Risk Evaluation model). In 1994, with a revision in 2003, The European Atherosclerosis Society, The European Society of Cardiology and The European Society of Hypertension issued a set of recommendations regarding prevention of coronary heart diseases. This guideline is based on several assessment techniques, which have been developed to assess the risk of CVD in asymptomatic subjects, i.e. identification of asymptomatic high-risk patients. The SCORE-model integrates gender, age, smoking, systolic blood pressure and either total cholesterol or the cholesterol/HDL ratio as risk factors (Graham et al.).

In order to make a more detailed diagnosis, the SCORE model is not sufficient and imaging techniques are used. Imaging methods are therefore used mostly on patients in the high-risk group or during research.

### IMAGING TECHNIQUES

Coronary angiography (CAG) is currently the gold standard imaging technique for defining the degree of stenosis. CAG images the lumen of the vessel in two dimensions, but is restricted only to the lumen and not the vessel wall thereby CAG can not distinguish between an artery with a stable plaque and an artery with a vulnerable plaque. CAG is often used to determine whether a patient needs surgery; angioplasty or bypass. In order to determine if a point of luminal narrowing is an advanced plaque, other techniques are needed i.e. intravascular coronary ultrasound (IVUS) or angioscopy.
IVUS provides two-dimensional cross-sectional images of the plaque and vessel wall, and is considered as a method good for characterization of vessel wall and morphology and the degree of calcification, but poor for assessing the lipids in the lesion. However, IVUS is invasive and requires expertise and expense: therefore, its use is not wide spread. Angioscopy is another useful method in understanding and identifying atherosclerosis. Angioscopy is a direct visualization of plaque surface and has the capability of detecting colour of plaque and thrombosis. Angioscopy is, however, invasive and technically difficult, and so far it is has not been able to detect the degree of plaque extension. Another imaging technique that currently is receiving much attention is Magnetic Resonance imaging (MRI). MRI is non-invasive and able to identify carotid plaque at high risk of stroke. On the other hand, MRI is not the best technique to image coronary arteries, because of small plaque sizes and location of the coronary arteries. Other imaging techniques are under development, i.e. elastography, thermography and optical coherence tomography (Schaar et al.).

The imaging techniques mentioned are all under development and alone, none can identify a vulnerable plaque, but they are useful tools in understanding both the molecular events and plaque turnover prior to rupture. Presently, the only opportunity to diagnose CVD at an early stage is to utilize a range of risk factors for established coronary heart disease, peripheral artery disease and cerebrovascular atherosclerotic disease of the patient in question, as well as close relatives of the patient.

### PRESENT BIOCHEMICAL MARKERS

At present, several biochemical markers are known as risk factors for atherosclerosis. Recently much attention has been directed to the measurement of biochemical marker concentrations in serum; both lipids such as total cholesterol, low-density lipoprotein cholesterol (LDL-C) and the high-density lipoprotein cholesterol (HDL-C) and inflammatory markers such as C-Reactive Protein (CRP), Interleukin-6 (IL-6), Interleukin-18 (IL-18), Tumor Necrosis Factor-alpha (TNFα), CD40, CD40 ligand (CD40L) and others.

Among lipoprotein markers, there have been at least two noteworthy advances. The size of LDL particles seems to predict the degree of atherosclerosis progression. Increased concentrations of small LDL particles are more related to CVD risk than increased concentrations of large particles(Gardner, Fortmann, and Krauss).

The level of HDL-C is strongly related to triglyceride, and high triglyceride level is correlated to a higher risk of CHD. A cohort study by Jeppesen et al.(2003) found high TG/low HDL-C as the strongest risk factors of IHD (ischemic heart disease)(Jeppesen et al.).

Lipid profiles are important for evaluation of risk factors, but do not allow understanding and measurement of the molecular events associated with plaque turnover. A number of biochemical markers have been suggested as risk factors for CVD, although not specific product of the disease. These include CRP and Bone natriuretic peptide (BNP) (see Table 1). Table 1 summarizes some of the known markers of CVD.

**Table 1: A selection of present biochemical markers in CVD.**

| **Marker** | **Type** | **Description** |
|---|---|---|
| C-reactive protein (CRP) | Inflammatory | Produced in the liver, increases during inflammatory states. |
| Pregnancy-associated plasma protein (PAPP-A) | Inflammatory | Zinc-binding protein that acts as an enzyme, specifically a metallopeptidase. |
| Interleukin-6 (IL-6) | Inflammatory cytokine | Elevated level in heart failure and myocardial infarction. |
| Inteleukin-8 (IL-8) | Inflammatory cytokine | Elevated in myocardial infarctions. |
| Interleukin-18 | Inflammatory cytokine | Elevated in myocardial infarction. |
| TNF-α (Tumor Necrosis Factor) | Cytokine | Conc. Elevated in the settings of heart failure. |
| MCP-1 | Chemokine | Recruits monocytes from the blood into early atherosclerotic lesion. |
| Intercellular adhesion molecule-1 (ICAM-1) | Adhesion molecule | Elevated in myocardial infarctions and stroke. |
| Vascular cellular adhesion molecule-1 (VCAM-1) | Adhesion molecule | Elevated in myocardial infarctions and stroke. |
| Brain natriuretic peptide (BNP) | Neurohormonal activity | Produced in atria and ventricles of normal healthy heart. |
| Lipoprotein-associated phospholipase A2 (Lp-PLA₂) | Phospholipase | LDL-associated Lp-PLA₂ has proatherogenic effects. |
| Creatine phospokinase (CK-MB) | Enzyme | Useful as early detection of myocardial infarction. |
| Myeloperoxidase (MPO) | Heme enzyme | Activates metallo-proteases and promotes destabilization of plaque. |
| Myoglobulin | Heme protein | Released upon tissue necrosis. |
| CD40L | Protein | Released in the early stages of atherogenesis through to plaque rupture. Elevated in stroke. |
| Troponin T (TnT) | Protein | Tool for risk stratification. |
| Heart-Type Fatty Acid-binding protein (H-FABP) | Protein | H-FARB is released from the heart immediately after infarction. |
| Microalbuminurea | Protein | Marker of vascular endothelial dysfunction. |
| Low density lipoprotein cholesterol (LDL-C) | Lipoprotein | Transport cholesterol in the blood. |
| High Density lipoprotein cholersterol (HDL-C) | Lipoprotein | Holds antioxidant and antiinflammatory properties. |
| Triglyceride | Lipid | |
| PIIINP | Procollagen | Marker of type III collagen turnover. |

Thus, a range of different biochemical markers have been suggested as markers of cardiovascular events. Wang et al (2006) have measured 10 different biochemical markers in 3200 patients participating in the Framingham study, described in Table 1. The conclusion was that the measurement of 10 biochemical markers only contributes moderately to diagnosis over and above standard risk factors. Of the 10 biochemical markers, B-type natriuretic peptide level, C-reactive protein level and the urinary albumin-to-creatinine ratio showed the best correlation between marker and death/cardiovascular events (Wang et al.).

### C-REACTIVE PROTEIN

C-reactive protein (CRP) is an acute phase serum protein produced by the liver in response to different clinical conditions such as, inflammation, infection, or trauma (Gabay & Kushner 1999). The production of CRP is induced by cytokines such as IL-6, released from the affected or damaged tissues. The physiological role of CRP is yet unknown and discussions on its pro-or antiinflammatory actions are ongoing.

There is accumulating evidence that the CRP is a risk factor for CVD in humans. In a study by Ridker et al. 2002, CRP was shown to be a better predictor of future cardiovascular events than LDL cholesterol, in a large population consisting of 28,000 healthy women followed for eight years for the occurrences of acute myocardial infarction, stroke, coronary revascularization, or death from CVD. Many other studies have also reported that baseline CRP levels constitute an independent risk factor for cardiovascular events (Thompson et al. 1995, Mendall et al. 1996, Kuller et al. 1996, Ridker et al. 1997, Tracy et al. 1997, Ridker et al. 2000).

It has been speculated that circulating CRP only reflects the general inflammation occurring in the atherosclerotic process and is not an active component in the pathogenesis of the disease. However, several lines of evidence also support the view that CRP has a role in atherogenesis. First, chronic infections giving rise to CRP are also associated with increased risk for CVD (Leinonen & Saikku 2002). Secondly, we and others have identified CRP is in different levels of atherosclerotic lesions (Reynolds & Vance 1987, Hatanaka et *al.* 1995). Finally, CRP has been shown to have proatherogenic properties *in vitro*: CRP may activate endothelial cells to produce adhesion molecules (Pasceri et *al.* 2000). It may also decrease the production of eNOS in endothelial cells (Venugopal et *al.* 2002) and enhance the uptake of LDL by macrophages (Zwaka et *al.* 2001)

Ying et al. describe a method of bioassay for the quantification of peptide fragments comprising a neo-epitope formed by cleavage of CRP by a proteinase, wherein a sample comprising the peptide fragments is contacted with CRP neo-epitope-specific monoclonal antibodies and the level of binding of the antibodies and the peptide fragment is determined.

The present invention provides a method of bioassay for the quantification of peptide fragments of CRP comprising an N-terminal neo-epitope amino acid sequence KAFVFP, said method comprising contacting a sample comprising said peptide fragments with a monoclonal antibody or fragment thereof having specific binding affinity for said N-terminal neo-epitope amino acid sequence KAFVFP, and determining the level of binding of said monoclonal antibody or fragment thereof to peptide fragments in said sample.

Preferably, the invention further provides, wherein the sample is a patient derived sample, said method further comprising comparing the determined level of said binding of said peptide fragments with values characteristic of (a) comparable healthy individuals and/or (b) a pathological atherosclerotic condition.

The invention also provides a monoclonal antibody or fragment thereof that has specific binding affinity for the following sequence at the N-terminal of a peptide: KAFVFP.

The invention further provides a cell line producing the monoclonal antibody or fragment thereof, and an immunoassay kit comprising the monoclonal antibody or fragment thereof, and a competition agent which binds said monoclonal antibody or fragment thereof, and optionally one or more of a wash reagent, a buffer, a stopping reagent, an enzyme label, an enzyme label substrate, calibration standards, an anti-mouse antibody and instructions for conducting an assay using said kit.

### BRAIN NATRIURETIC PEPTIDE

Brain (B-type) natriuretic peptide (BNP) is a peptide hormone secreted by the ventricles of the heart in response to excessive stretching of cardiac myocytes in the ventricles. T-proBNP (the inactive N-terminal fragment) is, along with the active hormone (BNP), released to the blood stream upon cleavage of proBNP. Both BNP and NT-proBNP have been suggested as potential biochemical markers of cardiovascular events (Wang et al.).

### CHEMOKINES

Chemokines are also potential markers of CVD; chemokines are low molecular weight cytokines produced in inflammation. One major chemokine in relation to CVD is monocyte chemo attractant protein 1 (MCP-1). MCP-1 appears to play an early and important role in the recruitment of monocytes to atherosclerotic lesions. In a study using a monkey model of atherosclerosis, plasma concentrations of MCP-1 were highly associated with plaque size and plaque complications (Register et al.).

### LIPIDS INCLUDING CHOLESTEROL

Recently much attention has been directed to the measurement of cholesterol concentrations in serum; both total cholesterol, as well as the concentrations of low-density lipoprotein cholesterol (LDL-C) and the high-density lipoprotein cholesterol (HDL-C). Among lipoprotein markers, there have been at least two noteworthy advances. First, LDL particle size seems to predict the degree of atherosclerosis progression. Increased concentrations of small LDL particles are more related to CVD risk than increased concentrations of large particles (Gardner et al). Secondly, the cholesteryl oleate content of LDL particles may become a particularly important marker of CVD risk. In monkeys, enrichment of lipoprotein particle cores with cholesteryl oleate was strongly and positively associated with more severe coronary artery atherosclerosis (Rudel et al) and was additive to the contributions of LDL and HDL cholesterol concentrations. These findings in experimental animals are further supported by earlier human studies (Lawrie et al) that showed plasma lipoproteins with lower proportions of cholesteryl linoleate (and conversely higher proportions of cholesteryl oleate) are typical of patients with complications of CHD (coronary heart disease) compared to normal controls.

The level of HDL-C is strongly related to triglyceride, and high triglyceride level is correlated to a higher risk of CHD. A cohort study by Jeppesen et al found high TG/low HDL-C as the strongest risk factors of IHD (ischemic heart disease).

These lipid profiles are important for evaluation of risk factors, but do not allow understanding and measurement of the molecular events associated with plaque turnover. A number of biochemical markers have been suggested as risk factors for CVD, although these are not the specific products of the disease. These include CRP and ApoE.

### LIPOPROTEINS

The biomarker most commonly used to predict CVD is cholesterol concentration (both total and the cholesterol/HDL ratio). These are used along with other risk factors, such as blood pressure and level of LDL. Both factors are used in the previously mentioned SCORE-model. The level of LDL is important as LDL transports cholesterol in the blood and accumulation of oxidized LDL can promote atherosclerosis (Graham et al)**.** In addition, a significant association between CHD and triglyceride (TG) levels are found, in which an increased risk of CHD was associated with increasing TG levels, independent of both LDL-C and HDL-C levels, although the level of cholesterol is viewed as one of the major risk factors of CVD (Jeppeson et al).

### APO-E

Apolipoprotein E is found in chylomicrons, VLDL, and HDL. It is mainly synthesised in the liver, but also in many other organs such as brain, spleen, kidney (Siest et al. 1995). ApoE plays an essential role in lipoprotein metabolism by acting as a ligand for two receptors: the LDL receptor and Apo E specific chylomicron remnant receptor. The interaction between ApoE with these receptors gives a basis for the metabolic regulation of cholesterol. Polymorphism at the apoE gene locus results in three alleles found in most populations: ε2, ε3 and ε4 that determine six apoE phenotypes. Isoforms differ from each other by one amino acid at positions 112 and 158. Apo E2 has cysteine on both residues and E4 has arginine at both positions. Apo E3 contains cysteine at position 112 and arginine at 158. Allele frequencies differ in different populations. Some studies have assessed the possible relationship between apoE polymorphism and atherosclerosis. A meta-analysis of 14 observation studies demonstrated ε4 allele as associated with coronary disease among both men and women (Wilson et al. 1996). Furthermore, ε4 allele has been associated with carotid artery atherosclerosis (Terry et al. 1996, Cattin et al. 1997, Haraki et al. 2002).

ApoE is 299 amino acids long and transports lipoprotein, fat soluble vitamins, and cholesterol into the lymph system and further into the blood circulation. ApoE is primarily synthesized in the liver. Currently, there are seven mammalian receptors for ApoE which belong to conserved low density lipoprotein receptor gene family.

### ADDITIONAL BIOCHEMICAL MARKERS

Microalbuminurea (albumin to creatinine level) is also a potential and independent marker. The urinary albumin excretion rate is a marker of changes in the kidney and, when compared with a small creatinine elevation, it may indicate atherosclerosis (Wang et al.).

Of the procollagen markers, the marker for type III collagen turnover rate (PIIINP) has been investigated as a prognostic marker for hypertension and has been associated with myocardial infarction. Satta et al. examined the correlation between abdominal aortic aneurysm (AAA) and the concentration of the procollagen (PIIINP) in blood. They showed that the turnover of collagen type III is increased in patients with AAA and may be due to enhanced synthesis, enhanced degradation or a combination of both. In the same experiment, the carboxyterminal propeptide of type I procollagen (PICP) was measured, and there was no accelerated synthesis of type I collagen in the aneurysm sac.

### PROTEIN PROFILE OF PLAQUE

Human arteries can be divided into larger or elastic arteries, medium or muscular arteries, and small arteries. The walls of arteries are composed of intima, media and adventitia, separated by the internal elastic lamina and external elastic lamina. The intima consists of connective tissue, smooth muscle cells and a few isolated macrophages. The boundaries of the intima can be defined as a layer between the luminal surface of the endothelium and the internal elastic lamina.

The arterial intima can further be divided into two layers. The inner layer, called a proteoglycan layer, composed of abundant proteoglycans, smooth muscle cells and macrophages. The lower layer, musculoelastic layer, is composed of abundant smooth muscle cells and elastic fibers. In the normal conditions, the two layers of the intima are barely visible by light microscopy, but are distinct and prominent when intimal thickening occurs.
The media is the muscular part of the arterial wall, composed of smooth muscle cells, elastin, collagen fibrils.

The adventitia, outer layer, is highly microvascular and contains collagens, elastic fibrils, smooth muscle cells, and lymphatic channels.

Human atherosclerotic plaques are characterized by a lipid-rich core covered by a fibrous cap composed of fibrillar collagens, elastin, proteoglycans and SMC. Proteoglycans hyaluronan are major nonfibrillar components of the extracellular matrix that have the potential to affect lesion development by regulating events such as lipid accumulation, thrombosis, and cell proliferation and migration and by affecting the material properties of the tissue (Wight 1995). Infiltrating ApoE and CRP are also present and we have demonstrated localisation of both in atherosclerotic plaques of coronary arteries at different stages of the atherosclerotic disease.

### ApoE and CRP DISTRIBUTION IN HUMAN BEINGS

Table 2 below shows the distribution of ApoE and CRP in the human body.

**Table 2:**

| Protein | Sites of expression |
|---|---|
| APOE | Blood, Serum, Plasma, Liver, Saliva, Monocyte, Cerebellum, Cerebrospinal Fluid, Frontal Cortex, Hippocampus, Temporal Cortex |
| CRP | Blood, Kidney, Liver, Peritoneal Fluid, Plasma, Serum |

Table 3 below illustrates known interactions of ApoE and CRP with proteins demonstrated in vivo and/or in vitro.

**Table 3**

| Protein | Interactions with proteins |
|---|---|
| ApoE | Albumin, Amyloid beta A4 protein, Macroglobulin, Microtubule associated protein tau, LDL receptor, Cathepsin B, Neurofilament 3, Phospholipid transfer protein, Prion protein, VLDL receptors, Scavenger receptors class B, |
| CRP | Serum amyloid P, Complement factor H, Fibronectin 1, Histone 1, FC gamma RI, FC gamma RIIb, CD32, Platelet glycoprotein VI, Leptin. |
| | *Non protein interaction*: Calcium, Cholesterol |

### COLLAGEN DISTRIBUTION IN HUMAN BEINGS

Collagen is widely distributed in the human body, i.e. ~ 30% of the protein mass in the human body is composed of collagen. In Table 4, the major collagen types are listed with their major tissue distribution.

**Table 4**

| Collagen type | Tissue distribution |
|---|---|
| I | Skin, bone, tendon, ligament, cornea |
| II | Cartilage, vitreous |
| III | Skin, vessel, intestine, uterus |
| IV | Basement membranes |
| V | Bone, skin, cornea, placenta |
| VI | Bone, cartilage, cornea, skin, vessel |
| VII | Skin, bladder, oral mucosa, umbilical cord, amnion |
| VIII | Descemet's membrane, vessel, bone, brain, heart, kidney, skin, cartilage |
| XIII | Endothelial cells, skin, eye, heart, skeletal muscle |
| XIV | Vessel, bone, skin, cartilage, eye, nerve, tendon, uterus |
| XXI | Vessel, heart, stomach, kidney, skeletal muscle, placenta |

Type I collagen is the most abundant collagen and is found in most connective tissue. It is especially important for the structure of bone and skin. The major content of collagen in the human body is distributed in the skin and bone, where the major collagenous components are type I and III collagens. Collagen type III is a major component of large arteries, and is also found in smaller amounts in tissues rich type I collagen. In addition, collagen type IV is found in the basement membrane and around blood vessels and nerves. The most common localization of type V collagen is within the characteristic collagen fibrils, in association with the collagen type I and III (Garrone et al).

Some collagens have a restricted tissue distribution: for example, type II, which are found almost exclusively in cartilage (Mayne R.).

Collagen fibrils often consist of more than one collagen type. For example, the type I collagen fibrils often contain small amounts of types III, V and XII, whereas the type II collagen fibrils of cartilage also contain types IX and XI.

### COLLAGENS IN ARTERIES

In arteries, six types of collagen are found (types I, III, IV, V, VI and VIII), where type I and III are the most abundant, 80 - 90 % of collagen content. Type I and III are also predominant in the vessel wall. They appear to be co-distributed in different amounts within all three layers of the artery wall, synthesis of collagen type I and III tends to be located in the intima (Mayne R) .

### COLLAGENS AND OTHER STRUCTURAL PROTEINS IN PLAQUE - TURNOVER

During development of atherosclerotic plaques collagen is accumulated in the fibrous cap (Stary H.C.). In a study by Katsuda et al (1992) collagen types I, III and IV were found in the thickening intima at all stages of the lesion in aortic human tissues. Collagen type VI was distributed in the basement membrane in the region of intimal cells and in advanced lesions also detected around the elongated SMC. Earlier studies of type I and III have provided evidence of an equal distribution in atherosclerotic arterial wall (Shekhonin et al). According to McCullagh et al (1980) type III is the predominant collagen in normal human aortic media (appr. 70 % of the extractable collagen). A recent study by Eriksen et *al* (2006) found a decrease of total collagen content in human aortic valve depending on the degree of stenosis. The molecular mechanism of stenosis is thought to be similar to atherosclerosis. In healthy aortic valves, the collagen content is mainly type I and III. During stenosis, the total content of collagen decreases, which is presumably caused by an increased turnover of collagen type I. Type I collagen accounted for approximately 60-70% of total collagen; whereas the proportion of type III collagen was 30-40% both in healthy valves and in calcified valves.

Type V collagen also increases in advanced atherosclerotic lesions and is distributed throughout the extracellular matrix in both aortic media and in the subendothelial region of the plaques (McCullagh et al).

There seems to be a consensus that the main collagen types to be found in atherosclerotic plaque are type I and III, whether they are equally distributed in healthy and atherosclerotic vessel remains to be further investigated.

In the study by Katsuda et al (1992) no collagen was detected in the center of the atheroma of more advanced lesions.

### ELASTIN

Elastin is one of the most stable proteins in the body and is found in most connective tissue caused by its elasticity and resilience. Elastin dominates the protein content of the arterial wall, where it is the major extracellular matrix protein.

Elastin is the main component in elastic fibers and is related to calcification. Vascular calcification occurs at two distinct sites within the vessel wall: the intima and the media. Intimal calcification is related to atherosclerosis, mainly within the necrotic core. Calcified elastic fiber constitutes the plaque shoulder where the plaques are most prone to rupture; suggesting that calcification of elastic fiber may affect plaque stability (Bobryshev Y. V.). In atherosclerosis, the content of elastic fibers decreases along with lipid deposition, this generates an enhanced susceptibility to elastin degrading enzymes. Thereby the content of elastin in contrast to collagen decreases as the lesion develops.

### DISTRIBUTION OF ELASTIN IN HUMAN BEINGS

Table 5 shows the distribution of Elastin in the human body.

**Table 5:**

| Protein | Sites of expression |
|---|---|
| Elastin | Aorta and other Blood vessels, Lung, Skin Fibroblasts |

Table 6 illustrates known interactions of Elastin with proteins demonstrated in vivo and/or in vitro.

**Table 6:**

| Protein | Interactions with proteins |
|---|---|
| Elastin | Decorin, Elastase, Fibrillin, Fibulin, Lysozyme, Lysyl Oxidase, Galectin, Biglycan, Nidogen, Ficolin, Proteinase3. |

### PROTEOGLYCANS AS MATRIX COMPONENTS

Proteoglycans (PG) are polysaccharide-protein macromolecules localized predominately in the intercellular matrix of vessel wall (Salisbury and Wagner 1981). PGs are macromolecules characterized by the presence of one, or more, long un-branched and highly polyanionic sugar side chains called GAGs, covalently attached to a core protein through a link region. The repeating unit of the GAG consists of an amino sugar, either N-acetyl-glucosamine (GlcNAc) or N-acetyl-galactosamine (GaINAc), and a hexuronic acid, either glucouronic acid (GlcA) or iduronic acid (IdoA). One or both of the sugars in the repeating unit contain one or more sulfate groups (Rodriguez-Lee 2007). In addition to the GAG chains, most core proteins carry N- and/or O-linked oligosaccharides.

### CLASSIFICATION AND NOMENCLATURE OF PGs

PGs are a very heterogeneous group of macromolecules. A single type of core protein can vary in the number and type of attached GAG chains. The length of the chains and the arrangement of the sulfated residues along the chains vary also.

Four main classes of GAGs are distinguished according to the structure of the repeating disaccharide unit: chondroitin sulfate (CS) and dermatan sulfate (DS), heparin sulfate (HS) and heparin, hyaluronan, and keratin sulfate (KS).

Hyaluronan is the simplest of GAGs. In contrast to all of the others, it does not contain any sulfated sugars. All of its disaccharide units are identical, its chain length is enormous and it is not linked to any core protein.

KS is a sulfated polylactosamine chain. KS-I has originally been described in cornea, and is N-linked to aspargine residues in the core protein, whereas KS-II or cartilage KS, is O-linked to serine or threonine residues (Funderburgh 2000).
PGs can be classified according to several parameters:
- Attached GAG chain (CS/DS- or HS containing PGs)
- Topographic distribution in relation to the cell (extracellular and basement membrane PGs, cell-associated PGs or intracellular PGs)
- Core protein homology (hyalectans, small leucine-rich PGs (SLRPs)

Chondroitin/dermatan sulfate PGs (Versican, aggrecan, neurocan, and brevican) belong to the family of hyaluronan-binding proteoglycans. This gene family is collectively termed hyalectans. Each family member has a characteristic distribution, with aggrecan prominent in cartilage, neurocan and brevican prominent in the central nervous system, and versican present in a variety of soft tissues, including arterial walls. The gene and protein structure of versican follows a domain template. The amino-terminal globular end (G1) binds to GAG hyaluronan, and the carboxy-terminal globular domain (G3) resembles the selectin family of proteins, consisting of a C-type lectin adjacent to two epidermal growth factor (EGF) domains and a complement regulatory region. The middle region of versican core protein is encoded by two large exons that specify the CS attachment regions of versican. The region encoded by exon 7 is called αGAG, whereas the region encoded by exon 8 is called βGAG. Four mRNA transcripts arise from alternative splicing of versican, giving rise to V0, V1, V2, and V3 which differ in the length of the core protein and the number of attached GAGs (Dours-Zimmermann and Zimmermann). The number of potential GAG attachment sites in human versican is: 17-23 for V0, 12-15 for V1, 5-8 for V2, and none for V3 (Wight 617-23).

Decorin and biglycan are members of SLRP-family that comprises at least nine members grouped into three classes (I, II, and III) and different subfamilies. They are all characterized by the presence of a central domain containing leucine-rich repeats to achieve strong presence of a central domain containing leucine-rich repeats to achieve strong protein-protein interactions. Decorin and biglycan are members of class I, and show highest amino-acid homology of the family (-57%) and are the only SLRPs with a propeptide. The propeptide is highly conserved across species and may function as a recognition sequence for xylosyltransferase, the first enzyme involved in synthesis of the GAG chain.

Versican, decorin, and biglycan are the major CS/DS PGs in the matrix of the mammalian arterial wall (Wight et al. 1986). The size of Versican V0 core protein is 370 kDa, which makes it roughly 10 times larger than decorin 36 kDa and biglycan 38 kDa. Side-chains show a wide range of sizes, but generally average around 40-90 kDa each.

Heparan sulfate proteoglycans: HSPGs are divided into five distinct classes of cell-associated and pericellular PGs and they account for at least 95% of the HS of mammalian cell surfaces, basement membranes and ECMs. The cell-associated HSPGs include integral membrane syndecans and anchored glypicans. Pericellular HSPGs include mainly perlecan, agrin. These PGs are termed pericellular because of their close association with the plasmamembrane via integrins (Whitelock and Iozzo).

Perlecan is a modular HSPG that is expressed in nearly all basement membranes as well as mesenchymal organs and connective tissues and is one of the largest single-chain polypeptides found in vertebrate and invertebrate animals. The five modules of perlecan and its HS side-chains take part in a large number of molecular interactions such as with fibroblast growth factor - 2, vascular endothelial growth factor (VEGF), platelet-derived growth factor (PDGF), and other matrix proteins. The core protein of human perlecan is -470 kDa and, together with numerous O-linked oligosaccharides and four HS side chains, it can reach a molecular weight of over 800 kDa (Knox and Whitelock).

### PROTEOGLYCAN DISTRIBUTION

Proteoglycans (PGs) are macromolecules distributed almost everywhere in the human body. The structure and size of PGs vary extremely. The basic structure of all PGs includes a core protein and at least one, but often many carbohydrate chains - glycosaminoglycans (GAGs). PGs can be found intracellularly, on the surface of cells, and in the extracellular matrix. The structural diversity of PGs suggests numerous biological functions, see Table 7.

**Table 7:**

| Proteoglycan family | Proteoglycan | Distribution | Function |
|---|---|---|---|
| Keratan sulphate - Small leucine rich PG | Lumican | Cornea | Collagen fibril organization and growth. Corneal transparency. Epithelial cell migration and tissue repair. |
| Chondroitin sulphate | Versican | Smooth muscle cells of blood vessels. Epithelial cells of skin. Cells of central and peripheral nervous system. | Cell adhesion, migration and proliferation. |
| Dermatan Sulphate (Small leucine rich PGs) | Decorin | Connective tissue. Artery wall. | Plays a role in matrix assembly. |
| | Biglycan | Extracellular matrix tissue: bone, cartilage, tendon, arteries. | Role in mineralization of the bone. |
| Heparan sulphate | Perlecan | Extracellular matrix of blood vessels. | Binds to and cross-links many extracellular matrix components and cell-surface molecules. |
| Chondroitin sulphate - Large aggregating PG | Aggrecan | Cartilage. Extracellular matrix. | Gives tissues ability to resist compressive loads. |

Table 7 above gives an overview of PG distribution and function.

### PROTEOGLYCANS IN ARTERIES

At least five types of PGs are present in the extracellular matrix of the artery wall; versican - which interacts with hyaluronan to form large aggregates; small-leucine rich decorin and biglycan, which interact with fibrillar matrix components like collagen and elastin; heparan sulphate - perlecan, which is a component of basal lamina and keratin sulphate - lumican (Talusan et al.).

Versican is one of several ECM molecules that accumulate in lesions of atherosclerosis. Although a number of studies indicate that versican is clearly capable of binding to LDL, versican is generally not detected in the lipid-rich center of the necrotic core (Evanko et al.).

Lumican has been shown to directly bind to macrophages and to enhance macrophage migration. Lumican may therefore directly influence macrophage behavior in the vascular intima as well as stimulate the formation of the necrotic core, characteristic of advanced atherosclerotic lesions (Funderburgh et al. 1997).

Biglycan is found in the fibrous cap. Versican and biglycan have affinity for LDL and form insoluble complexes, which accelerates oxidation of LDL. Biglycan may contribute to the pathogenesis of atherosclerosis by trapping lipoproteins in the artery wall. Changes in the proteoglycan metabolism of the intima of arteries constitute the initial lesions of atherosclerosis and the accumulation of proteoglycans play a fundamental role in the progression of atherosclerosis (Kunz J.).

Perlecan was reported in human intimal hyperplasia as one of the central components of intimal extracellular matrix, by mass spectrometry-based analysis and by immunohistochemistry.

Table 8 illustrates distribution of some PGs in immunohistochemical stainings of PGs in normal and atherosclerotic arteries (Evanko et al).

**Table 8:**

| | Normal Vessel | | Fibrous Core | | | | |
|---|---|---|---|---|---|---|---|
| PG or GAG | Endothelial cells | SMCs | Endothelial cells | SMC's | Macrophages | Fibrous Cap | Plaque core |
| Perlecan | +++ | ++ | +++ | +++ | ++ | + | +++ |
| Decorin | + | ++ | + | + | +++ | + | +++ |
| Biglycan | -/+ | ++ | ++ | +++ | + | +++ | -/+ |
| Versican | - | ++ | -/+ | +++ | - | +++ | - |
| Hyaluronan | ++ | + | ++ | +++ | +++ | +++ | +++ |
| Staining results: - undetectable; -/+ variably detectable; + detectable; ++ moderate; +++ strong | | | | | | | |

### PROTEOGLYCAN INVOLVEMENT IN MATRIX REMODELLING

A study of atherosclerosis progression in nonhuman primates has demonstrated that accumulation of specific PGs varies with lesion severity and with the distribution of cells and growth factors, suggesting that different PGs play distinct roles during progression of atherosclerosis. Different levels of specific PGs may directly affect material properties of the tissue via their contribution to altering structural arrangements of fibrous matrix components such as elastin and collagen.

Versican and hyaluronan show similar localization in the matrix, suggesting aggregate formation between the two in the atherogenesis. The marked increase in versican and hyaluronan in early lesions could suggest that they play a role in early atherosclerotic lesions, such as proliferation and migration of SMCs and leukocytes. Furthermore, versican and hyaluronan are principal matrix components of human restenotic lesions and have been shown to contribute to neo-intimal thickening after vascular injury *in vitro.* An abundance of versican early in atherogenesis could also predispose the extracellular matrix to increase lipid entrapment due to the binding of lipoproteins to chondroitin sulphate chains of versican. This idea is supported by co-localization of versican with apoprotein (a) and apolipoprotein E in transplant arteriopathy (Evanko et al). Loss of versican from the plaque may result in matrix instability.

This is further evidenced by upregulation of versican gene observed after vascular injury. Versican was also here identified in all stages of atherogenesis; in the intima of early developing plaques, but also throughout advanced lesions and at the borders of lipid-filled necrotic cores as well as at the plaque-thrombus interface (Wight and Merrilees 2005). These observations implicate versican in lipid accumulation, inflammation, and thrombosis. Furthermore, versican plays an important role in the assembly of ECM and in control of elastic fiber fibrillogenesis, which is of fundamental importance in ECM remodelling during vascular disease (Wight and Merrilees 2005).

The role of biglycan in arterial cell biology is unclear. Some immunohistochemical studies have showen biglycan association with collagen I and III staining in human restenotic lesions (Evanko et al.) .

The importance of biglycan as matrix protein was further stated by the generation of BALB/cA mice homozygous for a null mutation of the biglycan gene, where 50% of biglycan-deficient male mice died suddenly within the first 3 months of life as a result of from aortic rupture. This observation suggests biglycan to be essential for the structural and functional integrity of the aortic wall, as well as a potential role of biglycan gene defects in the pathogenesis of aortic dissection and rupture in humans. (Heegaard et al. 2007)

Other studies indicate that biglycan is a major PG associated with elastin in primate arteries; these observations are similar to those of in human coronary arteriopathy (Evanko et al).

Decorin has been shown to bind to collagen and regulate collagen fibril formation (Brown and Vogel) (Danielson et al.).

### PROTEASE PROFILES

Proteases hydrolyse peptide bonds and are responsible for the degradation of extracellular matrix proteins such as collagen, proteoglycans and elastin in atheroma, see Table 9. In atherosclerotic plaques three main types are found: metallo-proteinases (i.e. MMPs), serine proteases and cysteine proteases (i.e. cathepsins). Cathepsins and MMPs are responsible for degradation of all extracellular matrix proteins. As matrix is essential for plaque stability, its removal from the fibrous cap by proteases may invoke plaque rupture (Stary H.C.).

In Table 9 a variety of proteases found in atherosclerotic plaque are listed.

**Table 9 Proteases detected in atherosclerotic plaques.**

| Protease | Degradation substrates |
|---|---|
| Cathepsin K | Proteoglycans, elastin, collagen |
| Cathepsin S | Proteoglycans, elastin, collagen |
| Cathepsin L | Proteoglycans, Collagen type I |
| Cathepsin B | Proteoglycans |
| MMP-1 | Collagen type I, II and III |
| MMP-2 | Proteoglycans, elastin |
| MMP-3 | Proteoglycans, collagen type III, elastin |
| MMP-8 | Proteoglycans, collagen type I, II and III |
| MMP-9 | Elastin, collagen type I and III |
| MMP-13 | Proteoglycans, collagen type I, II and III |
| MMP-18 | Collagen type I |

The main source of MMP expression in the plaque is suspected to be related to macrophage and SMC activity. Macrophages in plaques contain abundant MMP-1, -8, -9, and -13 and co-localize with sites of collagen and proteoglycan degradation in situ (Kunz J.). Furthermore, own data suggest localization of MMP-8 and Cathepsin K in atherosclerotic plaques.

### MATRIX METALLOPROTEINASES (MMP)

MMP is a large group of endopeptidases, capable of degrading most components of the ECM. Presently, more than 25 MMPs have been identified. Metallo-proteinases are characterized by an active site containing a metal atom, typically zinc, and are secreted as zymogens. Specific tissue inhibitors, TIMPs, regulate the activity of MMPs. A great variety of MMPs are found in the atherosclerotic plaques. They are most often located in macrophages bordering the fibrous cap, within plaque shoulders in SMC and macrophages and are rarely identified within the fibrous cap (Kunz J.). ]

MMPs are classified in different groups according to their substrate specificity: Collagenases, which degrade fibrillar collagen, like collagen type I, II, III and V but also proteoglycans; Gelatinases, which degrade proteoglycans, collagen type IV, V, VII and elastin; Stromelysin that is active against proteoglycans and elastin (Rouis M). These three subgroups are of particular interest with regards to matrix remodelling in atherosclerotic plaques.

### GELATINASES

Insoluble elastin is digested by MMP-2 and -9, both belonging to the gelatinase-family of MMPs. MMP-9 has an important role affecting the size and composition of atherosclerotic plaque. In unstable human atherosclerotic plaques and in vulnerable regions of plaques, greater expression and concentration of MMP-9 have been observed. Moreover, MMP-9 is found intracellularly (indicating active synthesis) in coronary plaques more often in patients with unstable angina compared with those with stable angina. Blood MMP-9 level increases in association with coronary atherosclerosis and predicts adverse cardiovascular events (Sundstrom and Vasan). A recent study by Kuzuya et al (2006) indicates that MMP-2 is responsible for accumulation of SMC in the fibrous cap and thereby inducing plaque instability.

### STROMELYSIN

MMP-3 belongs to the stromelysin proteases and is capable of degrading both elastin and proteoglycans. A study by Yamada et al (2002) indicates that MMP-3 may prove to be a reliable mean of predicting the genetic risk of myocardial infarction in women.

### COLLAGENASES

MMP-1, -8 and -13 have all been identified in atherosclerotic plaques where they degrade proteoglycans and collagen types I and III.

MMP-1, -8 and -13 are collagenases, which cleave collagen into two fragments that are further degraded by MMP-2, -3 or -9.

MMP-8 is expressed by neutrophils, not commonly found in human atheroma but has been identified in atherosclerotic plaques. MMP-8 may be partly responsible for degradation of the fibrous cap as MMP-8 has a preference for collagen type I (Herman et al), having a three fold greater activity in degradation of collagen I than MMP-1 and 13. This is supported by Turu et al (2006), in this study the content of MMP-8 in the plasma are significantly higher for patients with vulnerable plaques, than patients with stable plaques.

MMP-13 has been reported to cleave SLRPS, with high specificity for biglycan. Degradation of biglycan by MMP-13 at a specific cleavage site (...G₁₇₇/V₁₇₈) has previously been demonstrated by Monfort et al. (2005) and proposed to play a important role in early detection of cartilage degradation in osteoarthritis.)

### CATHEPSINS

Human cysteine cathepsins consist of 11 members, including cathepsins B, K, L, and S, and are predominantly expressed within the endosomal/lysosomal compartments of cells. Cathepsins are capable of catalysing the hydrolytic breakdown of proteoglycans, collagen and elastin.

In abdominal aortic aneurysm (AAA) high levels of cathepsins S, K, and L were found compared to normal aorta. Normal human vascular SMC contain no detectable cathepsin K by immunostaining, but cells within atherosclerotic plaques are clearly positive. Cathepsin K is localized in rupture-prone areas such as the fibrous cap, plaque shoulders and at the actual site of plaque ruptures (Chapman et al). Cathepsin S is found to co-localize with regions of increased elastin breakdown in atherosclerotic plaques, and reduced atherosclerosis is observed in cathepsin S- and K-deficient mice (Liu et al).

Both cathepsin L and K degrade several proteoglycans and collagen type I and II, cathepsin K degrades within covalently cross-linked triple helices, while cathepsin L cleaves only in the nonhelical telopeptide regions. Cathepsin K is localized in the fibrous cap and plaque shoulder. Cathepsin K expression in normal arteries is very low. Early human atherosclerotic lesions showed cathepsin K expression in the intimal and medial SMCs. In advanced atherosclerotic plaques, cathepsin K was localized mainly in macrophages and SMCs of the fibrous cap (Lutgens et al). Cathepsin K protein levels were increased in atherosclerotic lesions when compared with normal arteries, whereas cathepsin K mRNA levels were similar in both atherosclerotic and normal arteries. Furthermore, it was shown that cathepsin K mRNA and protein levels were highest in advanced but stable human atherosclerotic plaques compared with early atherosclerotic lesions and lesions containing thrombus (Chapman et al).

Cathepsin S is only sparsely expressed in intimal and medial SMCs in early human atherosclerotic lesion and fatty streaks. In advanced human atherosclerotic plaques cathepsin S was localized in macrophages and SMCs of the fibrous cap. EC lining the lumen of the vessel itself and the plaque microvessels also expressed cathepsin S. Furthermore, cathepsin S mRNA and protein levels were increased in human atheroma compared with normal arteries (Lutgens et al). Cathepsin S can degrade proteoglycans, elastin and collagen (Liu et al).

Presently, the determination of CVD risk is occurring at a late stage in atherosclerosis progression; a point in which there is a significant risk of fibrous plaque rupture. There is a need for diagnostic or prognostic assays that will provide information regarding atherosclerosis or CVD risk at both earlier stage and late stages. The findings of Katsuda et al (1992) suggest that there are enzymatic mechanisms for removal of collagens from advanced lesions, suggesting indeed a major role of neo-epitopes in arteriosclerosis.

The present disclosure provides a method of bioassay for the quantification of peptide fragments comprising a neo-epitope formed by cleavage of a protein of an atherosclerotic plaque by a proteinase, said method comprising contacting a sample comprising said peptide fragments with an immunological binding partner having specific binding affinity for a said neo-epitope and determining the level of binding of said immunological binding partner to peptide fragments in said sample.

The result of said assay may produce an index indicative of the degree of risk in a particular patient of rupture of an atherosclerotic plaque or of the vulnerable status of the atherosclerotic plaques of a patient.

Patients having a value for said index above a threshold level may be recommended for further investigation by plaque imaging methods (including those discussed above) or for the prescribing of medication for treatment of atherosclerosis or for surgical treatment of atherosclerosis, and such follow up investigations or treatment may form part of the method of the invention.

Proteins of the atherosclerotic plaque include lumican, versican, perlecan, decorin, biglycan, collagen type III, CRP, ApoE and elastin. Collagen type I is not considered to be proteins of the atherosclerotic plaque. Proteins present in the atherosclerotic plaque which are exposed there to proteases to a higher degree than elsewhere in the body are of particular interest.

Said immunological binding partner may have specific binding affinity for peptide fragments comprising a C-terminal neo-epitope or an N-terminal neo-epitope.

### CRP AND ApoE ASSAYS

Peptide assays for peptide fragments of CRP (SEQ ID NO 658) or ApoE (SEQ ID NO 659) are described herein. For ApoE, preferably the chosen fragments occur in all of the identified isotypes of ApoE, ε2, ε3 and ε4.

Even though both CRP and ApoE are abundant throughout the human body, their localization in the atherosclerotic tissue exposes them to the action of local proteases. These molecules are thereby good and specific candidates as biochemical markers.

Several candidate proteases may be responsible for the digestion of CRP and ApoE in the plaque as the literature reports many different proteases in the atherosclerotic plaque. Most likely, this is the result of the large range of complicated processes eventually leading to plaque rupture. However, early phases may consist of a range of MMPs, whereas later stages may rely more on cathepsin K degradation of the matrix, resulting in different neo-epitope profiles dependent on the levels of disease. We have through a range of in vitro cleavages of pure native proteins determined that the enzymes listed in the following table cleave CRP and ApoE at at least following cleavage sites (marked *) :

**Table 13. CRP and APOE fragments generated by specific proteases.**

| Protease/Protein | Neo-epitope | SEQ ID NO |
|---|---|---|
| APOE + MMP3 | A*KVEQAVETEPEPELR*Q | 660 |
| APOE + MMP9 | A*KVEQAVETEPEPELR*Q | 661 |
| APOE + MMP1 | V*AEVRAKLEEQAQQI*R | 662 |
| APOE + MMP3 | A*KVEQAVETEPEPELR*Q | 663 |
| APOE + MMP3 | A*MLGQSTEELRV*R (M-oxidized) | 664 |
| APOE + ADAMTS1 | E*QAVETEPEPELR*Q | 665 |
| APOE + ADAMTS1 | R*QQTEWQSGQRWE*L | 666 |
| APOE + ADAMTS1 | L*AVYQAGAREGAERGLS*A | 667 |
| APOE + ADAMTS1 | R*AKLEEQAQQIR*L | 668 |
| APOE + ADAMTS1 | A*KLEEQAQQIRLQ*A | 669 |
| APOE + CathepsinK | A*KVEQAVETEPEPELR*Q | 670 |
| APOE + CathepsinK | K*VEQAVETEPEPELR*Q | 671 |
| APOE + CathepsinK | E*QAVETEPEPELR*Q | 672 |
| APOE + CathepsinK | D*EVKEQVAEVRAKLE*E | 673 |
| Protease/Protein | Neo-epitope | |
| CRP + CatK | K*ESDTSYVSLKAPLT*K | 674 |
| CRP + CatK | G*GNFEGSQSLVGDIG*N | 675 |
| CRP + MMP9 | A*LKYEVQGEVFTKPQ*L | 676 |
| CRP + MMP9 | G*IVEFWVDGKPRV*R | 677 |
| CRP + MMP1/MMP3 | R*KAFVFPKE*S | 678 |
| CRP + MMP3 | K*YEVQGEVFTKPQLWP*- | 679 |
| CRP + MMP3 | D*SFGGNFEGSQS*L | 680 |
| CRP + MMP3 | D*FVLSPDEINT*I | 681 |
| CRP + MMP3 | S*LKKGYTVGAEA*S | 682 |
| CRP + MMP3 | A*FGQTDMSRKA*F | 683 |
| CRP + MMP3 | S*LKKGYTVGAEAS*I | 684 |
| CRP + MMP3 | G*EVFTKPQLWP*- | 685 |
| CRP + MMP3 | S*IILGQEQDSFGGN*F | 686 |
| CRP + MMP3 | K*YEVQGEVFTKPQ*L | 687 |

Accordingly, in a method described herein, said peptide fragments preferably comprise a neo-epitope formed by cleavage of CRP and ApoE by a protease at a site marked by the sign * in any one of the following partial sequences of CRP and APOE, or the immunological binding partner is specifically reactive with a sequence defined between the *s in one of the following sequences:

**Table 14. Cleavage fragments of CRP and APOE.**

| ApoE fragments |
|---|
| A*KVEQAVETEPEPELR*Q |
| A*KVEQAVETEPEPELR*Q |
| V*AEVRAKLEEQAQQI*R |
| A*KVEQAVETEPEPELR*Q |
| A*MLGQSTEELRV*R (M-oxidized) |
| E*QAVETEPEPELR*Q |
| R*QQTEWQSGQRWE*L |
| L*AVYQAGAREGAERGLS*A |
| R*AKLEEQAQQIR*L |
| A*KLEEQAQQIRLQ*A |
| A*KVEQAVETEPEPELR*Q |
| K*VEQAVETEPEPELR*Q |
| E*QAVETEPEPELR*Q |
| D*EVKEQVAEVRAKLE*E |
| |

| CRP fragments |
|---|
| K*ESDTSYVSLKAPLT*K |
| G*GNFEGSQSLVGDIG*N |
| A*LKYEVQGEVFTKPQ*L |
| G*IVEFWVDGKPRV*R |
| R*KAFVFPKE*S |
| K*YEVQGEVFTKPQLWP*- |
| D*SFGGNFEGSQS*L |
| D*FVLSPDEINT*I |
| S*LKKGYTVGAEA*S |
| A*FGQTDMSRKA*F |
| S*LKKGYTVGAEAS*I |
| G*EVFTKPQLWP*- |
| S*IILGQEQDSFGGN*F |
| K*YEVQGEVFTKPQ*L |

Suitable immunological binding partners described herein may therefore be specifically reactive with any of the following sequences at the N terminal of a peptide:

| APOE | SEQ ID NO | CRP | SEQ ID NO |
|---|---|---|---|
| KVEQAV | 688 | AFVFPK | 699 |
| AEVRAK | 689 | YEVQGE | 700 |
| MLGQST | 690 | KAFVFP | 701 |
| QAVETE | 691 | SFGGNF | 702 |
| QQTEWQ | 692 | FVLSPD | 703 |
| AVYQAG | 693 | LKKGYT | 704 |
| AKLEEQ | 694 | FGQTDM | 705 |
| KLEEQA | 695 | LKKGYT | 706 |
| VEQAVE | 696 | IILGQE | 707 |
| QAVETE | 697 | YEVQGE | 708 |
| EVKEQV | 698 | LKYEVQ | 709 |
| | | IVEFWV | 710 |
| | | ESDTSY | 711 |
| | | GNFEGS | 712 |

or with any of the following sequences at the C-terminal of a peptide:

| APOE | | CRP | |
|---|---|---|---|
| TEPEPE | 714 | KAFVFPK | 725 |
| EQAQQI | 715 | AFVFPK | 726 |
| TEELRV | 716 | KPQLWP | 727 |
| PEPELR | 717 | FVFPKE | 728 |
| SGQRWE | 718 | PDEINT | 729 |
| EGAERG | 719 | DMSRKA | 730 |
| QAQQIR | 720 | VGAEAS | 731 |
| QQIRLQ | 721 | KPQLWP | 732 |
| EPEPEL | 722 | DSFGGN | 733 |
| PEPELR | 723 | VFTKPQ | 734 |
| EVRAKL | 724 | | |

In the method of the invention the monoclonal antibody has specific binding affinity for the N-terminal neo-epitope sequence KAFVFP.

Further cleavage sites defining neo-epitopes that may be assayed in a similar manner can be identified by exposing CRP and AppoE or another atherosclerotic plaque protein to any of the enzymes described herein and isolating and sequencing peptides thereby produced.

The result of an assay according to the invention may be combined with one or more other measured biomarkers to form a composite index of diagnostic or prognostic value.

The term `immunological binding partner' as used herein includes polyclonal and monoclonal antibodies and also specific binding fragments of antibodies such as Fab or F(ab')₂. Thus, said immunological binding partner may be a monoclonal antibody or a fragment of a monoclonal antibody having specific binding affinity.

The term 'protein' used herein includes lipoproteins and proteoglycans and other protein-(non-protein) naturally occurring conjugates.

Generally, all previously known immunoassay formats can be used in accordance with this invention including heterogeneous and homogeneous formats, sandwich assays, competition assays, enzyme linked assays, radio-immune assays and the like. Thus, optionally, said method is conducted as a competition immunoassay in which said immunological binding partner and a competition agent are incubated in the presence of said sample and the competition agent competes with the peptide fragments in the sample to bind to the immunological binding partner.

Said competition agent may be a synthetic peptide or a purified native peptide formed by cleavage of the protein to which the neo-epitope belongs to reveal said neo-epitope. Thus, the peptide may be derived from CRP.

One suitable method could be a competition immunoassay using monoclonal antibodies or antibody binding fragments binding to neo-epitopes of fragments of any of these proteins or neo-epitopes on peptide fragments from other proteins derived from atherosclerotic plaques. Appropriately selected synthetic peptides coated onto the solid surface of a microtitre plate could compete with the sample for binding to the monoclonal antibodies or binding fragments. Alternatively, purified, native fragments from one or more of these proteins carrying the neo-epitope recognised by the monoclonal antibody or binding fragment could be used on the solid surface. Yet another alternative is to immobilise the monoclonal antibody or binding fragment on the solid surface and then co-incubate the sample with a synthetic peptide appropriately linked to a signal molecule, e.g. horseradish peroxidase or biotin. The sample may be a sample of urine, serum, blood, plasma or other, e.g. atherosclerotic plaque biopsy.

In certain preferred methods, the sample is a patient derived sample, and the method further comprises comparing the determined level of said binding of said peptide fragments with values characteristic of (a) comparable healthy individuals and/or (b) a pathological atherosclerotic condition and optionally associating a higher level of the measured peptide (normally indicated by a higher level of binding) with a more severe degree of a said condition.

An aspect of the present invention relates to the development of monoclonal antibodies recognising neo-epitopes as described above. This can be achieved by immunising mice with synthetic peptides originating from the amino acid sequence of the protein molecule concerned (including the sequences listed above or sequences terminating therein), fusing the spleen-cells from selected mice to myeloma cells, and testing the monoclonal antibodies for binding to neo-epitopes on relevant synthetic peptides. Specificity for neo-epitopes can be ensured by requiring reactivity with a synthetic peptide and a lack of reactivity with either a C-prolongated form of the immunising peptide (for a C-terminal neo-epitope) or an N-terminal prolongated form of the immunising peptide (for an N-terminal neo-epitope). Antibodies for neo-epitopes may also be evaluated to establish a lack of binding capacity to native protein. Alternatively, specificity for a neo-epitope can be ensured by requiring the reactivity of the antibody to be negatively dependent on the presence of biotin or other functional groups covalently linked to one of the terminal amino acids.

The disclosure describes an immunological binding partner which is specifically immunoreactive with a neo-epitope formed by cleavage of a said protein by a protease at an end-site in any one of the partial sequences set out above, and may be for instance a monoclonal antibody or a binding fragment thereof.

The disclosure describes a cell line producing a monoclonal antibody against a C-terminal or N-terminal neo-epitope formed by cleavage of an atherosclerotic plaque protein at the end-sites of sequences in any one of the partial sequences o set out above.

The disclosure further describes a peptide comprising a C-terminal or N-terminal neo-epitope formed by cleavage of a said protein in any one of the partial sequences of these proteins set out above. Such a peptide may be conjugated as a hapten to a carrier for producing an immune response to said peptide, or immobilised to a solid surface or conjugated to a detectable marker for use in an immunoassay.

The disclosure further describes an isolated nucleic acid molecule coding for a peptide comprising a C-terminal or N-terminal neo-epitope formed by cleavage of a said protein in any one of the partial sequences set out above.

The disclosure further describes a vector comprising a nucleic acid sequence comprising an expression signal and a coding sequence which codes for the expression of a peptide comprising a C-terminal or N-terminal neo-epitope formed by cleavage of a said protein in any one of the partial sequences set out above and further includes a host cell transformed with such a vector and expressing a said peptide.

Yet another aspect of the disclosure relates to kits, which can be used conveniently for carrying out the methods described above. Such kits may include (1) a microtitre plate coated with synthetic peptide; (2) a monoclonal antibody or antibody binding fragment of the invention reactive with said synthetic peptide; and (3) a labelled anti-mouse IgG immunoglobulin. Alternatively, such kits may include (1) a microtitre plate coated with purified native protein fragments; (2) a monoclonal antibody recognising a neo-epitope on fragments of any one of said proteins, and reactive with said purified fragments; and (3) a labelled anti-mouse IgG immunoglobulin. Alternatively, such kits may include (1) a microtitre plate coated with streptavidin; (2) a synthetic peptide linked to biotin; (3) a monoclonal antibody recognising a neo-epitope on said protein fragments and reactive with said synthetic peptide; and (4) a labelled anti-mouse IgG immunoglobulin. Yet another alternative could be kits including (1) a microtitre plate coated with streptavidin; (2) a synthetic peptide linked to biotin; (3) a monoclonal antibody recognising a neo-epitope on said protein fragments (and reactive with said synthetic peptide) and conjugated to horseradish peroxidase.

Thus, the invention includes an immunoassay kit comprising a monoclonal antibody being specifically immunoreactive with the N-terminal neo-epitope sequence KAFVFP, and a competition agent which binds said monoclonal antibody, and optionally one or more of a wash reagent, a buffer, a stopping reagent, an enzyme label, an enzyme label substrate, calibration standards, an anti-mouse antibody and instructions for conducting a said immunoassay.

The assays described herein are useful in the diagnosis of atherosclerotic disease in patients. In addition, the tests are useful for the assessment of disease progression, and the monitoring of response to therapy. The immunological binding partners of the invention may also be used in immunostaining to show the presence or location of cleavage products of any atherosclerotic plaque protein described herein.

The disclosure will be further explained and illustrated with reference to the accompanying drawings, in which:
Figure 1 shows Biglycan staining (magnifications 2, 4, 4 and 10 x respectively) using a monoclonal mouse antibody on an aortic sample with type III lesion.
Figure 2 shows Cathepsin K staining (magnifications 2, 4, 10 and 10 x respectively) using a monoclonal mouse antibody on an aortic sample with type III lesion.
Figure 3 shows Biglycan staining (magnifications 2, 4, 10 and 10 x respectively) using a monoclonal mouse antibody on an aortic sample with type V lesion.
Figure 4 shows Cathepsin K staining (magnifications 2, 4, 10 and 10 x respectively) using a monoclonal mouse antibody on an aortic sample containing type V lesion.
Figure 5 shows cleavage products of biglycan generated by proteases: MMP2, MMP3, MMP8, cathepsin K, cathepsin S, cathepsin B, and cathepsin L. M=Rainbow marker. -enz = no enzyme digestion, run on a gel in Example 2.
Figures 6 to 8 show competition study results obtained in Example 4.
Figure 9 shows competition study results obtained in Example 6.
Figures 10 and 11 show competition study results obtained in Example 7.

### Reference Example 1.

For analysis of localization of proteoglycans and proteases we performed immunohistochemical stainings of human arterial samples derived from left coronary descending arteries (LAD).
In the following, co-localization of Cathepsin K protease and biglycan is demonstrated.

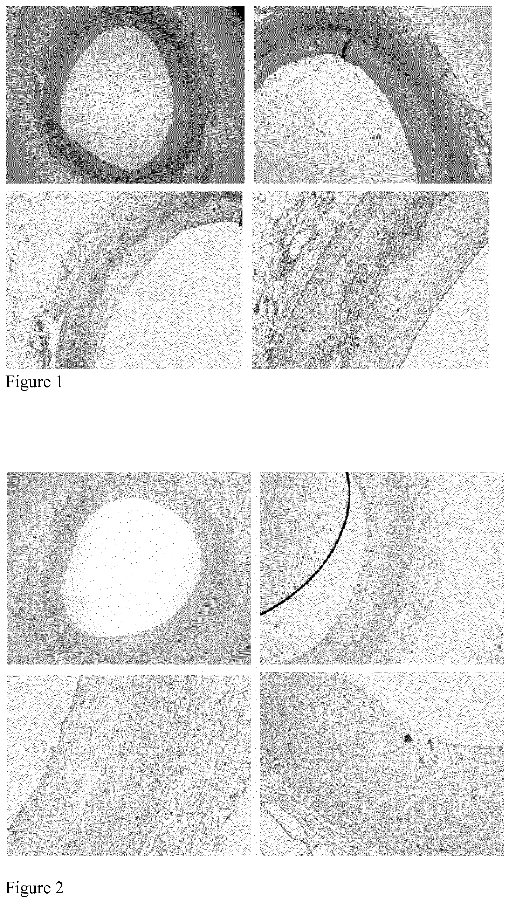

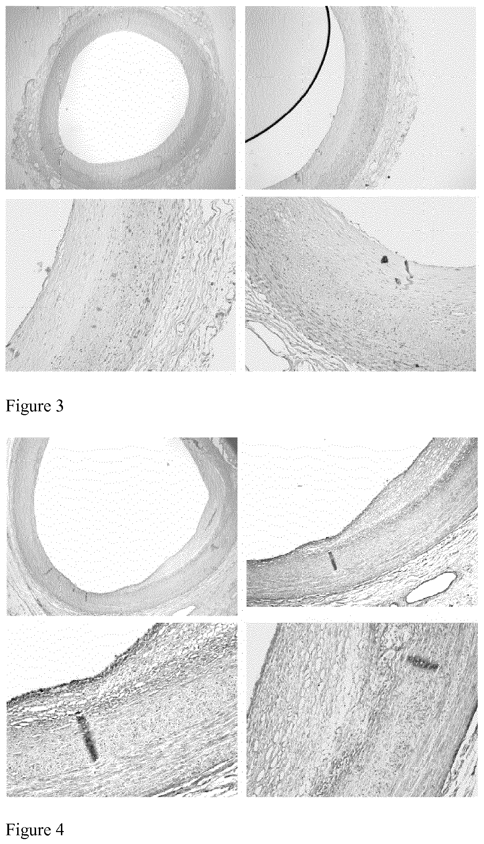

Immunohistochemical staining as seen in Figures 1 and 2 revealed a co-localization of biglycan and cathepsin K. This may suggest that biglycan is a preferred substrate of cathepsin K.
The same immunohistochemical staining was performed on the aortic samples, where the atherosclerotic plaque was formed and as a result of this normal aortic architecture was replaced by macrophage foam cell infiltrates and calcifications. The results of these immunostainings are collected in Figures 3 and 4.

Immunohistochemical staining of biglycan and cathepsin K were shown to co-localize in a progressed atherosclerotic lesion. These results together generate hypothesis of specific cathepsin K cleavage sites in biglycan, resulting in increased neo-epitope generation in atherosclerotic lesions. To test this hypothesis, we cleaved biglycan with different proteases.

### Reference Example 2.

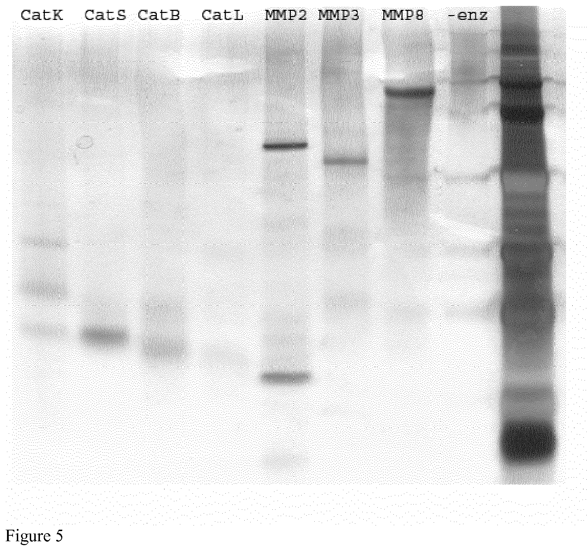

Degradation of biglycan for assessment of degradation fragments. Biglycan from bovine articular cartilage (B8041 - Sigma-Aldrich) was cleaved by following proteases: MMP2, MMP3, MMP8, Cathepsin K, Cathepsin S, Cathepsin B, and Cathepsin L. Fragments of proteoglycans generated by enzymatic cleavage of above mentioned proteases were separated on 10% NuPage® Bis-Tris gels and afterwards silver-stained by "Silver Express" - silver staining kit (Invitrogen cat.nr. LC6100, lot.nr.341099). Results of separation of proteolytically derived and biglycan and silver-stainings are represented by Figure 5.

### Reference Example 3

Mice were immunised with collagen type III derived peptides conjugated with ovalbumin. Sera were screened for reactivity with screening peptide sequences conjugated to biotin. Monoclonal antibody secreting clones were produced and screened using the screening sequences. Clones were checked for lack of reactivity with elongated versions of the target peptide which are continued with the adjacent sequences from collagen type III (deselection peptide) and for lack of reactivity with a nonsense peptide. None of the clones positive for the target sequences reacted with either the elongated or the nonsense sequences.

The target sequences, immunogens, screening sequences and deselection sequences were as follows:

| No: | Target sequence | Immunogen | Screening Sequence | De-selection sequence | Mouse No. |
|---|---|---|---|---|---|
| NB51 | KNGETG 356 | | KNGETGPQGP-PG-K-Biotin | KDGETGAAGPPGK-Biotin | 278; 279; |
| | | | | KDGEAGAQGPPGK-Biotin | 289; 345; |
| | | | | PGKNGETPGPQ-GP-K-Biotin | 346; 347 |
| NB26 | IAGITG 429 | | IAGITGARGL-AG-K-Biotin | LGIAGITGARGL-AG-K-Biotin | 146; 147; |
| | | | | | 148; 149; |
| | | | IAGLTGARGL-AG-K-Biotin) | | 156; 157; |
| NB52 | IAGITG | | IAGITGARGL-AG-K-Biotin | LGIAGITGARGL-AG-K-Biotin | 348; 349; |
| | | | | | 357; 358; |
| | | | IAGLTGARGL-AG-K-Biotin | | 359; |
| NB27 | KDGTSG 763 | | KDGTSGHPGP-IG-K-Biotin | PGKDGTSGHP-GP-K-Biotin | 158; 159; |
| | | | | | 167; 168; |
| | | | KDGSSGHPGP-IG-K-Biotin | | 169; 178; |
| NB67 | APGPLG | | Biotin-AQ-GPPGAPGPLG | Biotin-DD-GPSGAEGPPG | 167; 168; |
| | | | | | 169; |
| | | | Biotin-AQ-GPPGSPGPLG | Biotin-GP-PGAPGPLGIA | 178; |
| | 581 | | | | 179; |
| | | | | | 189; |
| NB68 | NTGAPG | | NTGAPGSPGVSG-K-Biotin | AGNTGAPGSP-GV-Biotin | 234; 235; |
| | | | | | 236; 237; |
| | 422 | | NSGSPGNPGVAG-K-Biotin | | 238; 239; |
| NB69 | AIGPSG | | AIGPSGPAGKDG-K-Biotin | PGAIGPSGPAG-KD - Biotin | 245; 246; |
| | | | | | 247; 248; |
| | 380 | | AIGPAGPAGKDG-K-Biotin | | 249; 256; |
| NB57 | AGGFAP | | Biotin-CG-EKAGGFAP | Biotin-GG-EKAGGFAPYY | 1; |
| | | | | | 2; |
| | 781 | | Biotin-CG-EKSGGFSP | | 3; |
| | | | | | 4; |
| | | | | | 5; |
| | | | | | 6; |

### Reference Example 4

### Reactivity of collagen type III neo-epitope monoclonal antibodies with human urine

The reactivity of selected monoclonal antibody clones from example 3 with human urine was determined in a competition assay format using the immunising peptides as competition agent. In a typical procedure, 96 well streptavidin coated plates were coated for 30 min with 10 ng/mL Biotin-peptide in PBS-BTE at 20° C with shaking and washed 5x in washing buffer. 20 of diluted sample was added (either urine or peptide solution). 100 µL of unpurified antibody solution (supernatant from cell culture) diluted as detailed below was added. The plates were incubated for 1 hr at 20°C with shaking at 300 rpm and were then washed 5x in washing buffer. 100 µL secondary antibody-POD (1:5000) was added and incubated for 1hr at 20°C with shaking at 300 rpm before washing 5x in washing buffer. 100 µL TMB was added and incubated for 15 min in darkness shaking at 300 rpm before adding 100 µL stopping solution. The plates were read at 450 nm on an ELISA reader with 650 nm as reference. Competition therefore occurred between the peptide on the plate and peptide in solution for the antibody and the amount of plate bound antibody was determined by the peroxidase colour forming reaction.

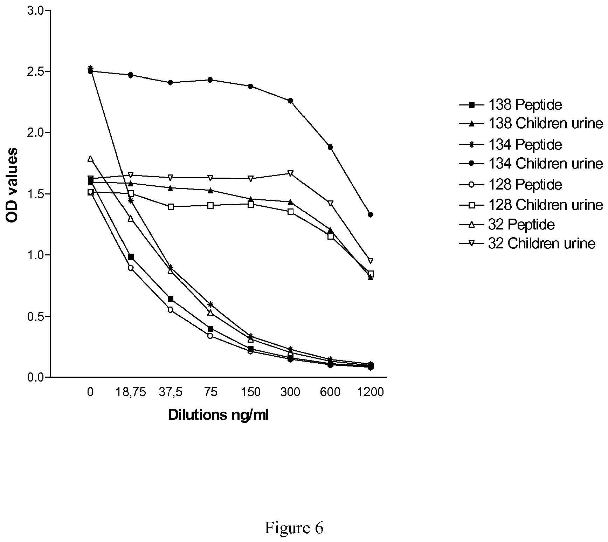

Results are seen in Figure 6 for four different clones. It can be seen that the antibodies each detect relevant sequences in urine.

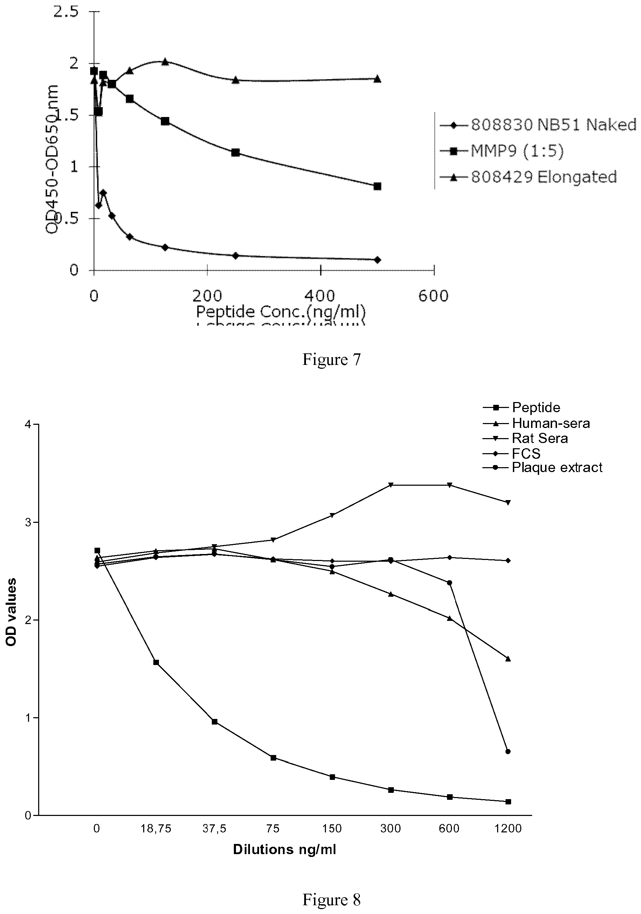

Further competition studies were performed on one selected clone to test competition for antibody binding between the immunising peptide and native collagen type III cleaved in vitro by MMP9. Results are shown in Figure 7 for the cleaved collagen, the peptide KNGETG and an elongated version of that sequence. It can be seen that the antibody binds the immunising peptide sequence and the enzyme cleaved collagen, but not the extended sequence.

Further competition studies on the same clone are seen in Figure 8 where the competition agents were the peptide KNGETG, human serum, rat serum, FCS (fetal calf serum), and atherosclerotic plaque extracts respectively. It is seen that the antibody is reactive with the peptide, the plaque extract and human serum, but not the rat serum or FCS.

### Reference Example 5

### Raising of anti-sera to decorin, biglycan and versican sequences

Anti-sera were raised and monoclonal antibodies were obtained as in Example 3, but using the following immunogens, screening sequences and deselection sequences:

| *No:* | *Epitope* | *Target sequence* | *Immunogen* | *Screening Sequence* | *De-selection sequence* | *Mouse No.* |
|---|---|---|---|---|---|---|
| *NB62* | ***Decorin-176N*** | ***IVIELG*** | ***IVIELGTNPL-GGC-KLH*** | ***IVIELGTNPL-KS-K-Biotin*** | ***QMIVIELGTNPLK-K-Biotin*** | **7;8;9; *10;12; 13*** |
| | | ***91*** | | ***LVIELGGNPL-KN-K-Biotin*** | ***NVLVIELGGNPL-K-Biotin*** | |
| | | | | ***IVVELGGNPL-TN-K-Biotin*** | | |
| *NB63* | ***Biglyean-108C*** | ***NNDISE*** | ***OVA-CGG-LDLQNNDISE*** | ***Biotin**-**TL-LDLQNNDISE*** | ***Biotin-LDLQNNDISELR*** | ***14;15; 16;17; 18;19*** |
| | | ***116*** | | | | |
| *NB64* | ***Versican-87N*** | ***QNGNIK*** | ***QNGNIKIGQD-GGC-KLH*** | ***QNGNIKIGQD-YK-Biotin*** | ***VAQNGNIKIGQD-K-Biotin*** | ***23;24 25;26; 27;28;*** |
| | | ***143*** | | ***QDGNIKIGQD**-**YK-Biotin*** | ***VAQDGNIKIGQD-K-Biotin*** | |

### Reference Example 6

### Reactivity of decorin neo-epitope monoclonal antibody with human urine

A competition ELISA was carried out generally as in Example 5 using one anti-decorin unpurified monoclonal antibody (NB62)

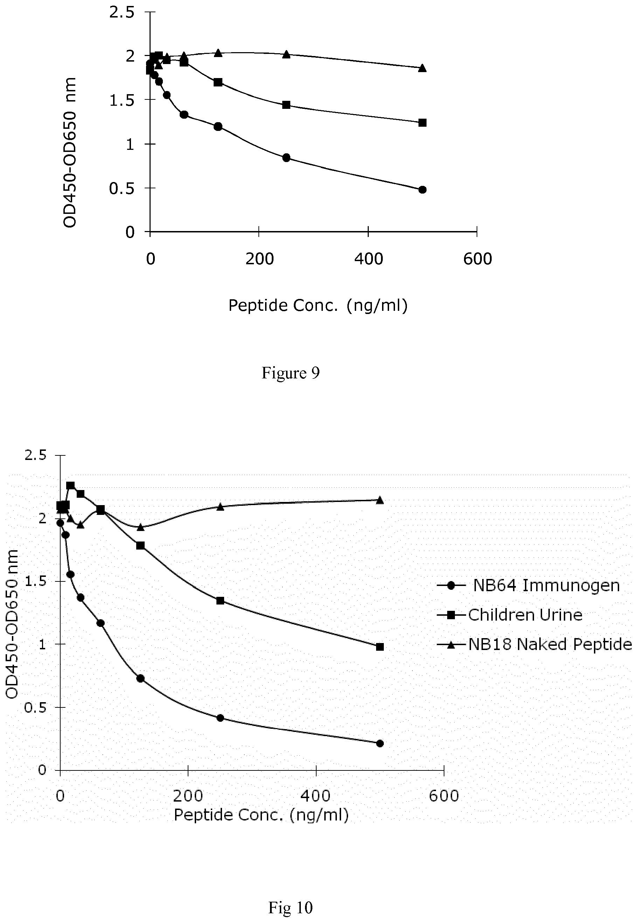

Results are shown in Figure 9. Reactivity is seen against the peptide sequence against which the antibody was raised and selected and against urine, but not against the irrelevant peptide sequence NB18.

### Reference Example 7

### Reactivity of versican neo-epitope monoclonal antibody with human urine

A competition ELISA was carried out generally as in Example 5 using two anti-versican unpurified monoclonal antibody clones raised against sequence (NB64).

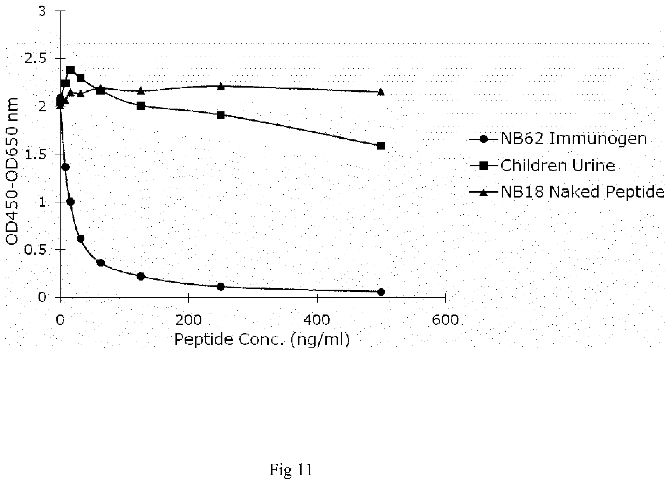

Results are shown in Figures 10 and 11 for the respective clones. In each case reactivity is seen against the peptide sequence against which the antibody was raised and selected and against urine, but not against the irrelevant peptide sequence NB18.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### Reference List

Bobryshev YV. Calcification of elastic fibers in human atherosclerotic plaque. Atherosclerosis 2005;180:293-303.
Brown, D. C. and K. G. Vogel. "Characteristics of the in vitro interaction of a small proteoglycan (PG II) of bovine tendon with type I collagen." Matrix. 9.6 (1989): 468-78.
Cattin L, Fisicaro M, Tonizzo M, Valenti M, Danek GM, Fonda M, Da Col PG, Casagrande S, Pincetri E, Bovenzi M, and Baralle F. Polymorphism of the apolipoprotein E gene and early carotid atherosclerosis defined by ultrasonography in asymptomatic adults. Arterioscler Thromb Vasc Biol. 1997 Jan;17(1):91-4.
Chapman HA, Riese RJ, Shi GP. Emerging roles for cysteine proteases in human biology. Annu.Rev.Physiol 1997;59:63-88.
Clarkson TB, Kaplan JR. Stage of Reproductive Life, Atherosclerosis Progression and Estrogen Effects on Coronary Artery Atherosclerosis, In: Lobo RA, editor. Treatment of the Postmenopausal Woman: Basic and Clinical Aspects, 3 ed. San Diego: Elsevier; 2007. p. 509-28.
Danielson, K. G., et al. "Targeted disruption of decorin leads to abnormal collagen fibril morphology and skin fragility." J.Cell Biol. 136.3 (1997): 729-43.
Dours-Zimmermann, M. T. and D. R. Zimmermann. "A novel glycosaminoglycan attachment domain identified in two alternative splice variants of human versican." J.Biol.Chem. 269.52 (1994): 32992-98.
Eriksen HA, Satta J, Risteli J, Veijola M, Vare P, Soini Y. Type I and type III collagen synthesis and composition in the valve matrix in aortic valve stenosis. Atherosclerosis 2006;189:91-98.
Evanko, S. P., et al. "Proteoglycan distribution in lesions of atherosclerosis depends on lesion severity, structural characteristics, and the proximity of platelet-derived growth factor and transforming growth factor-beta." Am.J.Pathol. 152.2 (1998): 533-46.
Funderburgh, J. L. "Keratan sulfate: structure, biosynthesis, and function." Glycobiology 10.10 (2000): 951-58.
Funderburgh, J. L., et al. "Macrophage receptors for lumican. A corneal keratan sulfate proteoglycan." Invest Ophthalmol.Vis.Sci. 38.6 (1997): 1159-67.
Gabay C and Kushner I. Acute-phase proteins and other systemic responses to inflammation. N Engl J Med. 1999 Feb 11;340(6):448-54.
Gardner CD, Fortmann SP, Krauss RM. Association of small low-density lipoprotein particles with the incidence of coronary artery disease in men and women. JAMA 1996;276:875-81.
Garrone R, Lethias C, Le Guellec D. Distribution of minor collagens during skin development. Microsc.Res Tech. 1997;38:407-12.
Graham I, Atar D, Borch-Johnsen K, Boysen G, Burell G, Cifkova R et al. European guidelines on cardiovascular disease prevention in clinical practice: executive summary. Atherosclerosis 2007;194:1-45.
Haraki T, Takegoshi T, Kitoh C, Wakasugi T, Saga T, Hirai JI, Aoyama T, Inazu A and Mabuchi H, Carotid artery intima-media thickness and brachial artery flow-mediated vasodilation in asymptomatic Japanese male subjects amongst apolipoprotein E phenotypes. J Intern Med. 2002 Aug;252(2):114-20.
Hatanaka K, Li XA, Masuda K, Yutani C and Yamamoto A, Immunohistochemical localization of C-reactive protein-binding sites in human atherosclerotic aortic lesions by a modified streptavidin-biotin-staining method. Pathol Int. 1995 Sep;45(9):635-41.
Heegaard AM, Corsi A, Danielsen CC, Nielsen KL, Jorgensen HL, Riminucci M, Young MF and Bianco P, Biglycan deficiency causes spontaneous aortic dissection and rupture in mice. Circulation. 2007 May 29;115(21):2731-8. Epub 2007 May 14.
Herman MP, Sukhova GK, Libby P, Gerdes N, Tang N, Horton DB et al. Expression of neutrophil collagenase (matrix metalloproteinase-8) in human atheroma: a novel collagenolytic pathway suggested by transcriptional profiling. Circulation 2001;104:1899-904.
Jeppesen J, Hein HO, Suadicani P, Gyntelberg F. High triglycerides/low high-density lipoprotein cholesterol, ischemic electrocardiogram changes, and risk of ischemic heart disease. Am Heart J 2003;145:103-08.
Lawrie TD, Mcalpine SG, Rifkind BM, Robinson JF. Serum fatty-acid patterns in coronary-artery disease. Lancet 1961;1:421-24.
Katsuda S, Okada Y, Minamoto T, Oda Y, Matsui Y, Nakanishi I. Collagens in human atherosclerosis. Immunohistochemical analysis using collagen type-specific antibodies. Arterioscler.Thromb. 1992;12:494-502.
Knox, S. M. and J. M. Whitelock. "Perlecan: how does one molecule do so many things?" Cell Mol.Life Sci. 63.21 (2006): 2435-45.
Kuller LH, Tracy RP, Shaten J and Meilahn EN, Relation of C-reactive protein and coronary heart disease in the MRFIT nested case-control study. Multiple Risk Factor Intervention Trial. Am J Epidemiol. 1996 Sep 15;144(6):537-47.
Kunz J. Matrix metalloproteinases and atherogenesis in dependence of age. Gerontology. 2007;53:63-73.
Kuzuya M, Nakamura K, Sasaki T, Cheng XW, Itohara S, Iguchi A. Effect of MMP-2 deficiency on atherosclerotic lesion formation in apoE-deficient mice. Arterioscler.Thromb.Vasc.Biol 2006;26:1120-25.
Leinonen M and Saikku P, Evidence for infectious agents in cardiovascular disease and atherosclerosis. Lancet Infect Dis. 2002 Jan;2(1):11-7.
Liu J, Sukhova GK, Sun JS, Xu WH, Libby P, Shi GP. Lysosomal cysteine proteases in atherosclerosis. Arterioscler.Thromb.Vasc.Biol 2004;24:1359-66.
Lutgens, S. P., et al. "Cathepsin cysteine proteases in cardiovascular disease." FASEB J. 21.12 (2007): 3029-41.
Mayne R. Collagenous proteins of blood vessels. Arteriosclerosis. 1986;6:585-93.
McCullagh KG, Duance VC, Bishop KA. The distribution of collagen types I, III and V (AB) in normal and atherosclerotic human aorta. J Pathol 1980;130:45-55.
Mendall MA, Patel P, Ballam L, Strachan D and Northfield TC. C reactive protein and its relation to cardiovascular risk factors: a population based cross sectional study.,BMJ. 1996 Apr 27;312(7038):1061-5.
Monfort J, Nacher M, Montell E, Vila J, Verges J and Benito P, Chondroitin sulfate and hyaluronic acid (500-730 kda) inhibit stromelysin-1 synthesis in human osteoarthritic chondrocytes. Drugs Exp Clin Res. 2005;31(2):71-6.
Pasceri V, Willerson JT and Yeh ET, Direct proinflammatory effect of C-reactive protein on human endothelial cells.Circulation. 2000 Oct 31;102(18):2165-8.
Register TC, Cann JA, Kaplan JR, Williams JK, Adams MR, Morgan TM et al. Effects of soy isoflavones and conjugated equine estrogens on inflammatory markers in atherosclerotic, ovariectomized monkeys. J Clin Endocrinol Metab 2005;90:1734-40.
Reynolds GD and Vance RP. C-reactive protein immunohistochemical localization in normal and atherosclerotic human aortas. Arch Pathol Lab Med. 1987 Mar;111(3):265-9.
Ridker PM, Intrinsic fibrinolytic capacity and systemic inflammation: novel risk factors for arterial thrombotic disease. Haemostasis. 1997;27 Suppl 1:2-11.
Ridker PM, Hennekens CH, Buring JE and Rifai N. C-reactive protein and other markers of inflammation in the prediction of cardiovascular disease in women. N Engl J Med. 2000 Mar 23;342(12):836-43.
Rodriguez-Lee M, Bondjers G and Camejo G, Fatty acid-induced atherogenic changes in extracellular matrix proteoglycans. Curr Opin Lipidol. 2007 Oct;18(5):546-53
Rouis M. Matrix metalloproteinases: a potential therapeutic target in atherosclerosis. Curr Drug Targets.Cardiovasc Haematol Disord. 2005;5:541-48.
Rudel LL, Haines J, Sawyer JK, Shah R, Wilson MS, Carr TP. Hepatic origin of cholesteryl oleate in coronary artery atherosclerosis in African green monkeys. Enrichment by dietary monounsaturated fat. J Clin Invest 1997;100:74-83.
Salisbury BG and Wagner, W DJ Biol Chem. 1981 Aug 10;256(15):8050-7,'Isolation and preliminary characterization of proteoglycans dissociatively extracted from human aorta'.
Satta J, Juvonen T, Haukipuro K, Juvonen M, Kairaluoma MI. Increased turnover of collagen in abdominal aortic aneurysms, demonstrated by measuring the concentration of the aminoterminal propeptide of type III procollagen in peripheral and aortal blood samples. J Vasc.Surg. 1995;22:155-60.
Schaar JA, Mastik F, Regar E, den Uil CA, Gijsen FJ, Wentzel JJ et al. Current diagnostic modalities for vulnerable plaque detection. Curr Pharm Des. 2007;13:995-1001.
Siest G, Pillot T, Regis-Bailly A, Leininger-Muller B, Steinmetz J, Galteau MM and Visvikis S, Apolipoprotein E: an important gene and protein to follow in laboratory medicine. Clin Chem. 1995 Aug;41(8 Pt 1):1068-86.
Shin, J., J. E. Edelberg, and M. K. Hong. "Vulnerable atherosclerotic plaque: clinical implications." Curr.Vasc.Pharmacol. 1.2 (2003): 183-204.
Shekhonin BV, Domogatsky SP, Muzykantov VR, Idelson GL, Rukosuev VS. Distribution of type I, III, IV and V collagen in normal and atherosclerotic human arterial wall: immunomorphological characteristics. Coll.Relat Res 1985;5:355-68.
Stary HC. Composition and classification of human atherosclerotic lesions. Virchows Arch A.Pathol Anat.Histopathol. 1992;421:277-90.
Sundstrom J, Vasan RS. Circulating biomarkers of extracellular matrix remodeling and risk of atherosclerotic events. Curr Opin Lipidol. 2006;17:45-53.
Talusan, P., et al. "Analysis of intimal proteoglycans in atherosclerosis-prone and atherosclerosis-resistant human arteries by mass spectrometry." Mol.Cell Proteomics. 4.9 (2005): 1350-57.
Thompson D, Banks RE, Forbes MA, Storr M, Higginson J, Raynes J, Illingworth JM, Perren TJ, Selby PJ and Whicher JT, The acute phase protein response in patients receiving subcutaneous IL-6. Clin Exp Immunol. 1995 Oct;102(1):217-23.
Terry JG, Howard G, Mercuri M, Bond MG and Crouse JR 3rd. Apolipoprotein E polymorphism is associated with segment-specific extracranial carotid artery intima-media thickening., Stroke. 1996 Oct;27(10):1755-9.
Tracy RP, Lemaitre RN, Psaty BM,Ives DG, Evans RW, Cushman M, Meilahn EN and Kuller LH, Relationship of C-reactive protein to risk of cardiovascular disease in the elderly. Results from the Cardiovascular Health Study and the Rural Health Promotion Project. Arterioscler Thromb Vasc Biol. 1997 Jun;17(6):1121-7.
Turu MM, Krupinski J, Catena E, Rosell A, Montaner J, Rubio F et al. Intraplaque MMP-8 levels are increased in asymptomatic patients with carotid plaque progression on ultrasound. Atherosclerosis 2006;187:161-69.
Venugopal SK, Devaraj S, Yuhanna I, Shaul P and Jialal I. Demonstration that C-reactive protein decreases eNOS expression and bioactivity in human aortic endothelial cells., Circulation. 2002 Sep 17;106(12) :1439-41.
Wang TJ, Gona P, Larson MG, Tofler GH, Levy D, Newton-Cheh C et al. Multiple biomarkers for the prediction of first major cardiovascular events and death. N Engl J Med 2006;355:2631-39.
Whitelock, J. M. and R. V. Iozzo. "Heparan sulfate: a complex polymer charged with biological activity." Chem.Rev. 105.7 (2005): 2745-64.
Wight, T. N. "The extracellular matrix and atherosclerosis." Curr.Opin.Lipidol. 6.5 (1995): 326-34.
Wight, T. N., et al. "Vascular cell proteoglycans: evidence for metabolic modulation." Ciba Found.Symp. 124 (1986): 241-59.
Wight TN, Versican: a versatile extracellular matrix proteoglycan in cell biology.Curr Opin Cell Biol. 2002 Oct;14(5):617-23.
Wight TN and Merrilees MJ, Proteoglycans in atherosclerosis and restenosis: key roles for versican. Circ Res. 2004 May 14;94(9):1158-67.
Wilson PW, Schaefer EJ, Larson MG and Ordovas JM. Apolipoprotein E alleles and risk of coronary disease. A meta-analysis. Arterioscler Thromb Vasc Biol. 1996 Oct;16(10):1250-5.
Yamada Y, Izawa H, Ichihara S, Takatsu F, Ishihara H, Hirayama H et al. Prediction of the risk of myocardial infarction from polymorphisms in candidate genes. N Engl J Med 2002;347:1916-23
Ying SC. et al. Localization of sequence-determined neoepitopes and neutrophil digestion fragments of C-reactive protein utilizing monoclonal antibodies and synthetic peptides. MOLECULAR IMMUNOLOGY, 1992; 29(5):677-687.
Zwaka TP, Hombach V and Torzewski J. C-reactive protein-mediated low density lipoprotein uptake by macrophages: implications for atherosclerosis., Circulation. 2001 Mar 6;103(9):1194-7.

## Claims

1. A method of bioassay for the quantification of peptide fragments of C-reactive protein (CRP) comprising an N-terminal neo-epitope amino acid sequence KAFVFP, said method comprising contacting a sample comprising said peptide fragments with a monoclonal antibody or fragment thereof having specific binding affinity for said N-terminal neo-epitope amino acid sequence KAFVFP, and determining the level of binding of said monoclonal antibody or fragment thereof to peptide fragments in said sample.

2. A method as claimed in claim 1, wherein said method is conducted as a competition immunoassay in which said monoclonal antibody or fragment thereof and a competition agent are incubated in the presence of said sample and the competition agent competes with the peptide fragments in the sample to bind to the monoclonal antibody or fragment thereof.

3. A method as claimed in claim 2, wherein said competition agent is a synthetic peptide or is a purified native peptide formed by cleavage of CRP so as to reveal said neo-epitope.

4. A method as claimed in any preceding claim, wherein the sample is a sample of urine, serum, blood, or plasma.

5. A method as claimed in any preceding claim, wherein the sample is a patient derived sample, said method further comprising comparing the determined level of said binding of said peptide fragments with values characteristic of (a) comparable healthy individuals and/or (b) a pathological atherosclerotic condition.

6. A monoclonal antibody or fragment thereof, wherein said monoclonal antibody or fragment thereof has specific binding affinity for the following sequence at the N terminal of a peptide: KAFVFP.

7. A cell line producing a monoclonal antibody or fragment thereof as claimed in claim 6.

8. An immunoassay kit comprising a monoclonal antibody or fragment thereof as claimed in claim 6, and
a competition agent which binds said monoclonal antibody or fragment thereof, and optionally one or more of a wash reagent, a buffer, a stopping reagent, an enzyme label, an enzyme label substrate, calibration standards, an anti-mouse antibody and instructions for conducting an assay using said kit.

## Patentansprüche

1. Verfahren für einen Bioassay für die Quantifizierung von Peptidfragmenten von C-reaktivem Protein (CRP), das eine N-terminale Neo-Epitop-Aminosäuresequenz KAFVFP umfasst, wobei das Verfahren ein Inberührungbringen einer Probe, die die Peptidfragmente umfasst, mit einem monoklonalen Antikörper oder einem Fragment davon, das eine spezifische Bindungsaffinität für die N-terminale Neo-Epitop-Aminosäuresequenz KAFVFP aufweist, und ein Bestimmen des Bindungsgrades des monoklonalen Antikörpers oder des Fragments davon an Peptidfragmente in der Probe umfasst.

2. Verfahren nach Anspruch 1, wobei das Verfahren als ein Konkurrenz-Immunoassay durchgeführt wird, wobei der monoklonale Antikörper oder das Fragment davon und ein Konkurrenzmittel in der Gegenwart der Probe inkubiert werden und das Konkurrenzmittel mit den Peptidfragmenten in der Probe konkurriert, um an den monoklonalen Antikörper oder das Fragment davon zu binden.

3. Verfahren nach Anspruch 2, wobei das Konkurrenzmittel ein synthetisches Peptid ist oder ein gereinigtes natives Peptid ist, das durch eine Spaltung von CRP ausgebildet wird, um das Neo-Epitop zu offenbaren.

4. Verfahren nach einem der vorhergehenden Ansprüche, wobei die Probe eine Urin-, Serum-, Blut- oder Plasmaprobe ist.

5. Verfahren nach einem der vorhergehenden Ansprüche, wobei die Probe eine von einem Patienten stammende Probe ist, wobei das Verfahren ferner ein Vergleichen des bestimmten Bindungsgrades der Peptidfragmente mit Werten umfasst, die für (a) vergleichbare gesunde Individuen und/oder (b) einen pathologischen atherosklerotischen Zustand bezeichnend sind.

6. Monoklonaler Antikörper oder Fragment davon, wobei der monoklonale Antikörper oder das Fragment davon eine spezifische Bindungsaffinität für die folgende Sequenz an dem N-Terminus eines Peptids aufweist: KAFVFP.

7. Zelllinie, die einen monoklonalen Antikörper oder ein Fragment davon nach Anspruch 6 produziert.

8. Immunoassay-Kit, das einen monoklonalen Antikörper oder ein Fragment davon nach Anspruch 6 und ein Konkurrenzmittel, das den monoklonalen Antikörper oder das Fragment davon bindet, und optional ein Waschreagens, einen Puffer, ein Stoppreagens, eine Enzymmarkierung, ein Enzymmarkierungssubstrat, Kalibrierungsstandards, einen Anti-Maus-Antikörper und/oder Anweisungen zum Durchführen eines Assays unter Verwendung des Kits umfasst.

## Revendications

1. Procédé de dosage biologique pour la quantification de fragments peptidiques de protéine C réactive (CRP) comprenant une séquence d'acides aminés néo-épitopique N-terminale KAFVFP, ledit procédé comprenant la mise en contact d'un échantillon comprenant lesdits fragments peptidiques avec un anticorps monoclonal ou un fragment de celui-ci ayant une affinité de liaison spécifique pour ladite séquence d'acides aminés néo-épitopique N-terminale KAFVFP, et la détermination du niveau de liaison dudit anticorps monoclonal ou d'un fragment de celui-ci à des fragments peptidiques dans ledit échantillon.

2. Procédé selon la revendication 1, dans lequel ledit procédé est conduit sous la forme d'un immunoessai de compétition dans lequel ledit anticorps monoclonal ou un fragment de celui-ci et un agent de compétition sont incubés en présence dudit échantillon et l'agent de compétition entre en concurrence avec les fragments peptidiques dans l'échantillon à se lier à l'anticorps monoclonal ou à un fragment de celui-ci.

3. Procédé selon la revendication 2, dans lequel ledit agent de compétition est un peptide synthétique ou est un peptide natif purifié formé par clivage de la CRP de manière à révéler ledit néo-épitope.

4. Procédé selon l'une quelconque des revendications précédentes, dans lequel l'échantillon est un échantillon d'urine, de sérum, de sang ou de plasma.

5. Procédé selon l'une quelconque des revendications précédentes, dans lequel l'échantillon est un échantillon dérivé de patient, ledit procédé comprenant en outre la comparaison du niveau déterminé de ladite liaison desdits fragments peptidiques avec des valeurs caractéristiques (a) d'individus en bonne santé comparables et/ou (b) une condition athéroscléreuse pathologique.

6. Anticorps monoclonal ou fragment de celui-ci, dans lequel ledit anticorps monoclonal ou fragment de celui-ci présente une affinité de liaison spécifique pour la séquence suivante à l'extrémité N d'un peptide : KAFVFP.

7. Lignée cellulaire produisant un anticorps monoclonal ou un fragment de celui-ci selon la revendication 6.

8. Kit d'immunoessai comprenant un anticorps monoclonal ou un fragment de celui-ci selon la revendication 6, et un agent de compétition qui se lie audit anticorps monoclonal ou fragment de celui-ci, et éventuellement un réactif de lavage et/ou un tampon et ou un réactif d'arrêt et/ou un marqueur enzymatique et/ou un substrat de marqueur enzymatique et/ou des standards d'étalonnage et/ou un anticorps anti-souris, et des instructions pour réaliser un dosage à l'aide dudit kit.
